# EUROPEAN PATENT SPECIFICATION

(11) **EP 2 844 660 B1**
(45) Date of publication and mention of the grant of the patent: **01.11.2017**
(21) Application number: 13784613.5
(22) Date of filing: 02.05.2013
(51) Int. Cl.: C07D 487/04, A61K 31/5025, A61P 35/00, A61P 25/00

(54) **TRIAZOLOPYRIDAZINE COMPOUNDS, USE AS INHIBITORS OF THE KINASE LRRK2, AND METHODS FOR PREPARATION THEREOF**
TRIAZOLOPYRIDAZINVERBINDUNGEN, VERWENDUNG ALS HEMMER VON KINASE-LRRK2 UND VERFAHREN ZUR HERSTELLUNG DAVON
COMPOSÉS DE TRIAZOLOPYRIDAZINE, UTILISATION COMME INHIBITEURS DE LA KINASE LRRK2, ET PROCÉDÉS POUR LEUR PRÉPARATION

(30) Priority: 02.05.2012 US 201261641364 P
(43) Date of publication of application: 11.03.2015
(73) Proprietor: Southern Research Institute, Birmingham, AL 35205-5305 (US); The UAB Research Foundation, Birmingham, AL 35294-0107 (US)
(72) Inventor: GALEMMO, Robert, A., Birmingham, AL 35205-5305 (US); WEST, Andrew, B., Birmingham, AL 35205-5305 (US); MADDRY, Joseph, A., Birmingham, AL 35205-5305 (US); ANANTHAN, Subramaniam, Birmingham, AL 35205-5305 (US); PATHAK, Ashish, Kumar, Birmingham, AL 35205-5305 (US); VALIYAVEETTIL, Jacob, Birmingham, AL 35205-5305 (US)
(74) Representative: Zanoli, Enrico
(86) International application number: PCT/US2013/039249
(87) International publication number: WO 2013/166276

(56) References cited:
- EP-A1- 1 481 977
- WO-A1-99/37303
- WO-A1-2008/069500
- WO-A1-2008/124838
- WO-A1-2011/141756
- WO-A2-2008/030744
- DE-A1- 2 030 218
- DE-A1- 2 109 577
- DE-A1- 2 202 744
- DE-A1- 2 202 745
- US-A- 3 989 832
- US-A1- 2004 192 696
- US-A1- 2004 192 696
- US-A1- 2008 312 225
- US-A1- 2010 099 683
- US-B1- 6 440 967
- CHEN D ET AL: "Synthesis, structures of novel zinc and copper compounds based on pyridazino[3,2-c]1,2,4-triazole derivatives", JOURNAL OF MOLECULAR STRUCTURE, ELSEVIER, AMSTERDAM, NL, vol. 920, no. 1-3, 28 February 2009 (2009-02-28), pages 342-349, XP025938331, ISSN: 0022-2860, DOI: 10.1016/J.MOLSTRUC.2008.11.030 [retrieved on 2008-11-24]
- LEGRAVEREND M ET AL: "SYNTHESIS OF S-TRIAZOLOÄ4,3-BÜPYRIDAZINE C-NUCLEOSIDES (1)", JOURNAL OF HETEROCYCLIC CHEMISTRY, WILEY-BLACKWELL PUBLISHING, INC, US, vol. 18, no. 5, 1 January 1981 (1981-01-01), pages 893-898, XP009029010, ISSN: 0022-152X, DOI: 10.1002/JHET.5570180509

## Description

### TECHNICAL FIELD

The present disclosure is concerned with certain triazolopyridazine compounds that are capable of inhibiting certain protein kinases, and especially the leucine-rich repeat kinase 2 (LRRK2) protein. Compounds of the present disclosure can be used to treat a number of disorders caused by or associated with abnormal LRRK2 kinase activity. Compounds of the present disclosure can be used to treat disorders including neurodegenerative diseases such as Parkinson's disease; precancerous conditions and cancer; autoimmune disorders such as Crohn's disease, rheumatoid arthritis and psoriasis; and leprosy (Hansen's disease).

### BACKGROUND ART

The LRRK2 gene encodes a protein kinase and it has been disclosed that missense mutations in this gene can lead to a number of diseases such as various neurodegenerative diseases including Parkinson's disease. Additionally, LRRK2 is genetically linked to precancerous conditions and cancer; autoimmune disorders such as Crohn's disease, rheumatoid arthritis and psoriasis; and leprosy. For instance, it has been suggested that certain mutations in LRRK2 can lead to Parkinson's disease through the up-regulation of the kinase activity of the protein kinase. It is suspected that this protein kinase may be over-active in Parkinson's disease. There has been much interest raised by the recent discovery that different autosomal dominant point mutations within the gene encoding for LRRK2 predispose humans to develop late-onset Parkinson's disease (OMIM accession number 609007), with a clinical appearance indistinguishable from idiopathic Parkinson's disease. See Paisan-Ruiz et al. (2004), "Cloning of the gene containing mutations that cause PARK 8-linked Parkinson's disease." Neuron. 44, 595-600; Mata et al. (2006), "LRRK2 in Parkinson's disease: protein domains and functional insights." Trends Neurosci. 29, 286-293; Taylor et al. (2006), "LRRK2: a common pathway for Parkinsonism, pathogenesis and prevention?" Trends Mol. Med. 12, 76-82. The genetic analysis undertaken to date indicates that mutations in LRRK2 are relatively frequent, not only accounting for 5-10% of familial Parkinson's disease, but also being found in a significant proportion of sporadic Parkinson's disease cases. See Farrer et al. (2005), "LRRK2 mutations in Parkinson disease." Neurology 65, 738-740; and Zabetian et al. (2005), "A clinic-based study of the LRRK2 gene in Parkinson disease yields new mutations." Neurology 65, 741-744. Little is known about how LRRK2 is regulated in cells, what its physiological substrates are and how mutations cause or increase risk of Parkinson's disease. The domain structure of LRRK2 is depicted in WO 2011/141756 A1. See Figure 1 therein, which also shows mutations which have been reported in patients with Parkinson's disease. The defining feature of the LRRK2 enzyme is a leucine rich repeat (LRR) motif (residues 1010-1291), a Ras-like small GTPase (residues 1336-1510), a region of high amino acid conservation that has been termed the C-terminal of Ras complex (COR) domain (residues 1511-1878), a protein kinase catalytic domain (residues 1879-2132) and a C-terminal VVD40 motif (2231-2276). See Bosgraaf et al. (2003), "Roc, a Ras/GTPase domain in complex proteins." Biochim. Biophys. Acta. 1643, 5-10; and Marin (2006), "The Parkinson disease gene LRRK2: evolutionary and structural insights." Mol. Biol. Evol. 23, 2423-2433.

The protein kinase domain of LRRK2 belongs to the tyrosine-like serine threonine protein kinases and is most similar to the kinase RIP (Receptor Interacting Protein), which play key roles in innate immunity signaling pathways. See Manning et al. (2002), "The protein kinase complement of the human genome." Science 298, 1912-1934. Almost 40 single amino acid substitution mutations have been linked to autosomal-dominant Parkinson's disease. Mata et al., supra; Taylor et al., supra; WO 2011/141756 A1. It has also been reported that the most prevalent mutant form of LRRK2 accounting for approximately 6% of familial Parkinson's disease and 3% of sporadic Parkinson's disease cases in Europe, comprises an amino acid substitution of Gly2019 to a Ser residue. Gly20l9 is located within the conserved DYG-Mg'-binding motif, in subdomain-VII of the kinase domain. Mata et al., supra. More recent reports suggest that this mutation enhances the autophosphorylation of LRRK2, as well as its ability to phosphorylate myelin basic protein 2-3-fold. West et al. (2005), "Parkinson's disease-associated mutations in leucine-rich repeat kinase 2 augment kinase activity." Proc. Natl. Acad. Sci. USA 102, 16842-16847; and Greggio et al. (2006), "Kinase activity is required for the toxic effects of mutant LRRK21dardarin." Neurobiol. Dis. 23, 329-341. These observations suggest that over-activation of LRRK2 predisposes humans to develop some forms of Parkinson's disease. As discussed in "Chromosomal amplification of leucine-rich repeat kinase-2 (LRRK2) is required for oncogenic MET signaling in papillary renal and thyroid carcinomas", Looyeng et al., Proceedings of the National Academy of Sciences of the United States of America (2011), 108(4), 1439-1444, S1439/1-S1439/10; language: English; database: CAPLUS, DOI:10.1073/pnas.1012500108; the receptor tyrosine kinase MET is frequently amplified in human tumors, resulting in high cell surface densities and constitutive activation even in the absence of growth factor stimulation by its endogenous ligand, hepatocyte growth factor (HGF). LRRK2 was identified and shown to be amplified and overexpressed in papillary renal and thyroid carcinomas. Down-regulation of LRRK2 in cultured tumor cells compromises MET activation and selectively reduces downstream MET signaling to mTOR and STAT3. Loss of these critical mitogenic pathways induces cell cycle arrest and cell death due to loss of ATP production, indicating that MET and LRRK2 cooperate to promote efficient tumor cell growth and survival in these cancers.

Missense mutations in LRRK2, as discussed above cause late-onset Parkinson's disease (PD). In addition, common genetic variation in LRRK2 modifies susceptibility to Crohn's disease and leprosy. See "LRRK2 inhibition attenuates microglial inflammatory responses", Moehle et al., Journal of Neuroscience (2012), 32(5), 1602-1611. Language: English, Database: CAPLUS, DOI: 10.1523/JNEUROSCI.5601-11.2012.

Included among the genes identified as being associated with leprosy susceptibility or resistance, PARK2 and LRRK2 have been discussed as participating in the regulation of host-cell apoptosis. See "Leprosy susceptibility: genetic variations regulate innate and adaptive immunity, and disease outcome," Cardoso et al., Future Microbiology (2011), 6(5), 533-549. Cardoso et al. further report that the same genes associated with leprosy are also associated with autoimmune (Crohn's disease, rheumatoid arthritis, psoriasis) or neurodegenerative diseases (Parkinson's and Alzheimer's).

WO 2011/141756 relates to certain pyrazolopyridines compounds that are capable of inhibiting one or more kinases, more particularly, LRRK2. The compounds find applications in the treatment of a variety of disorders, including cancer and neurodegenerative diseases such as Parkinson's disease.

### SUMMARY OF THE DISCLOSURE

Certain triazolopyridazine compounds have been discovered according to the present disclosure that inhibit LRRK2, and therefore find utility in the treatment of a number of disorders neurodegenerative diseases such as Parkinson's disease; precancerous conditions and cancer; autoimmune disorders such as Crohn's disease, rheumatoid arthritis and psoriasis; and leprosy. In an aspect, the present disclosure pertains to a compound according to any one of the foregoing aspects one to five which is selected from the group consisting of:
1-(piperidin-1-yl)-2-((3-(thiophen-2-yl)-[1,2,4]triazolo[4,3-b]pyridazin-6-yl)thio)butan-1-one,
2-((3-(thiophen-2-yl)-[1,2,4]triazolo[4,3-b]pyridazin-6-yl)thio)propanoic acid,
1-(piperidin-1-yl)-2-((3-(thiophen-2-yl)-[1,2,4]triazolo[4,3-b]pyridazin-6-yl)thio)propan-1-one,
1-(azetidin-1-yl)-2-((3-(thiophen-2-yl)-[1,2,4]triazolo[4,3-b]pyridazin-6-yl)thio)propan-1-one,
1-(pyrrolidin-1-yl)-2-((3-(thiophen-2-yl)-[1,2,4]triazolo[4,3-b]pyridazin-6-yl)thio)propan-1-one and
2-(methyl((3-(thiophen-2-yl)-[1,2,4]triazolo[4,3-b]pyridazin-6-yl)methyl)amino)-1-(piperidin-1-yl)propan-1-one,
or the derivative thereof, wherein said derivative is selected from the group consisting of pharmaceutically acceptable salts thereof, deuterated forms thereof, ratio-actively labeled forms thereof, enantiomers, diastereomers and solvates thereof. In another aspect, the present disclosure pertains to a pharmaceutical composition comprising a compound or derivative according to the foregoing aspect, and a pharmaceutically acceptable carrier.

The present disclosure pertains also to a method of treating a patient having a disease caused by or associated with abnormal LRRK2 kinase activity, which comprises administering to the patient an effective treatment amount of at least one compound or derivative according to one of the foregoing aspects.

The present disclosure pertains also to a method for treating a patient having a neurodegenerative disease, which comprises administering to the patient an effective treatment amount of at least one compound or derivative according one of the foregoing aspects.

The present disclosure pertains also to the method according to the foregoing aspect, wherein the neurodegenerative disease is Parkinson's disease.

The present disclosure pertains also to a method for treating a patient suffering from cancer, which comprises administering to the patient an effective treatment amount of at least one compound or derivative according to either one of the foregoing aspects.

The present disclosure pertains also to the method according to the foregoing aspect, wherein said cancer is renal cancer or thyroid cancer.

The present disclosure pertains also to a method for treating a patient having an autoimmune disease, which comprises administering to the patient an effective treatment amount of at least one compound or derivative according to one of the foregoing aspects.

The present disclosure pertains also to the method according to the foregoing aspect, wherein the autoimmune disease is selected from the group consisting of Crohn's disease, rheumatoid arthritis and psoriasis.

The present disclosure pertains also to a method for treating a patient having leprosy, which comprises administering to the patient an effective treatment amount of at least one compound or derivative according to one of the foregoing aspects.

The present disclosure is also concerned with enhancing the blood brain barrier transmission of the above disclosed compounds by modifying the compounds and/or employing the compounds along with another component capable of enhancing transmission of the compound through the blood brain barrier.

Still other objects and advantages of the present disclosure will become readily apparent by those skilled in the art from the following detailed description, wherein it is shown and described only the preferred embodiments, simply by way of illustration of the best mode. Accordingly, the description is to be regarded as illustrative in nature and not as restrictive.

### BEST AND VARIOUS MODES FOR CARRYING OUT DISCLOSURE

Certain triazolopyridazine compounds have been discovered according to the present disclosure that inhibit LRRK2, and therefore find utility in a number of disorders neurodegenerative diseases such as Parkinson's disease; precancerous conditions and cancer; autoimmune disorders such as Crohn's disease, rheumatoid arthritis and psoriasis; and leprosy.

Particular embodiments of the present disclosure are compounds selected from the group consisting of:
1-(piperidin-1-yl)-2-((3-(thiophen-2-yl)-[1,2,4]triazolo[4,3-b]pyridazin-6-yl)thio)butan-1-one,
2-((3-(thiophen-2-yl)-[1,2,4]triazolo[4,3-b]pyridazin-6-yl)thio)propanoic acid,
1-(piperidin-1-yl)-2-((3-(thiophen-2-yl)-[1,2,4]triazolo[4,3-b]pyridazin-6-yl)thio)propan-1-one,
1-(azetidin-1-yl)-2-((3-(thiophen-2-yl)-[1,2,4]triazolo[4,3-b]pyridazin-6-yl)thio)propan-1-one,
1-(pyrrolidin-1-yl)-2-((3-(thiophen-2-yl)-[1,2,4]triazolo[4,3-b]pyridazin-6-yl)thio)propan-1-one and
2-(methyl((3-(thiophen-2-yl)-[1,2,4]triazolo[4,3-b]pyridazin-6-yl)methyl)amino)-1-(piperidin-1-yl)propan-1-one,
and derivatives thereof, wherein said derivative is selected from the group consisting of pharmaceutically acceptable salts thereof, deuterated forms thereof, ratio-actively labeled forms thereof, enantiomers, diastereomers and solvates thereof. An example of an aryl ring is phenyl. The heterocyclic rings can include one or two or three heteroatoms such as N, S or O and can be heteroaryl rings. Examples of 5- and 6-membered N-heterocyclic groups are pyridyl, pyrrolidinyl, piperidinyl, piperazinyl, pyridinyl, pyrrolyl, pyrazolyl, pyrazinyl pyrimidinyl, pyridazinyl, imidazoyl, imidazolidinyl, 1,2,3-triazole, 1,2,4-triazole, indolyl and benzimidazolyl. Examples of O-heterocyclic groups are furanyl, pyranyl and benzofuranyl. Examples of S-heterocyclic groups are thiopyranyl, thienyl and benzothiophene. Examples of heterocyclic groups containing both N and O are morpholinyl, oxazole, isooxazole and benzisoxazole. Example of heterocyclic groups containing both N and S are thiomorpholine, thiazole, isothiazole and benzothiazole. Examples of heterocyclic groups containing 4 to 7 atoms in the ring for R⁵ are represented by the following formulae: 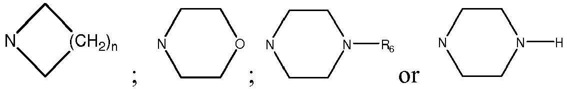 wherein n is a whole number integer from 1 to 3 and more typically 2 or 3 and may be optionally substituted or unsubstituted. The 4- to 8-membered rings formed by NR⁶R⁷ consist of the nitrogen atom to which R⁶ and R⁷ are bonded, carbon ring members and optionally an additional ring member selected from the group consisting of: O, S, SO, SO₂, N, and N(C₁-C₄-alkyl). The rings may be unsaturated, partially saturated or aromatic. Examples of saturated rings include those enumerated for R⁵. Other ring structures include pyridyl, pyrrolidinyl, piperidinyl, piperazinyl, pyridinyl, pyrrolyl, pyrazolyl, pyrazinyl pyrimidinyl, pyridazinyl, imidazoyl, imidazolidinyl, 1,2,3-triazole, 1,2,4-triazole, indolyl, benzimidazolyl, oxazole, isooxazole, benzisoxazole thiazole, isothiazole, benzothiazole and the partially hydrogenated or saturated counterparts thereof as well as thomorpholinyl. In sulfur containing rings, the sulfur ring member may by oxidized to form an -S(=O)- or -S(=O)₂-ring member.

The alkyl group typically contains 1-12 carbon atoms. The alkyl group more typically contains 1-4 carbon atoms. Examples of suitable alkyl groups include methyl, ethyl and propyl. Examples branched alkyl groups include isopropyl and t-butyl.

Examples of halogen groups are F, Cl, Br and I. Haloalkyl groups include any one of the foregoing alkyl groups in which one or more of the hydrogen is replaces by a corresponding number of identical or different halogen. Illustrative examples of haloalkyl groups include fluoromethyl, difluoromethyl, trifluoromethyl, chloromethyl, bromomethyl, iodomethyl, chloro-difluoromethyl, 2-fluoroethyl, 2-chloroethyl, 2-bromoethyl, 2-iodoethyl, 1,2-difluoroethyl, 2,2-difluoroethyl, 1-chloro-2-fluoroethyl, 2-chloro-2-fluoroethyl, 1-bromo-2-fluoroethyl, 2-bromo-2-fluoroethyl, 1-flouro-2-iodoethyl, 2-fluoro-2-iodoethyl, 1,1,2-trifluoroethyl, 1,2,2-trifluoroethyl, 2,2,2-trifluoroethyl, and the like.

Examples of unsubstituted 5- or 6-member saturuated hydrocarbon ring are cyclopentyl and cyclohexyl.

When any of the above groups is substituted, the substitutions include at least one alkyl group, halogen group, haloalkyl, hydroxyl, alkoxy group containing 1-12 carbon atoms and more typically 1-6 or 1-4 carbon atoms, amino group or aminoalkyl group.

When R³ and R⁴ are combined to form a ring moiety, the ring member typically contains 3-6 atoms in the ring and can be saturated or unsaturated hydrocarbon ring or a heterocyclo ring containing at least on hetero atom selected from the group consisting of O, S and N. When R³ and R⁴ are taken together to form a double or triple bond, such is typically substituted with alkyl or unsubstituted.

"Derivatives" of the compounds disclosed herein are pharmaceutically acceptable salts, prodrugs, deuterated forms, radio-actively labeled forms, enantiomers, diastereomers, solvates and combinations thereof. The "combinations" mentioned in this context refer to derivatives falling within at least two of the groups: pharmaceutically acceptable salts, deuterated forms, radio-actively labeled forms, enantiomers, diastereomers, and solvates.

The compounds according to this disclosure may form prodrugs at hydroxyl or amino functionalities using alkoxy, amino acids, etc. groups as the prodrug forming moieties. For instance, the hydroxymethyl position may form mono-, di- or triphosphates and again these phosphates can form prodrugs. Preparations of such prodrug derivatives are discussed in various literature sources (examples are: Alexander et al., J. Med. Chem. 1988, 31, 318; Aligas-Martin et al., PCT WO 2000/041531, p. 30). The nitrogen function converted in preparing these derivatives is one (or more) of the nitrogen atoms of a compound of the disclosure.

"Pharmaceutically acceptable salts" refer to derivatives of the disclosed compounds wherein the parent compound is modified by making acid or base salts thereof. The compounds of this disclosure form acid addition salts with a wide variety of organic and inorganic acids and include the physiologically acceptable salts which are often used in pharmaceutical chemistry. Such salts are also part of this disclosure. Typical inorganic acids used to form such salts include hydrochloric, hydrobromic, hydroiodic, nitric, sulfuric, phosphoric, hypophosphoric acid, and the like. Salts derived from organic acids, such as aliphatic mono- and dicarboxylic acids, phenyl substituted alkanoic acids, hydroxyalkanoic and hydroxyalkandioic acids, aromatic acids, aliphatic and aromatic sulfonic acids may also be used. Such pharmaceutically acceptable salts thus include acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, naphthalene-2-benzoate, bromide, isobutyrate, phenylbutyrate, β-hydroxybutyrate, butyne-1,4-dioate, hexyne-1,4-dioate, caprate, caprylate, chloride, cinnamate, citrate, formate, fumarate, glycollate, heptanoate, hippurate, lactate, malate, maleate, hydroxymaleate, malonate, mandelate, mesylate, nicotinate, isonicotinate, nitrate, oxalate, phthalate, teraphthalate, phosphate, monohydrogenphosphate, dihydrogenphosphate, metaphosphate, pyrophosphate, propiolate, propionate, phenylpropionate, salicylate, sebacate, succinate, suberate, sulfate, bisulfate, pyrosulfate, sulfite, bisulfite, sulfonate, benzenesulfonate, p-bromobenzenesulfonate, chlorobenzenesulfonate, ethanesulfonate, 2-hydroxyethanesulfonate, methanesulfonate, naphthalene-1-sulfonate, naphthalene-2-sulfonate, *p*-toleunesulfonate, xylenesulfonate, tartarate, and the like.

It is understood that the compounds of the present disclosure relate to all optical isomers and stereo-isomers at the various possible atoms of the molecule, unless specified otherwise. Compounds may be separated or prepared as their pure enantiomers or diasteriomers by crystallization, chromatography or synthesis.

The deuterated forms contain heavy hydrogen including deuterium. The carbon labeled forms may contain carbon-13.

Examples of radio-actively labeled forms include compounds labeled with tritium, phosphorous-32, iodine-129, carbon-11, fluorine-18, and the like.

"Solvates" refers to the compound formed by the interaction of a solvent and a solute and includes hydrates. Solvates are usually crystalline solid adducts containing solvent molecules within the crystal structure, in either stoichiometric or nonstoichiometric proportions.

The term "comprising" (and its grammatical variations) as used herein is used in the inclusive sense of "having" or "including" and not in the exclusive sense of "consisting of." The terms "a" and "the" as used herein are understood to encompass the plural as well as the singular.

Examples of compounds according to the present disclosure are shown in the table below, wherein (I) means compounds according to the invention, and (R) means reference compounds:

| Example # | Compound Names |
|---|---|
| 1 (R) | ethyl 2-((3-(4-fluorophenyl)-[1,2,4]triazolo[4,3-b]pyridazin-6-yl)thio)butanoate |
| 2 (R) | ethyl 2-((3-(pyridin-4-yl)-[1,2,4]triazolo[4,3-b]pyridazin-6-yl)thio)acetate |
| 3 (R) | 2-((3-(thiophen-2-yl)-[1,2,4]triazolo[4,3-b]pyridazin-6-yl)thio)acetamide |
| 4 (R) | 2-((3-(pyridin-4-yl)-[1,2,4]triazolo[4,3-b]pyridazin-6-yl)thio)acetamide |
| 5 (R) | 2-((3-(thiophen-3-yl)-[1,2,4]triazolo[4,3-b]pyridazin-6-yl)thio)acetamide |
| 6 (R) | 2-((3-(4-fluorophenyl)-[1,2,4]triazolo[4,3-b]pyridazin-6-yl)thio)butanoic acid |
| 7 (R) | 2-((3-(4-fluorophenyl)-[1,2,4]triazolo[4,3-b]pyridazin-6-yl)thio)-N-(thiazol-2-yl)butanamide |
| 8 (R) | 2-((3-(4-fluorophenyl)-[1,2,4]triazolo[4,3-b]pyridazin-6-yl)thio)-1-(pyrrolidin-1-yl)butan-1-one |
| 9 (R) | 2-((3-(4-fluorophenyl)-[1,2,4]triazolo[4,3-b]pyridazin-6-yl)thio)-1-(piperidin-1-yl)butan-1-one |
| 10 (R) | 2-((3-cyclohexyl-[1,2,4]triazolo[4,3-b]pyridazin-6-yl)thio)acetamide |
| 11 (R) | 1-(piperidin-1-yl)-2-((3-(thiophen-2-yl)-[1,2,4]triazolo[4,3-b]pyridazin-6-yl)thio)ethanone |
| 12 (R) | 2-((3-(4-fluorophenyl)-[1,2,4]triazolo[4,3-b]pyridazin-6-yl)thio)-N-(quinolin-2-yl)butanamide |
| 13 (R) | 2-((3-(p-tolyl)-[1,2,4]triazolo[4,3-b]pyridazin-6-yl)thio)acetic acid |
| 14 (R) | 2-((3-(4-fluorophenyl)-[1,2,4]triazolo[4,3-b]pyridazin-6-yl)thio)-N-(2-(trifluoromethyl)phenyl)butanamide |
| 15 (R) | 2-((3-cyclopentyl-[1,2,4]triazolo[4,3-b]pyridazin-6-yl)thio)acetamide |
| 16 (R) | 2-((3-(pyridin-2-yl)-[1,2,4]triazolo[4,3-b]pyridazin-6-yl)thio)acetamide |
| 17 (R) | 2-((3-(4-fluorophenyl)-[1,2,4]triazolo[4,3-b]pyridazin-6-yl)thio)acetic acid |
| 18 (R) | 2-((3-(thiophen-2-yl)-[1,2,4]triazolo[4,3-b]pyridazin-6-yl)thio)butanoic acid |
| 19 (R) | 2-((3-(4-fluorophenyl)-[1,2,4]triazolo[4,3-b]pyridazin-6-yl)thio)acetamide |
| 20 (R) | 6-(piperidin-1-yl)-3-(thiophen-2-yl)-[1,2,4]triazolo[4,3-b]pyridazine |
| 21 (R) | 1-(pyrrolidin-1-yl)-2-((3-(thiophen-2-yl)-[1,2,4]triazolo[4,3-b]pyridazin-6-yl)thio)ethanone |
| 22 (R) | 2-((3-(thiophen-2-yl)-[1,2,4]triazolo[4,3-b]pyridazin-6-yl)thio)-N-(p-tolyl)acetamide |
| 23 (R) | N-(thiazol-2-yl)-2-((3-(thiophen-2-yl)-[1,2,4]triazolo[4,3-b]pyridazin-6-yl)thio)acetamide |
| 24 (R) | 1-morpholino-2-((3-(thiophen-2-yl)-[1,2,4]triazolo[4,3-b]pyridazin-6-yl)thio)ethanone |
| 25 (R) | 1-(pyrrolidin-1-yl)-2-((3-(p-tolyl)-[1,2,4]triazolo[4,3-b]pyridazin-6-yl)thio)ethanone |
| 26 (R) | 2-((3-(furan-2-yl)-[1,2,4]triazolo[4,3-b]pyridazin-6-yl)thio)acetamide |
| 27 (R) | 2-((3-(thiophen-2-yl)-[1,2,4]triazolo[4,3-b]pyridazin-6-yl)thio)acetic acid |
| 28 (R) | 2-((3-(furan-2-yl)-[1,2,4]triazolo[4,3-b]pyridazin-6-yl)thio)acetic acid |
| 29 (R) | 2-((3-(benzo[b]thiophen-2-yl)-[1,2,4]triazolo[4,3-b]pyridazin-6-yl)thio)acetamide |
| 30 (R) | 2-((3-(benzo[b]thiophen-2-yl)-[1,2,4]triazolo[4,3-b]pyridazin-6-yl)thio)acetic acid |
| 31 (R) | 2-((3-(benzofuran-2-yl)-[1,2,4]triazolo[4,3-b]pyridazin-6-yl)thio)acetic acid |
| 32 (R) | 2-((3-(benzofuran-2-yl)-[1,2,4]triazolo[4,3-b]pyridazin-6-yl)thio)acetamide |
| 33 (R) | 2-((3-(thiazol-2-yl)-[1,2,4]triazolo[4,3-b]pyridazin-6-yl)thio)butanoic acid |
| 34 (I) | 1-(piperidin-1-yl)-2-((3-(thiophen-2-yl)-[1,2,4]triazolo[4,3-b]pyridazin-6-yl)thio)butan-1-one |
| 35 (R) | 2-((3-(benzo[b]thiophen-3-yl)-[1,2,4]triazolo[4,3-b]pyridazin-6-yl)thio)acetamide |
| 36 (I) | 2-((3-(thiophen-2-yl)-[1,2,4]triazolo[4,3-b]pyridazin-6-yl)thio)propanoic acid |
| 37 (R) | 2-((3-(thiazol-2-yl)-[1,2,4]triazolo[4,3-b]pyridazin-6-yl)thio)acetamide |
| 38 (R) | 2-((3-(benzo[b]thiophen-3-yl)-[1,2,4]triazolo[4,3-b]pyridazin-6-yl)thio)acetic acid |
| 39 (R) | 2-((3-(5-methyl-4-phenylthiophen-3-yl)-[1,2,4]triazolo[4,3-b]pyridazin-6-yl)thio)acetic acid |
| 40 (R) | 2-((3-(5-methyl-4-phenylthiophen-3-yl)-[1,2,4]triazolo[4,3-b]pyridazin-6-yl)thio)acetamide |
| 41 (R) | 2-((3-(thiophen-2-yl)-[1,2,4]triazolo[4,3-b]pyridazin-6-yl)thio)hexanoic acid |
| 42 (R) | 2-((3-(thiazol-2-yl)-[1,2,4]triazolo[4,3-b]pyridazin-6-yl)thio)acetic acid |
| 43 (R) | 2-((3-(4-fluorophenyl)-[1,2,4]triazolo[4,3-b]pyridazin-6-yl)thio)-N-(thiazol-2-yl)acetamide |
| 44 (R) | 2-((3-(thiophen-2-yl)-[1,2,4]triazolo[4,3-b]pyridazin-6-yl)thio)butanamide |
| 45 (R) | 2-((3-(4-fluorophenyl)-[1,2,4]triazolo[4,3-b]pyridazin-6-yl)thio)butanamide |
| 46 (R) | 1-(pyrrolidin-1-yl)-2-((3-(thiophen-2-yl)-[1,2,4]triazolo[4,3-b]pyridazin-6-yl)thio)butan-1-one |
| 47 (R) | 1-morpholino-2-((3-(thiophen-2-yl)-[1,2,4]triazolo[4,3-b]pyridazin-6-yl)thio)butan-1-one |
| 48 (R) | 2-((3-(thiophen-2-yl)-[1,2,4]triazolo[4,3-b]pyridazin-6-yl)thio)-N-(thiophen-2-ylmethyl)butanamide |
| 49 (R) | 2-((3-(5-methylthiophen-2-yl)-[1,2,4]triazolo[4,3-b]pyridazin-6-yl)thio)acetamide |
| 50 (R) | 1-(azepan-1-yl)-2-((3-(thiophen-2-yl)-[1,2,4]triazolo[4,3-b]pyridazin-6-yl)thio)butan-1-one |
| 51 (R) | 1-(3-methylpiperidin-1-yl)-2-((3-(thiophen-2-yl)-[1,2,4]triazolo[4,3-b]pyridazin-6-yl)thio)butan-1-one |
| 52 (R) | N-cyclohexyl-2-((3-(thiophen-2-yl)-[1,2,4]triazolo[4,3-b]pyridazin-6-yl)thio)butanamide |
| 53 (R) | 2-((3-(4-(trifluoromethyl)phenyl)-[1,2,4]triazolo[4,3-b]pyridazin-6-yl)thio)butanoic acid |
| 54 (R) | 2-((3-(3,4-difluorophenyl)-[1,2,4]triazolo[4,3-b]pyridazin-6-yl)thio)butanoic acid |
| 55 (R) | 2-((3-(2-fluorophenyl)-[1,2,4]triazolo[4,3-b]pyridazin-6-yl)thio)butanoic acid |
| 56 (R) | 2-((3-(3-fluorophenyl)-[1,2,4]triazolo[4,3-b]pyridazin-6-yl)thio)butanoic acid |
| 57 (R) | 2-((3-(5-methylthiophen-2-yl)-[1,2,4]triazolo[4,3-b]pyridazin-6-yl)thio)-1-(piperidin-1-yl)ethanone |
| 58 (R) | 1-(4-methylpiperazin-1-yl)-2-((3-(thiophen-2-yl)-[1,2,4]triazolo[4,3-b]pyridazin-6-yl)thio)butan-1-one |
| 59 (R) | 2-((3-(5-methylthiophen-2-yl)-[1,2,4]triazolo[4,3-b]pyridazin-6-yl)thio)-1-(piperidin-1-yl)butan-1-one |
| 60 (R) | 1-(piperidin-1-yl)-2-((3-(thiazol-2-yl)-[1,2,4]triazolo[4,3-b]pyridazin-6-yl)thio)butan-1-one |
| 61 (R) | 2-((3-(5-chlorothiophen-2-yl)-[1,2,4]triazolo[4,3-b]pyridazin-6-yl)thio)butanoic acid |
| 62 (R) | 2-((3-(5-chlorothiophen-2-yl)-[1,2,4]triazolo[4,3-b]pyridazin-6-yl)thio)-1-(piperidin-1-yl)butan-1-one |
| 63 (R) | 2-((3-(5-chlorothiophen-2-yl)-[1,2,4]triazolo[4,3-b]pyridazin-6-yl)thio)-1-(pyrrolidin-1-yl)butan-1-one |
| 64 (R) | 1-(piperidin-1-yl)-2-((3-(thiophen-3-yl)-[1,2,4]triazolo[4,3-b]pyridazin-6-yl)thio)butan-1-one |
| 65 (R) | ethyl 2-((3-(4-methoxyphenyl)-[1,2,4]triazolo[4,3-b]pyridazin-6-yl)thio)butanoate |
| 66 (R) | 2-((3-(4-methoxyphenyl)-[1,2,4]triazolo[4,3-b]pyridazin-6-yl)thio)butanamide |
| 67 (R) | 2-((3-(4-methoxyphenyl)-[1,2,4]triazolo[4,3-b]pyridazin-6-yl)thio)-1-(piperidin-1-yl)butan-1-one |
| 68 (R) | ethyl 2-((3-(2-methoxyphenyl)-[1,2,4]triazolo[4,3-b]pyridazin-6-yl)thio)butanoate |
| 69 (R) | 2-((3-(2-methoxyphenyl)-[1,2,4]triazolo[4,3-b]pyridazin-6-yl)thio)butanamide |
| 70 (R) | 2-((3-(2-methoxyphenyl)-[1,2,4]triazolo[4,3-b]pyridazin-6-yl)thio)-1-(piperidin-1-yl)butan-1-one |
| 71 (R) | ethyl 2-((3-(3-methoxyphenyl)-[1,2,4]triazolo[4,3-b]pyridazin-6-yl)thio)butanoate |
| 72 (R) | 2-((3-(3-methoxyphenyl)-[1,2,4]triazolo[4,3-b]pyridazin-6-yl)thio)butanamide |
| 73 (R) | 2-((3-(3-methoxyphenyl)-[1,2,4]triazolo[4,3-b]pyridazin-6-yl)thio)-1-(piperidin-1-yl)butan-1-one |
| 74 (R) | ethyl 2-((3-(2-fluorophenyl)-[1,2,4]triazolo[4,3-b]pyridazin-6-yl)thio)butanoate |
| 75 (R) | ethyl 2-((3-(3-fluorophenyl)-[1,2,4]triazolo[4,3-b]pyridazin-6-yl)thio)butanoate |
| 76 (R) | ethyl 2-((3-(3,4-difluorophenyl)-[1,2,4]triazolo[4,3-b]pyridazin-6-yl)thio)butanoate |
| 77 (R) | ethyl 2-((3-(4-(trifluoromethyl)phenyl)-[1,2,4]triazolo[4,3-b]pyridazin-6-yl)thio)butanoate |
| 78 (R) | 2-((3-(4-(trifluoromethyl)phenyl)-[1,2,4]triazolo[4,3-b]pyridazin-6-yl)thio)butanamide |
| 79 (R) | 2-((3-(3,4-difluorophenyl)-[1,2,4]triazolo[4,3-b]pyridazin-6-yl)thio)butanamide |
| 80 (R) | 2-((3-(2-fluorophenyl)-[1,2,4]triazolo[4,3-b]pyridazin-6-yl)thio)butanamide |
| 81 (R) | 2-((3-(3-fluorophenyl)-[1,2,4]triazolo[4,3-b]pyridazin-6-yl)thio)butanamide |
| 82 (R) | ethyl 2-((3-(thiophen-2-yl)-[1,2,4]triazolo[4,3-b]pyridazin-6-yl)oxy)butanoate |
| 83 (R) | 2-((3-(thiophen-2-yl)-[1,2,4]triazolo[4,3-b]pyridazin-6-yl)oxy)butanoic acid |
| 84 (R) | 2-((3-(thiophen-2-yl)-[1,2,4]triazolo[4,3-b]pyridazin-6-yl)oxy)butanamide |
| 85 (R) | 1-(piperidin-1-yl)-2-((3-(thiophen-2-yl)-[1,2,4]triazolo[4,3-b]pyridazin-6-yl)oxy)butan-1-one |
| 86 (R) | N-phenyl-2-((3-(thiophen-2-yl)-[1,2,4]triazolo[4,3-b]pyridazin-6-yl)thio)butanamide |
| 87 (R) | 2-((3-(5-ethylthiophen-2-yl)-[1,2,4]triazolo[4,3-b]pyridazin-6-yl)thio)-1-(piperidin-1-yl)butan-1-one |
| 88 (R) | 2-((3-(5-ethylthiophen-2-yl)imidazo[1,2-b]pyridazin-6-yl)thio)butanoic acid |
| 89 (R) | 2-((3-(5-methylthiophen-2-yl)-[1,2,4]triazolo[4,3-b]pyridazin-6-yl)thio)butanoic acid |
| 90 (I) | 1-(piperidin-1-yl)-2-((3-(thiophen-2-yl)-[1,2,4]triazolo[4,3-b]pyridazin-6-yl)thio)propan-1-one |
| 91 (R) | 2-((3-(thiophen-2-yl)-[1,2,4]triazolo[4,3-b]pyridazin-6-yl)sulfonyl)acetamide |
| 92 (R) | 2-((3-(thiophen-2-yl)-[1,2,4]triazolo[4,3-b]pyridazin-6-yl)thio)pentanoic acid |
| 93 (R) | 1-(piperidin-1-yl)-2-((3-(thiophen-2-yl)-[1,2,4]triazolo[4,3-b]pyridazin-6-yl)thio)pentan-1-one |
| 94 (R) | 2-((3-(2-fluorophenyl)-[1,2,4]triazolo[4,3-b]pyridazin-6-yl)thio)-1-(piperidin-1-yl)butan-1-one |
| 95 (R) | 1-(piperidin-1-yl)-2-((3-(4-(trifluoromethyl)phenyl)-[1,2,4]triazolo[4,3-b]pyridazin-6-yl)thio)butan-1-one |
| 96 (R) | 2-((3-(3,4-difluorophenyl)-[1,2,4]triazolo[4,3-b]pyridazin-6-yl)thio)-1-(piperidin-1-yl)butan-1-one |
| 97 (R) | 2-((3-(3-fluorophenyl)-[1,2,4]triazolo[4,3-b]pyridazin-6-yl)thio)-1-(piperidin-1-yl)butan-1-one |
| 98 (R) | methyl 2-((3-(thiophen-2-yl)-[1,2,4]triazolo[4,3-b]pyridazin-6-yl)amino)butanoate |
| 99 (R) | 2-((3-(thiophen-2-yl)-[1,2,4]triazolo[4,3-b]pyridazin-6-yl)amino)butanamide |
| 100 (R) | 1-(piperidin-1-yl)-2-((3-(thiophen-2-yl)-[1,2,4]triazolo[4,3-b]pyridazin-6-yl)amino)butan-1-one |
| 101 (I) | 1-(azetidin-1-yl)-2-((3-(thiophen-2-yl)-[1,2,4]triazolo[4,3-b]pyridazin-6-yl)thio)propan-1-one |
| 102 (R) | 1-(4,4-difluoropiperidin-1-yl)-2-((3-(thiophen-2-yl)-[1,2,4]triazolo[4,3-b]pyridazin-6-yl)thio)butan-1-one |
| 103 (R) | ethyl 1-(2-((3-(thiophen-2-yl)-[1,2,4]triazolo[4,3-b]pyridazin-6-yl)thio)butanoyl)piperidine-3-carboxylate |
| 104 (R) | 1-(2-methylpiperidin-1-yl)-2-((3-(thiophen-2-yl)-[1,2,4]triazolo[4,3-b]pyridazin-6-yl)thio)butan-1-one |
| 105 (I) | 1-(pyrrolidin-1-yl)-2-((3-(thiophen-2-yl)-[1,2,4]triazolo[4,3-b]pyridazin-6-yl)thio)propan-1-one |
| 106 (R) | 2-((3-phenyl-[1,2,4]triazolo[4,3-b]pyridazin-6-yl)thio)-1-(piperidin-1-yl)butan-1-one |
| 107 (R) | 2-methyl-1-(pyrrolidin-1-yl)-2-((3-(thiophen-2-yl)-[1,2,4]triazolo[4,3-b]pyridazin-6-yl)thio)propan-1-one |
| 108 (R) | 1-(piperidin-1-yl)-2-((3-(pyrazin-2-yl)-[1,2,4]triazolo[4,3-b]pyridazin-6-yl)thio)propan-1-one |
| 109 (R) | 1-(piperidin-1-yl)-2-((3-(pyrimidin-2-yl)-[1,2,4]triazolo[4,3-b]pyridazin-6-yl)thio)propan-1-one |
| 110 (R) | 1-(piperidin-1-yl)-2-((3-(thiazol-4-yl)-[1,2,4]triazolo[4,3-b]pyridazin-6-yl)thio)propan-1-one |
| 111 (R) | 1-(pyrrolidin-1-yl)-2-((3-(thiazol-4-yl)-[1,2,4]triazolo[4,3-b]pyridazin-6-yl)thio)propan-1-one |
| 112 (R) | 2-((3-(1-methyl-1H-pyrazol-5-yl)-[1,2,4]triazolo[4,3-b]pyridazin-6-yl)thio)-1-(piperidin-1-yl)propan-1-one |
| 113 (R) | 2-((3-(1-methyl-1H-pyrazol-3-yl)-[1,2,4]triazolo[4,3-b]pyridazin-6-yl)thio)-1-(piperidin-1-yl)propan-1-one |
| 114 (R) | 1-(azetidin-1-yl)-2-((3-(thiophen-2-yl)-[1,2,4]triazolo[4,3-b]pyridazin-6-yl)thio)butan-1-one |
| 115 (R) | 2-((3-(1H-pyrazol-5-yl)-[1,2,4]triazolo[4,3-b]pyridazin-6-yl)thio)-1-(piperidin-1-yl)propan-1-one |
| 116 (R) | 1-(piperidin-1-yl)-2-((3-(pyridazin-4-yl)-[1,2,4]triazolo[4,3-b]pyridazin-6-yl)thio)propan-1-one |
| 117 (R) | N,N-diethyl-2-((3-(thiophen-2-yl)-[1,2,4]triazolo[4,3-b]pyridazin-6-yl)thio)propanamide |
| 118 (R) | N,N-dimethyl-2-((3-(thiophen-2-yl)-[1,2,4]triazolo[4,3-b]pyridazin-6-yl)thio)propanamide |
| 119 (R) | N-methyl-2-((3-(thiophen-2-yl)-[1,2,4]triazolo[4,3-b]pyridazin-6-yl)thio)propanamide |
| 120 (R) | 2-((3-(1-methyl-1H-imidazol-2-yl)-[1,2,4]triazolo[4,3-b]pyridazin-6-yl)thio)-1-(piperidin-1-yl)propan-1-one |
| 121 (R) | N,N-dimethyl-2-((3-(thiophen-2-yl)-[1,2,4]triazolo[4,3-b]pyridazin-6-yl)thio)butanamide |
| 122 (R) | N-(2-hydroxyethyl)-2-((3-(thiophen-2-yl)-[1,2,4]triazolo[4,3-b]pyridazin-6-yl)thio)propanamide |
| 123 (R) | N,N-dipropyl-2-((3-(thiophen-2-yl)-[1,2,4]triazolo[4,3-b]pyridazin-6-yl)thio)propanamide |
| 124 (R) | 2-((3-(thiazol-2-yl)-[1,2,4]triazolo[4,3-b]pyridazin-6-yl)thio)propanoic acid |
| 125 (R) | 1-(piperidin-1-yl)-2-((3-(thiazol-2-yl)-[1,2,4]triazolo[4,3-b]pyridazin-6-yl)thio)propan-1-one |
| 126 (R) | 1-(azetidin-1-yl)-2-((3-(thiazol-2-yl)-[1,2,4]triazolo[4,3-b]pyridazin-6-yl)thio)propan-1-one |
| 128 (R) | 2-methyl-1-(piperidin-1-yl)-3-((3-(thiophen-2-yl)-[1,2,4]triazolo[4,3-b]pyridazin-6-yl)oxy)propan-1-one. |
| 129 (R) | 2-methyl-1-(piperidin-1-yl)-3-((3-(thiophen-2-yl)-[1,2,4]triazolo[4,3-b]pyridazin-6-yl)amino)propan-1-one |
| 130 (R) | 2-methyl-3-(methyl(3-(thiophen-2-yl)-[1,2,4]triazolo[4,3-b]pyridazin-6-yl)amino)-1-(piperidin-1-yl)propan-1-one |
| 131 (R) | 1-(piperidin-1-yl)-3-(3-(thiophen-2-yl)-[1,2,4]triazolo[4,3-b]pyridazin-6-yl)propane-1,3-dione |
| 132 (R) | 1-(piperidin-1-yl)-2-((3-(thiophen-2-yl)-[1,2,4]triazolo[4,3-b]pyridazin-6-yl)methoxy)propan-1-one |
| 133 (R) | 1-(piperidin-1-yl)-2-(((3-(thiophen-2-yl)-[1,2,4]triazolo[4,3-b]pyridazin-6-yl)methyl)amino)propan-1-one |
| 134 (I) | 2-(methyl((3-(thiophen-2-yl)-[1,2,4]triazolo[4,3-b]pyridazin-6-yl)methyl)amino)-1-(piperidin-1-yl)propan-1-one |
| 135 (R) | (3-(pyrimidin-2-yl)-[1,2,4]triazolo[4,3-b]pyridazin-6-yl)methanol |
| 136 (R) | N-(2-amino-2-oxoethyl)-3-(pyrimidin-2-yl)-[1,2,4]triazolo[4,3-b]pyridazine-6-carboxamide |
| 137 (R) | 3-(3-(thiazol-2-yl)-[1,2,4]triazolo[4,3-b]pyridazin-6-yl)propan-1-ol |
| 138 (R) | ethyl 2-(3-(pyrimidin-2-yl)-[1,2,4]triazolo[4,3-b]pyridazine-6-carboxamido)acetate |
| 139 (R) | 2-(6-(3-(pyrrolidin-1-yl)propyl)-[1,2,4]triazolo[4,3-b]pyridazin-3-yl)thiazole |
| 140 (R) | (R)-methyl 2-(3-(pyrimidin-2-yl)-[1,2,4]triazolo[4,3-b]pyridazine-6-carboxamido)propanoate |
| 141 (R) | 2-(3-(pyrimidin-2-yl)-[1,2,4]triazolo[4,3-b]pyridazine-6-carboxamido)acetic acid |
| 142 (R) | N-(2-morpholino-2-oxoethyl)-3-(pyrimidin-2-yl)-[1,2,4]triazolo[4,3-b]pyridazine-6-carboxamide |
| 143 (R) | (R)-2-(3-(pyrimidin-2-yl)-[1,2,4]triazolo[4,3-b]pyridazine-6-carboxamido)propanoic acid |
| 144 (R) | (R)-N-(1-oxo-1-(pyrrolidin-1-yl)propan-2-yl)-3-(pyrimidin-2-yl)-[1,2,4]triazolo[4,3-b]pyridazine-6-carboxamide |
| 145 (R) | (R)-N-(1-Morpholino-1-oxopropan-2-yl)-3-(pyrimidin-2-yl)-[1,2,4]triazolo[4,3-b]pyridazine-6-carboxamide |
| 146 (R) | (R)-N-(1-(dipropylamino)-1-oxopropan-2-yl)-3-(pyrimidin-2-yl)-[1,2,4]triazolo[4,3-b]pyridazine-6-carboxamide |
| 147 (R) | 2-methyl-1-(pyrrolidin-1-yl)-3-(3-(thiazol-2-yl)-[1,2,4]triazolo[4,3-b]pyridazin-6-yl)propan-1-one |
| 148 (R) | ethyl 2-methyl-3-(3-(pyrimidin-2-yl)-[1,2,4]triazolo[4,3-b]pyridazin-6-yl)propanoate |
| 149 (R) | 2-methyl-1-(piperidin-1-yl)-3-(3-(thiazol-2-yl)-[1,2,4]triazolo[4,3-b]pyridazin-6-yl)propan-1-one |
| 150 (R) | 2-methyl-1-morpholino-3-(3-(pyrimidin-2-yl)-[1,2,4]triazolo[4,3-b]pyridazin-6-yl)propan-1-one |
| 151 (R) | N,N,2-trimethyl-3-(3-(pyrimidin-2-yl)-[1,2,4]triazolo[4,3-b]pyridazin-6-yl)propanamide |
| 152 (R) | 2-methyl-N,N-dipropyl-3-(3-(pyrimidin-2-yl)-[1,2,4]triazolo[4,3-b]pyridazin-6-yl)propanamide |
| 153 (R) | 1-(pyrrolidin-1-yl)-3-(3-(thiazol-2-yl)-[1,2,4]triazolo[4,3-b]pyridazin-6-yl)propan-1-one |
| 154 (R) | 1-morpholino-3-(3-(thiazol-2-yl)-[1,2,4]triazolo[4,3-b]pyridazin-6-yl)propan-1-one |
| 155 (R) | ethyl 4-(3-(thiazol-2-yl)-[1,2,4]triazolo[4,3-b]pyridazin-6-yl)butanoate |
| 156 (R) | 1-(pyrrolidin-1-yl)-4-(3-(thiazol-2-yl)-[1,2,4]triazolo[4,3-b]pyridazin-6-yl)butan-1-one |
| 157 (R) | 1-morpholino-3-(3-(thiazol-2-yl)-[1,2,4]triazolo[4,3-b]pyridazin-6-yl)propan-1-one |
| 158 (R) | ethyl 4-(3-(thiophen-2-yl)-[1,2,4]triazolo[4,3-b]pyridazin-6-yl)butanoate |
| 159 (R) | 1-(pyrrolidin-1-yl)-4-(3-(thiophen-2-yl)-[1,2,4]triazolo[4,3-b]pyridazin-6-yl)butan-1-one |
| 160 (R) | N,N-dimethyl-4-(3-(thiophen-2-yl)-[1,2,4]triazolo[4,3-b]pyridazin-6-yl)butanamide |
| 161 (R) | 1-morpholino-4-(3-(thiophen-2-yl)-[1,2,4]triazolo[4,3-b]pyridazin-6-yl)butan-1-one |
| 162 (R) | 2-methyl-1-(pyrrolidin-1-yl)-3-(3-(thiophen-2-yl)-[1,2,4]triazolo[4,3-b]pyridazin-6-yl)propan-1-one |
| 163 (R) | 2-methyl-1-morpholino-3-(3-(thiophen-2-yl)-[1,2,4]triazolo[4,3-b]pyridazin-6-yl)propan-1-one |
| 164 (R) | N,N,2-trimethyl-3-(3-(thiophen-2-yl)-[1,2,4]triazolo[4,3-b]pyridazin-6-yl)propanamide |
| 165 (R) | 1-morpholino-3-(3-(thiophen-2-yl)-[1,2,4]triazolo[4,3-b]pyridazin-6-yl)propan-1-one |
| 166 (R) | N,N-dimethyl-3-(3-(thiophen-2-yl)-[1,2,4]triazolo[4,3-b]pyridazin-6-yl)propanamide |
| 167 (R) | 3-(pyrimidin-2-yl)-[1,2,4]triazolo[4,3-b]pyridazine-6-carboxylic acid |
| 168 (R) | 3-(thiazol-2-yl)-[1,2,4]triazolo[4,3-b]pyridazine-6-carboxamide |
| 169 (R) | 3-(thiazol-2-yl)-[1,2,4]triazolo[4,3-b]pyridazine-6-carboxylic acid |
| 170 (R) | N,N-diethyl-3-(pyrimidin-2-yl)-[1,2,4]triazolo[4,3-b]pyridazine-6-carboxamide |
| 171 (R) | azetidin-1-yl(3-(pyrimidin-2-yl)-[1,2,4]triazolo[4,3-b]pyridazin-6-yl)methanone |
| 172 (R) | N,N-diethyl-2-((3-(thiophen-2-yl)-[1,2,4]triazolo[4,3-b]pyridazin-6-yl)thio)butanamide |
| 173 (R) | N-methyl-N-phenyl-3-(pyrimidin-2-yl)-[1,2,4]triazolo[4,3-b]pyridazine-6-carboxamide |
| 174 (R) | piperidin-1-yl(3-(pyrimidin-2-yl)-[1,2,4]triazolo[4,3-b]pyridazin-6-yl)methanone |
| 175 (R) | N,N-dipropyl-3-(thiazol-2-yl)-[1,2,4]triazolo[4,3-b]pyridazine-6-carboxamide |
| 176 (R) | morpholino(3-(pyrimidin-2-yl)-[1,2,4]triazolo[4,3-b]pyridazin-6-yl)methanone |
| 177 (R) | (3-methylpiperidin-1-yl)(3-(pyrimidin-2-yl)-[1,2,4]triazolo[4,3-b]pyridazin-6-yl)methanone |
| 178 (R) | N-(2-(benzyloxy)pyridin-3-yl)-3-(pyrimidin-2-yl)-[1,2,4]triazolo[4,3-b]pyridazine-6-carboxamide |
| 179 (R) | N-(4-morpholinophenyl)-3-(pyrimidin-2-yl)-[1,2,4]triazolo[4,3-b]pyridazine-6-carboxamide |
| 180 (R) | N-(benzo[d][1,3]dioxol-5-ylmethyl)-3-(pyrimidin-2-yl)-[1,2,4]triazolo[4,3-b]pyridazine-6-carboxamide |
| 181 (R) | (R)-ethyl 2-(3-(pyrimidin-2-yl)-[1,2,4]triazolo[4,3-b]pyridazine-6-carboxamido)propanoate |
| 182 (R) | N-benzyl-3-(pyrimidin-2-yl)-[1,2,4]triazolo[4,3-b]pyridazine-6-carboxamide |
| 183 (R) | (S)-N-(1-amino-1-oxo-3-phenylpropan-2-yl)-3-(pyrimidin-2-yl)-[1,2,4]triazolo[4,3-b]pyridazine-6-carboxamide |
| 184 (R) | N-(2-morpholinoethyl)-3-(pyrimidin-2-yl)-[1,2,4]triazolo[4,3-b]pyridazine-6-carboxamide |
| 185 (R) | (R)-methyl 3-phenyl-2-(3-(pyrimidin-2-yl)-[1,2,4]triazolo[4,3-b]pyridazine-6-carboxamido)propanoate |
| 186 (R) | N-(pyridin-2-ylmethyl)-3-(pyrimidin-2-yl)-[1,2,4]triazolo[4,3-b]pyridazine-6-carboxamide |
| 187 (R) | (R)-N-(1-amino-1-oxo-3-phenylpropan-2-yl)-3-(pyrimidin-2-yl)-[1,2,4]triazolo[4,3-b]pyridazine-6-carboxamide |
| 188 (R) | (R)-4-methyl-2-(3-(pyrimidin-2-yl)-[1,2,4]triazolo[4,3-b]pyridazine-6-carboxamido)pentanoic acid |
| 189 (R) | (R)-N-(1-amino-4-methyl-1-oxopentan-2-yl)-3-(pyrimidin-2-yl)-[1,2,4]triazolo[4,3-b]pyridazine-6-carboxamide |
| 190 (R) | N-(cyclopropylmethyl)-3-(pyrimidin-2-yl)-[1,2,4]triazolo[4,3-b]pyridazine-6-carboxamide |
| 191 (R) | 1-(piperidin-1-yl)-2-((3-(thiophen-2-yl)-[1,2,4]triazolo[4,3-b]pyridazin-6-yl)sulfonyl)butan-1-one |
| 192 (R) | N-(1-benzylpiperidin-4-yl)-3-(thiazol-2-yl)-[1,2,4]triazolo[4,3-b]pyridazine-6-carboxamide |
| 193 (R) | 2-(N-methyl(3-(thiophen-2-yl)-[1,2,4]thazolo[4,3-b]pyridazin-6-yl)amino)-1-(piperidin-1-yl)ethanone |
| 194 (R) | 1-(piperidin-1-yl)-2-(3-(thiazol-2-yl)-[1,2,4]triazolo[4,3-b]pyridazin-6-yl)ethanone |
| 195 (R) | 1-morpholino-2-(3-(thiazol-2-yl)-[1,2,4]triazolo[4,3-b]pyridazin-6-yl)ethanone |
| 196 (R) | 2-(N-methyl(3-(thiophen-2-yl)-[1,2,4]triazolo[4,3-b]pyridazin-6-yl)amino)-1-(pyrrolidin-1-yl)ethanone |
| 197 (R) | N,N-diethyl-2-(3-(thiazol-2-yl)-[1,2,4]triazolo[4,3-b]pyridazin-6-yl)acetamide |
| 198 (R) | N,N-dimethyl-2-(3-(thiazol-2-yl)-[1,2,4]triazolo[4,3-b]pyridazin-6-yl)acetamide |
| 199 (R) | N-(pyridin-4-yl)-2-(3-(thiazol-2-yl)-[1,2,4]triazolo[4,3-b]pyridazin-6-yl)acetamide |
| 200 (R) | 1-(pyrrolidin-1-yl)-2-(3-(thiazol-2-yl)-[1,2,4]triazolo[4,3-b]pyridazin-6-yl)ethanone |
| 201 (R) | 2-(methyl(3-(thiophen-2-yl)-[1,2,4]triazolo[4,3-b]pyridazin-6-yl)amino)-1-morpholinoethanone |
| 202 (R) | N-((3s,5s,7s)-adamantan-1-yl)-2-((3-(thiophen-2-yl)-[1,2,4]triazolo[4,3-b]pyridazin-6-yl)thio)propanamide |
| 203 (R) | N,N-dipropyl-2-(3-(thiazol-2-yl)-[1,2,4]triazolo[4,3-b]pyridazin-6-yl)acetamide |
| 204 (R) | 2-(3-(1-methyl-1H-pyrazol-3-yl)-[1,2,4]triazolo[4,3-b]pyridazin-6-yl)acetic acid |
| 205 (R) | 1-(azetidin-1-yl)-2-(3-(thiazol-2-yl)-[1,2,4]triazolo[4,3-b]pyridazin-6-yl)ethanone |
| 206 (R) | 2-(3-(1-methyl-1H-pyrazol-3-yl)-[1,2,4]triazolo[4,3-b]pyridazin-6-yl)acetamide |
| 207 (R) | 2-(3-(1-methyl-1H-pyrazol-3-yl)-[1,2,4]triazolo[4,3-b]pyridazin-6-yl)-1-(piperidin-1-yl)ethanone |
| 208 (R) | 2-(3-(1-methyl-1H-pyrazol-3-yl)-[1,2,4]triazolo[4,3-b]pyridazin-6-yl)-1-morpholinoethanone |
| 209 (R) | (3-(thiophen-2-yl)-[1,2,4]triazolo[4,3-b]pyridazin-6-yl)methanamine |
| 210 (R) | morpholino(4-(3-(thiophen-2-yl)-[1,2,4]triazolo[4,3-b]pyridazin-6-yl)phenyl)methanone |
| 211 (R) | 2-(methyl(3-(thiophen-2-yl)-[1,2,4]triazolo[4,3-b]pyridazin-6-yl)amino)-1-(4-methylpiperazin-1-yl)ethanone |
| 212 (R) | 6-(methylthio)-3-(thiophen-2-yl)-[1,2,4]triazolo[4,3-b]pyridazine |
| 213 (R) | 1-(piperidin-1-yl)-4-(3-(thiophen-2-yl)-[1,2,4]triazolo[4,3-b]pyridazin-6-yl)butane-1,4-dione |
| 214 (R) | 3,3-difluoro-1-(piperidin-1-yl)-3-(3-(thiophen-2-yl)-[1,2,4]triazolo[4,3-b]pyridazin-6-yl)propan-1-one |
| 215 (R) | 4,4-difluoro-1-(piperidin-1-yl)-4-(3-(thiophen-2-yl)-[1,2,4]triazolo[4,3-b]pyridazin-6-yl)butan-1-one |
| 216 (R) | 3,3,3-trifluoro-1-(piperidin-1-yl)-2-((3-(thiophen-2-yl)-[1,2,4]triazolo[4,3-b]pyridazin-6-yl)thio)propan-1-one |
| 217 (R) | 3-fluoro-1-(piperidin-1-yl)-2-((3-(thiophen-2-yl)-[1,2,4]triazolo[4,3-b]pyridazin-6-yl)thio)propan-1-one |
| 218 (R) | 4-fluoro-1-(piperidin-1-yl)-2-((3-(thiophen-2-yl)-[1,2,4]triazolo[4,3-b]pyridazin-6-yl)thio)butan-1-one |
| 219 (R) | 1-(2-fluoropiperidin-1-yl)-2-((3-(thiophen-2-yl)-[1,2,4]triazolo[4,3-b]pyridazin-6-yl)thio)butan-1-one |
| 220 (R) | 1-(3-fluoropiperidin-1-yl)-2-((3-(thiophen-2-yl)-[1,2,4]triazolo[4,3-b]pyridazin-6-yl)thio)butan-1-one |
| 221 (R) | 1-(4-fluoropiperidin-1-yl)-2-((3-(thiophen-2-yl)-[1,2,4]triazolo[4,3-b]pyridazin-6-yl)thio)butan-1-one |
| 222 (R) | 2-((3-(5-fluorothiophen-2-yl)-[1,2,4]triazolo[4,3-b]pyridazin-6-yl)thio)-1-(piperidin-1-yl)butan-1-one |
| 223 (R) | 2-((3-(4-fluorothiophen-2-yl)-[1,2,4]triazolo[4,3-b]pyridazin-6-yl)thio)-1-(piperidin-1-yl)butan-1-one |
| 224 (R) | 2-((3-(3-fluorothiophen-2-yl)-[1,2,4]triazolo[4,3-b]pyridazin-6-yl)thio)-1-(piperidin-1-yl)butan-1-one |
| 225 (R) | 3-iodo-1-(piperidin-1-yl)-2-((3-(thiophen-2-yl)-[1,2,4]triazolo[4,3-b]pyridazin-6-yl)thio)propan-1-one |
| 226 (R) | 4-iodo-1-(piperidin-1-yl)-2-((3-(thiophen-2-yl)-[1,2,4]triazolo[4,3-b]pyridazin-6-yl)thio)butan-1-one |
| 227 (R) | 1-(2-iodopiperidin-1-yl)-2-((3-(thiophen-2-yl)-[1,2,4]triazolo[4,3-b]pyridazin-6-yl)thio)butan-1-one |
| 228 (R) | 1-(3-iodopiperidin-1-yl)-2-((3-(thiophen-2-yl)-[1,2,4]triazolo[4,3-b]pyridazin-6-yl)thio)butan-1-one |
| 229 (R) | 1-(4-iodopiperidin-1-yl)-2-((3-(thiophen-2-yl)-[1,2,4]triazolo[4,3-b]pyridazin-6-yl)thio)butan-1-one |
| 230 (R) | 2-((3-(5-iodothiophen-2-yl)-[1,2,4]triazolo[4,3-b]pyridazin-6-yl)thio)-1-(piperidin-1-yl)butan-1-one |
| 231 (R) | 2-((3-(4-iodothiophen-2-yl)-[1,2,4]triazolo[4,3-b]pyridazin-6-yl)thio)-1-(piperidin-1-yl)butan-1-one |
| 232 (R) | 2-((3-(3-iodothiophen-2-yl)-[1,2,4]triazolo[4,3-b]pyridazin-6-yl)thio)-1-(piperidin-1-yl)butan-1-one |

### SYNTHESIS

Compounds according to the present disclosure can, for example, be prepared by the methods outlined below. A practitioner skilled in the art will understand the appropriate use of protecting groups [see: Greene and Wuts, *Protective Groups in Organic Synthesis*] and the preparation of known compounds found in the literature using the standard methods of organic synthesis. There may come from time to time the need to rearrange the order of the recommended synthetic steps, however this will be apparent to the judgment of a chemist skilled in the art of organic synthesis.

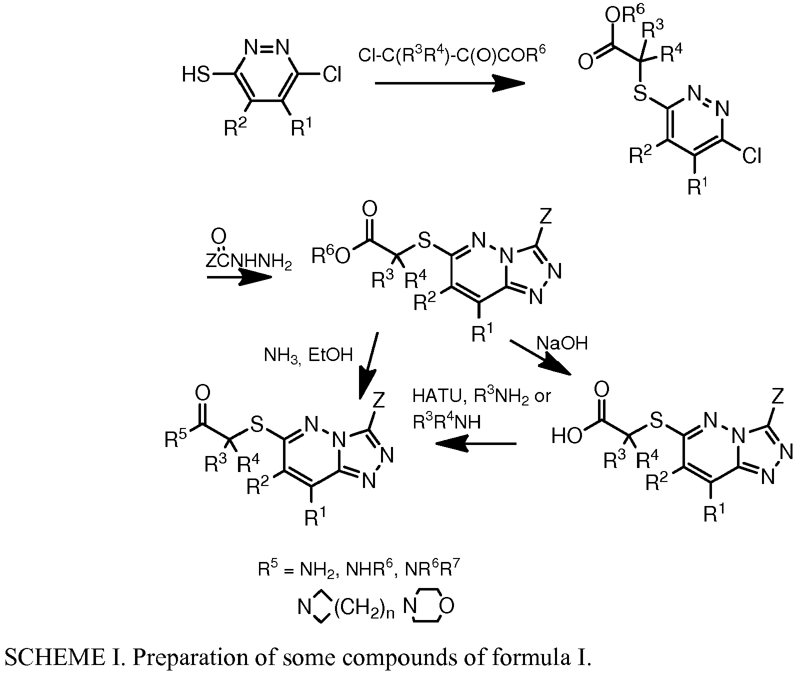

Compounds of formula I found in this disclosure can be prepared by a variety of methods; one example is illustrated in Scheme I for compounds where X = -C(R³R⁴)C(O)OH,-C(R³R⁴)C(O)OR⁵, -C(R³R⁴)C(O)NR⁶R⁷, -C(R³R⁴)C(O)NHR⁶ or -C(R³R⁴)C(O)NH₂. Commercially available 3-chloro-5-thiopyridazine [or an analog with substituted R¹ or R²] is alkylated with a 1-chloroacetic acid ester analog which may be substituted at R³ and R⁴ using a variety of bases (Na₂CO₃, NaOH, Et₃N) in solvents such as DMF, acetone, butanone, THF, EtOH and the like at a temperature range of -10 °C to reflux temperature. Transfromation of the resulting alkylation product to the triazolopyridazine can be managed by heating up to reflux temperature in an alcohol solvent such as butanol or ethanol a mixture of the chloropyridazine with an acylhydrazide substituted with the required group Z as described in formula I. This results in a concomitant N-alkylation of the chloropyridazine followed by a cyclocondensation to the triazolopyridazine product.

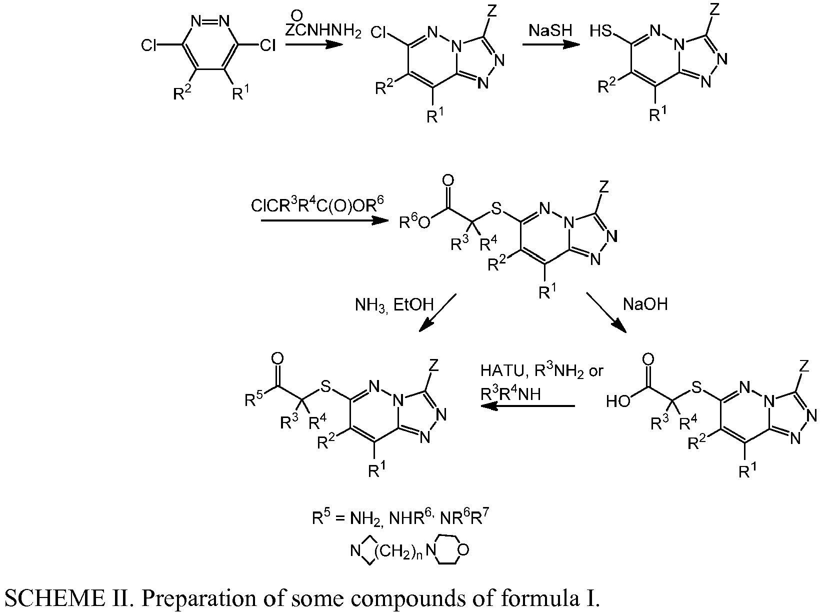

Scheme II illustrates an alternative approach where the order of the first three steps is interchanged from the order described in Scheme I. This method leads to the synthesis of the 6-chlorotriazolopyridazine which is a convenient intermediate for the preparation of a variety of analogs [Scheme III].

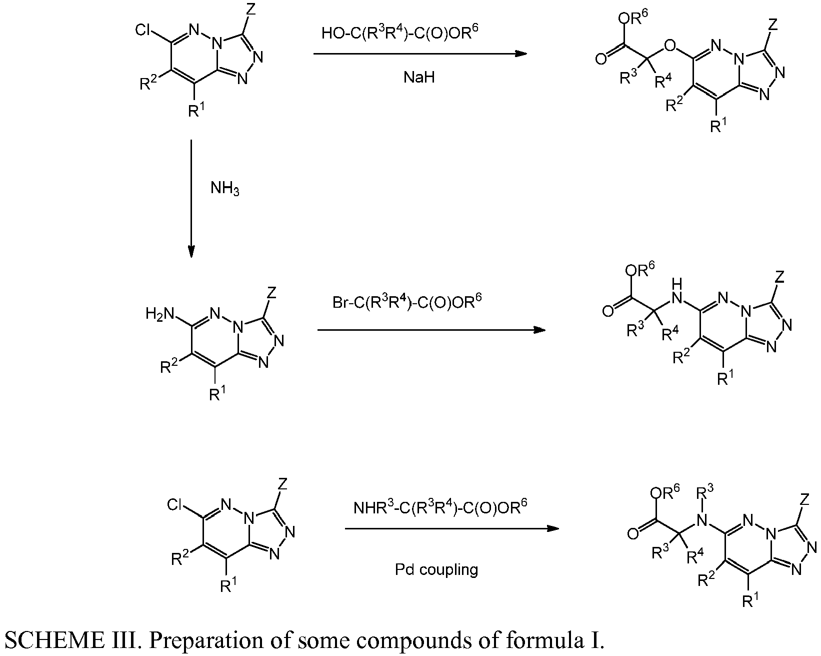

The 6-chlorotriazolopyridazine intermediate serves as a starting point for compounds where the thioether linkage is replaced by an ether linkage or an amine. The sodium salt of a hydroxyacetic acid ester analog prepared from NaH in dry DMF is stirred with cooling until the starting material is consumed. The resulting product is an -O- linked ester which can be hydrolyzed to the acid and coupled with an amine to give amides as described in Schemata I and II. Elaboration of 6-chlorotriazolopyridazine to an amine linked compound can best be realized by treating the chloro compound with neat distilled, liquid ammonia in a pressure bomb at high temperature and pressure until all of the 6-chlorotriazolopyridazine is consumed. The resulting 6-NH₂ analog is alkylated with an alpha-haloacetic acid ester analog to give an ester product which can be transformed to the acid and amide as described before. Finally metal coupling chemistry championed by Buchwald or by Chan and Lam would be useful for the direct transformation of the 6-chlorotriazolopyridazine to the N-linked esters with a judicious choice of conditions.

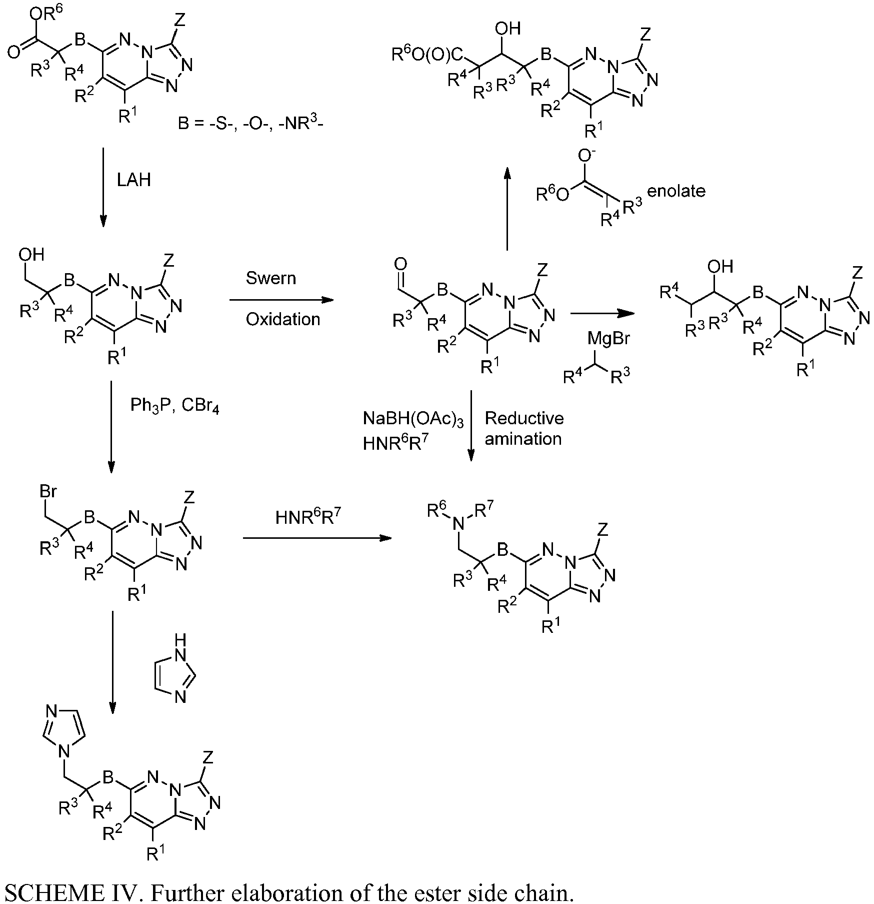

The triazolopyridazine with an ester side chain linked to it by a -S-, -O-, -N(R³)- provides a convenient starting point for many analogs of formula I. The terminal ester can be converted to an alcohol group by a reducing agent such as lithium aluminum hydride (LAH) and the like, in an ethereal solvent such as THF, Et₂O or dioxane at temperatures ranging from -78 °C to reflux temperature of the solvent. Three paths are open to the utilization of this alcohol: i) transformation into a leaving group such as a halogen (POCl₃ or CCl₄/Ph₃P for chloro-derivatives; PBr₃ or CBr₄/Ph₃P for bromo-derivatives etc.) or a sulfonate ester (methane sulfonyl chloride/ CH₂Cl₂/ Et₃N or p-toluene sulfonyl chloride/pyridine) then displacement with an amine H₂NR⁶, HNR⁶R⁷ (in a non-polar aprotic solvent such as DMF or DMSO, alternatively an alcoholic solvent such as EtOH or n-BuOH may be used, at temperatures ranging from 0 °C to reflux) or ammonia (liquid ammonia, pressure bomb with temperatures ranging from ambient to 200 °C); ii) the alcohol group is transformed into a leaving group as described above and subsequently displaced with a substituted or unsubstituted heterocycle (provided N remains unsubstituted) such as imidazole, pyrazole, triazoles, tetrazole, indole, indazole, benzimidazole in polar protic an aprotic solvents such as EtOH, n-BuOH, DMF or DMSO at temperatures ranging from 0 °C to the reflux temperature of the solvent, bases such as Na₂CO₃, KOH, Et₃N or Hunigs base may be used as well; iii) the alcohol may be oxidized to an aldehydes with reagents such as PDC or PCC in dichloromethane or the Swern oxidation (dichloromethane, DMSO, oxalyl chloride, then Et₃N). A practitioner skilled in art of organic chemical synthesis will recognize that the derived aldehydes are particularly useful intermediates. They may be transformed into amines by reductive amination under a variety of conditions with an amine such as NH₃, H₂NR⁶ or HNR⁶R⁷ with reagents systems such as: H₂(g) and Pd-C catalyst in alcohol solvent, NaBH(OAc)₃, CH₂Cl₂, NaBH₃(CN) etc. A second use is for enolate condensation chemistry with an ester or ketone with strong hindered base such as lithium diisopropylamide in a solvent such as THF, Et₂O or dioxane at temperatures ranging from -78 °C to ambient temperature. A third use is for addition by alkyl or aryl lithium or alkyl or aryl Grignard reagents or other metallated alkyl, aryl, heteroalkyl or heteroaryl species. Suitable solvents for these reactions include THF, Et₂O or dioxane; reaction temperatures range from -78 °C to reflux temperature of the solvent.

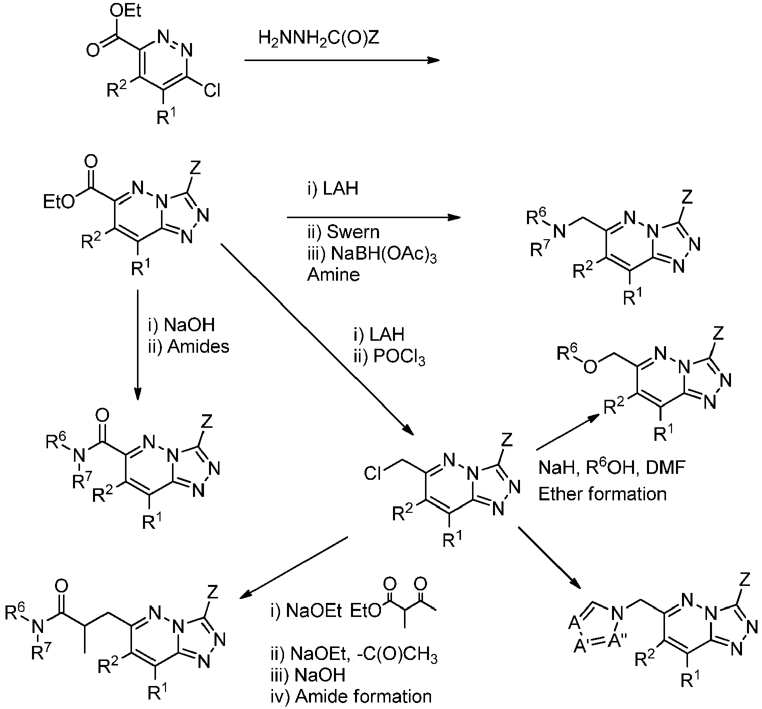

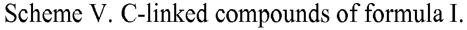

The commercially available ethyl 3-carboxyl-5-chloropyridazine is reacted with the appropriate acylhydrazide under conditions similar to those described previously. The resulting ethyl 6-carboxyltriazolopyridazine can be transformed to an amide by first hydrolysis of the ester functionality under basic or acidic conditions, then using methods described above, transformed to the amide with a peptide coupling reagent, a trialkyl amine base, in DMF with an appropriate amine. Reduction of the ester and oxidation of the resulting alcohol gives an aldehyde that can undergo reductive amination with an appropriate amine under the conditions described previously. The further utility of aldehydes of this type is described further in Scheme IV. Transformation of the alcohol resulting from reduction of the ester to a primary halo-compound gives an alkylating that can be used to prepare amine analogs or heteroaryl analogs and also homolgate the side chain by alkylation with a 2-alkyl acetoacetate ester with NaOEt in EtOH. The acetyl group is removed by a retroaldol in refluxing NaOEt in EtOH. The resulting acid is ready for amide formation using any of the conditions discussed previously. The primary halo-compound is also a useful intermediate for the formation of ethers using Williamson ether synthesis conditions.

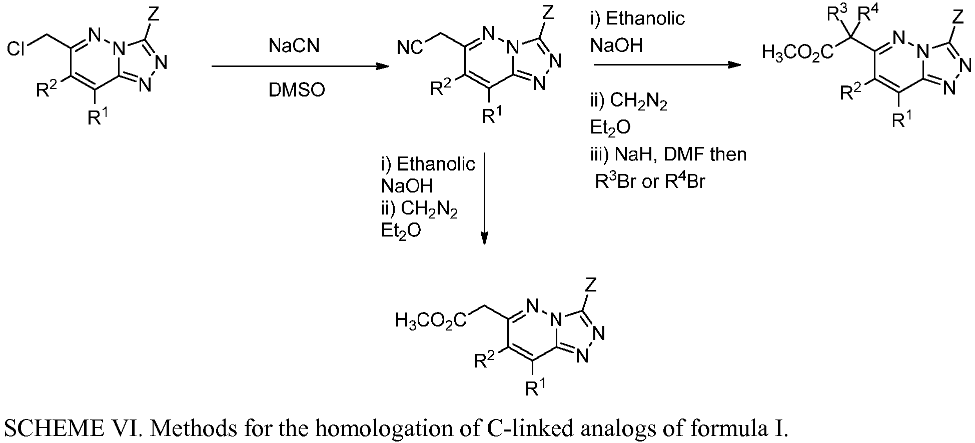

Scheme VI describes methodology for the homologation of the C-linked side chain by treating the primary halo-compound prepared in Scheme V with excess NaCN in DMSO or DMF at temperatures ranging from ambient to 60 °C. The resulting cyano compound is hydrolyzed to the acid with hot ethanolic NaOH, then treated with ethereal diazomethane to give a methyl ester. This ester can then be alkylated with appropriate alkyl halides to give subsitutent R³ or R⁴ as desired. The chemist also has the option of not alkylating the ester as illustrated. In both cases the resulting esters may be transformed to various compounds of formula I using the chemistry illustrated in Schemata I through V.

A variety of general methods are available for the preparation of fluorine containing compounds and one having ordinary skill in the art can readily adapt such methods for preparing ¹⁸F labeled analogs. Customarily, because ¹⁸F has a half-life (T_{1/2}) of ∼120 minute, the ¹⁸F label is introduced in the last step of the compound synthesis, purified and used immediately as a tracer in Postitron Emission Tomography. Typical procedures to accomplish this involve the SN₂ displacement of a bromine atom or sulfonate ester such as -OSO₂CF₃, -OSO₂CH₃ or-OSO₂C₆H₄CH₃ and the like by radioactive ¹⁸F anion generated locally by a cyclotron. The same strategy can be used for introducing an iodine radiolabel, i.e., by SN₂ displacement of a sulfonate ester analog.

Typical conditions to effect the ¹⁸F anion displacement are: Bu₄N ¹⁸F [VanBrocklin, Henry F. et al., Steroids, 59(1), 34-45; 1994]; a mixture comprising 2,2,2-cryptand, ¹⁸F⁻, K₂CO₃, and solvent (H₂O, MeCN), is reacted for 10 min at 120 °C [WO 2009/052970]; a mixture comprising K¹⁸F, 2,2,2-cryptand, DMSO, is reacted for 20 min at 120 °C [Nag, Sangram et al., Journal of Medicinal Chemistry, 54(20), 7023-7029; 2011]. Radiolabeled iodine can be intorduced using analogous conditions.

It is also contemplated that from time to time a set of enantiomers or diasteromers may be produced by the action of generating a chiral center(s) during synthesis. These compounds may be separated by means of chromatography, flash chromatography, HPLC or the like, with chiral columns in the case of enantiomers and sometimes diastereomers, or separated by means of crystallization or prepared enantiomerically pure by the judicious selection of synthetic methods. All of these processes are known to persons skilled in the art of organic synthesis and need not be described herein.

### EXPERIMENTAL

### Preparation of ethyl 2-((6-chloropyridazin-3-yl)thio)propanoate

The synthesis of ethyl 2-((6-chloropyridazin-3-yl)thio)propanoate starts with commercially available 2-chloro-6-thiopyridazine which was added to an aqueous basic solution such as NaOH solution and the mixture was stirred for approximately 10 -30 min before a solution of ethyl chloroacetate (837 mg, 7.35 mmol) in a polar solvent such as acetone was added dropwise over 10 - 30 min. Stirring was continued for 1-2 h, and then the reaction was allowed to stand atroom temperature. The resulting solid was collected by filtration, washed with water, and then dried to give ethyl 2-((6-chloropyridazin-3-yl)thio)propanoate.

### EXAMPLE 2: Ethyl 2-((3-(thiophen-2-yl)-[1,2,4]triazolo[4,3-b]pyridazin-6-yl)thio) acetate

To 2-thiophenecarbohydrazide (215 mg, 1.5 mmol) dissolved in n-BuOH (3 mL) was added ethyl 2-((6-chloropyridazin-3-yl)thio)propanoate (232 mg, 1 mmol), and the whole was heated for 5 h in a 140-145 °C oil bath. The reaction mixture was then cooled to room temperature (∼ 25 °C), and the solid residue removed by filtration. The filtrate was then diluted with methylene chloride (50 mL), and washed with saturated with NaHCO₃ (∼15 mL). The combined aqueous layers were washed twice with methylene chloride, and the organic layers were then combined, washed with brine, dried over MgSO₄, filtered, and evaporated. The resulting solid was then recrystallized from EtOH to give ethyl 2-((3-(thiophen-2-yl)-[1,2,4]triazolo[4,3-b]pyridazin-6-yl)thio)acetate.

The following compounds were prepared by an adaptation of this method (examples 1 and 2 are reference examples):

| Example | MS | NMR |
|---|---|---|
| 1 | calculated for C₁₇H₁₇FN₄O₂S·H was 361.1129 found 361.1129 | 8.36-8.42 (m, 2H, Ph), 8.31 (d, 1H, pyridazine, J = 9.8 Hz), 7.42-7.48 (m, 2H, Ph), 7.39 (d, 1H, pyridazine, J = 9.8 Hz), 4.53 (t, 1H, SCH, J = 6.8 Hz), 4.08-4.16 (dq, 1H, OCH2, J = 7.1 Hz and 11.0 Hz), 3.95-4.04 (d q, 2H, OCH₂, J = 7.0 Hz and 10.9 Hz), 1.98-2.09 (m, 2H, CH₂), 1.06 (t, 3H, CH₃, J = 7.0 Hz), 1.04 (t, 3H, CH₃, J = 9.5 Hz). |
| 2 | calculated for C₁₄H₁₃N₅O₂S·H was 316.0863 found 316.0858 | 8.79-8.82 (d d, 2H, J = 1.6, 4.3 Hz), 8.38 (d, 1H, pyridazine, J = 9.8 Hz), 8.28-8.31 (d d, 2H, Ph, J =1.5, 4.3 Hz), 7.51 (d, 1H, pyridazine, J = 9.8 Hz), 4.29 (s, 2H, SCH₂), 4.05 (q, 2H, OCH₂, J = 7.5 Hz), 1.07 (t, 3H, CH₃, J = 7.1 Hz). |

### REFERENCE EXAMPLE 27: 2-((3-(Thiophen-2-yl)-[1,2,4]triazolo[4,3-b]pyridazin-6-yl)thio)acetic acid

To ethyl 2-((3-(thiophen-2-yl)-[1,2,4]triazolo[4,3-b]pyridazin-6-yl)thio)acetate (50 mg, 0.156 mmol) dissolved in a 2/1 mixture of THF and MeOH was added 100 uL of 2N NaOH, and the whole was stirred at room temperature for 3 h. The reaction mixture was then evaporated to dryness, the residue dissolved in water (5 mL), and the solution acidified with 6M HCl. The resulting precipitate was filtered, washed twice with cold water, and dried to give acid 2-((3-(thiophen-2-yl)-[1,2,4]triazolo[4,3-b]pyridazin-6-yl)thio)acetic acid.

The following compounds were prepared by an adaptation of this method (example 36 is a compound of the invention and the remaining examples are reference examples):

| Example | MS | NMR |
|---|---|---|
| 6 | calculated for C₁₅H₁₃FN₄O₂S·H was 333.0816 found 333.0813 | 13.20 (br s, 1H, OH), 8.39-8.44 (m, 2H, Ph), 8.30 (d, 1H, pyridazine, J = 9.8 Hz), 7.40-7.46 (m, 2H, Ph), 7.37 (d, 1H, pyridazine, J = 9.8 Hz), 4.41 (t, 1H, SCH, J = 6.6 Hz), 1.98-2.09 (m, 2H, CH₂), 1.05 (t, 3H, CH₃, J = 7.2 Hz). |
| 13 | calculated for C₁₄H₁₂N₄O₂S·H was 301.0754 found 301.0752 | 13.04 (br s, 1H, OH), 8.28 (d, 1H, pyridazine, J = 9.8 Hz), 8.24-8.29 (mult app d), 1H, Ph, J = 8.2 Hz), 7.39 (d br d, 2H, Ph, J = 8.2 Hz), 7.39 (d, 1H, pyridazine, J = 9.4 Hz), 4.13 (s, 2H, SCH), 2.41 (s, 3H, CH₃). |
| 17 | calculated for C₁₅H₉FN₄O₂S·H was 305.0503 found 305.0499 | 13.08 (br s, 1H, OH), 8.38-8.44 (m, 2H, Ph), 8.29 (d, 1H, pyridazine, J = 9.8 Hz), 7.41 (d, 1H, pyridazine, J = 9.8 Hz), 7.37-7.43 (m, 2H, Ph), 4.12 (s, 2H, SCH₂). |
| 18 | calculated for C₁₃H₁₂N₄O₂S₂·H was 321.0474 found 321.0475 | 13.26 (br s, 1H, OH), 8.29 (d, 1H, pyridazine, J = 9.4 Hz), 8.20 (d d, 1H, thienyl, J = 1.1, 3.9 Hz), 7.87 (d d, 1H, thienyl, J = 1.1, 5.0 Hz), 7.37 (d, 1H, pyridazine, J = 9.8 Hz), 7.32 (d d, 1H, thienyl, J = 3.6, 4.7 Hz), 4.57 (t, 1H, SCH, J = 6.6 Hz), 2.04-2.24 (m, 2H, CH₂), 1.07 (t, 3H, CH₃, J = 7.5 Hz). |
| 27 | calculated for C₁₁H₈NaO₂S₂·H was 293.0161 found 293.0158 | 13.09 (br s, 1H, OH), 8.29 (d, 1H, pyridazine, J = 9.8 Hz), 8.19 (d d, 1H, thienyl, J = 1.2, 3.9 Hz), 7.86 (d d, 1H, benzo, J = 1.2, 5.1 Hz), 7.41 (d, 1H, pyridazine, J = 9.4 Hz), 7.30 (d d, 1H, benzo, J = 3.5, 5.0 Hz), 4.21 (s, 2H, SCH). |
| 28 | calculated for C₁₁H₈N₄O₃S·H was 277.0390 found 277.0395 | 13.08 (br s, 1H, OH), 8.30 (d, 1H, pyridazine, J = 9.8 Hz), 8.00-8.04 (m, 1H, furan), 7.53 (d, 1H, furan, J = 3.5 Hz), 7.53 (d, 1H, pyridazine, J = 9.8 Hz), 6.80 (d d, 1H, furan, J = 2.0, 3.5 Hz), 4.17 (s, 2H, SCH). |
| 30 | calculated for C₁₅H₁₀N₄O₂S₂·H was 343.0318 found 343.0320 | 13.24 (br s, 1H, OH), 8.57 (s, 1H, thienyl), 8.33 (d, 1H, pyridazine, J = 9.7 Hz), 8.06-8.12 (m, 1H, benzo), 7.96-8.01 (m, 1H, benzo), 7.46-7.51 (m, 3H, benzo), 4.26 (s, 2H, SCH). |
| 31 | calculated for C₁₅H₁₀N₄O₃S·H was 327.0546 found 327.0552 | 13.24 (br s, 1H, OH), 8.36 (d, 1H, pyridazine, J = 9.4 Hz), 7.99 (s, 1H, furanyl),7.77-7.82 (m, 2H, benzo), 7.51 (d, 1H, pyridazine, J = 9.8 Hz), 7.48 (t, 1H, benzo, J = 8.2 Hz), 7.39 (t, 1H, benzo, J = 7.7 Hz), 4.25 (s, 2H, SCH). |
| 33 | calculated for C₁₂H₁₁N₅O₂S₂·H was 322.0427 found 322.0423 | 13.27 (br s, 1H, OH), 8.38 (d, 1H, pyridazine, J = 9.3 Hz), 8.20 (d, 1H, thiazole, J = 3.1 Hz), 8.16 (d, 1H, thiazole, J = 3.2 Hz), 7.47 (d, 1H, pyridazine, J = 9.4 Hz), 4.71 (t, 1H, SCH, J = 6.5 Hz), 2.06-2.16 (m, 2H, CH₂), 1.06 (t, 3H, CH₃, J = 7.1 Hz). |
| 36 | calculated for C₁₂H₁₀N₄O₂S₂·H was 307.0318 found 307.0321 | 8.28 (d, 1H, pyridazine, J = 9.7 Hz), 8.21 (d d, 1H, thienyl, J = 1.1, 3.7 Hz), 7.86 (d d, 1H, thienyl, J = 1.2, 5.1 Hz), 7.35 (d, 1H, pyridazine, J = 9.7 Hz), 7.31 (d d, 1H, thienyl, J = 3.9, 5.1 Hz), 3.63 (q, 1H, SCH, J = 7.5 Hz), 1.68 (d, 3H, CH₃, J = 7.1 Hz). |
| 38 | calculated for C₁₅H₁₀N₄O₂S₂·H was 343.0318 found 343.0321 | 9.02-9.05 (m, 1H, benzo), 9.01 (s, 1H, thienyl), 8.35 (d, 1H, pyridazine, J = 9.4 Hz), 8.15-8.18 (m, 1H, benzo), 7.59-7.62 (m, 1H, benzo), 7.51-7.56 (m, 1H, benzo), 7.47 (d, 1H, pyridazine, J = 9.4 Hz), 4.22 (s, 2H, SCH). |
| 39 | calculated for C₁₈H₁₄N₄O₂S₂·H was 383.0631 found 383.0632 | 8.16 (d, 1H, pyridazine, J = 9.7 Hz), 8.09 (s, 1H, thienyl), 7.28 d, 1H, pyridazine, J = 9.8 Hz), 7.06-7.42 (Ph), 3.99 (s, 2H, SCH₂). |
| 41 | calculated for C₁₅H₁₆N₄O₂S₂·H was 349.0787 found 349.0791 | 8.18 (br s, 1H, thienyl), 8.13 (d, 1H, pyridazine, J = 9.8 Hz), 7.80 (d, 1H, thienyl, J = 5.1 Hz), 7.26-7.32 (m, 1H, thienyl), 7.23 (d, 1H, pyridazine, J = 9.8 Hz), 4.46 (br s, 1H, SCH), 1.95-2.08 (m, 2H, CH₂), 1.46 (br s, 2H, CH₂), 1.10-1.38 (m, CH₂), 0.60-0.80 (m, CH₃). |
| 42 | calculated for C₁₀H₇N₅O₂S₂·H was 294.0114 found 294.0114 | 13.00 (br s, 1H, OH), 8.38 (d, 1H, pyridazine, J = 9.8 Hz), 8.20 (d, 1H, thiazole, J = 3.2 Hz), 8.16 (d, 1H, thiazole, J = 3.1 Hz), 7.52 (d, 1H, pyridazine, J = 9.4 Hz), 4.26 (s, 2H, SCH₂). |
| 61 | calculated for C₁₃H₁₁ClN₄O₂S₂· H was 355.0085 found 355.0090 | 8.29 (d, 1H, pyridazine, J = 9.8 Hz), 8.03 (d, 1H, thienyl, J = 3.9 Hz), 7.39 (d, 1H, pyridazine, J = 9.8 Hz), 7.35 (d, 1H, thienyl, J = 4.3 Hz), 4.52 (t, 1H, SCH, J = 6.7 Hz), 2.06 (quint, 2H, CH₂, J = 7.2 Hz), 1.07 (t, 3H, CH₃, J = 7.3 Hz). |
| 88 | calculated for C₁₃H₁₁ClN₄O₂S₂· H was 349.0787 found 349.0793 | 8.26 (d, 1H, pyridazine, J = 9.7 Hz), 8.02 (d, 1H, thienyl, J = 3.5 Hz), 7.34 (d, 1H, pyridazine, J = 9.4 Hz), 7.04 (d, 1H, thienyl, J = 3.9 Hz), 4.53 (t, 1H, SCH, J = 6.7 Hz), 2.92 (q, 2H, CH₂, J = 7.6 Hz), 2.07 (quint, 2H, CH₂, J = 7.2 Hz), 1.32 (t, 3H, CH₃, J = 7.4 Hz), 1.08 (t, 3H, CH₃, J = 7.3 Hz). |
| 89 | calculated for C₁₄H₁₄N₄O₂S₂·H was 335.0631 found 335.0687 | 8.26 (d, 1H, pyridazine, J = 9.3 Hz), 8.00 (d, 1H, thienyl, J = 3.5 Hz), 7.34 (d, 1H, pyridazine, J = 9.4 Hz), 7.01 (d d, 1H, thienyl, J = 1.2, 3.5 Hz), 4.52 (t, 1H, SCH, J = 6.8 Hz), 2.56 (s, 3H, CH₃), 2.09 (quint, 2H, CH₂, J = 7.2 Hz), 1.07 (t, 3H, CH₃, J = 7.3 Hz). |
| 92 | calculated for C₁₄H₁₂N₄O₂S₂·H was 335.0631 found 335.0633 | 13.37 (br s, 1H, OH), 8.29 (d, 1H, pyridazine, J = 9.8 Hz), 8.21 (d d, 1H, thienyl, J = 1.1, 3.9 Hz), 7.87 (d d, 1H, thienyl, J = 1.2, 5.1 Hz), 7.37 (d, 1H, pyridazine, J = 9.7 Hz), 7.32 (d d, 1H, thienyl, J = 3.5, 5.0 Hz), 4.58 (t, 1H, SCH, J = 6.8 Hz), 1.95-2.06 (m, 2H, CH₂), 1.45-1.56 (m, 2H, CH₂), 0.93 (t, 3H, CH₃, J = 7.3 Hz). |

### REFERENCE EXAMPLE 3: 2-((3-(Thiophen-2-yl)-[1,2,4]triazolo[4,3-b]pyridazin-6-yl)thio)acetamide

Ethanolic ammonia (5 mL) was added to intermediate ethyl 2-((3-(thiophen-2-yl)-[1,2,4]triazolo[4,3-b]pyridazin-6-yl)thio)acetate (60 mg, 0.188 mmol), the reaction vessel sealed and the mixture stirred overnight at 90-95 °C. The mixture was then evaporated to dryness, the solid residue was filtered, washed 3 times with EtOH, and dried to give the desired 2-((3-(thiophen-2-yl)-[1,2,4]triazolo[4,3-b]pyridazin-6-yl)thio)acetamide.

The following compounds were prepared by an adaptation of this method (the following examples are reference examples):

| Example | MS | NMR |
|---|---|---|
| 6 | calculated for C₁₁H₉N₅OS₂·H was 292.0321 found 292.0320 | 8.26 (d, 1H, pyridazine, J = 9.8 Hz), 8.23 (d d, 1H, thienyl, J = 1.1, 3.9 Hz), 7.83 (d d, 1H, thienyl, J = 1.2, 5.1 Hz), 7.79 (br s, 1H, NH₂), 7.39 (d, 1H, pyridazine, J = 6.5 Hz), 7.33 (br s, 1H, NH₂), 7.30 (d d, 1H, thienyl, J = 3.9, 5.1 Hz), 4.07 (s, 2H, SCH₂). |
| 4 | calculated for C₁₂H₁₀N₆OS·H was 287.0710 found 287.0709 | 8.84 (d d, 2H, pyridyl, J = 1.9, 4.7 Hz), 8.36 (d d, 2H, pyridyl, J = 1.6, 4.7 Hz), 8.35 (d, 1H, pyridazine, J = 9.8 Hz), 7.80 (br s, 1H, NH), 7.47 (d, 1H, pyridazine, J = 9.8 Hz), 7.37 (br s, 1H, NH₂), 4.04 (s, 2H, SCH₂). |
| 5 | calculated for C₁₁H₉N₅OS₂·H was 292.0321 found 292.0320 | 8.66 (dd, 1H, thienyl, J = 1.2, 1.5 Hz), 8.26 (d, 1H, pyridazine, J = 9.4 Hz), 7.94 (d d, 1H, thienyl, J = 1.1, 5.1 Hz), 7.79 (d d, 1H, thienyl, J = 2.7, 3.0 Hz), 7.77.77-7.84 (m, 1H, NH₂), 7.38 (d, 1H, pyridazine, J = 9.8 Hz), 7.35 (br s, 1H, NH₂), 4.06 (s, 2H, SCH₂). |
| 10 | calculated for C₁₃H₁₇N₅OS·H was 292.1227 found 292.1226 | 8.12 (d, 1H, pyridazine, J = 9.4 Hz), 7.67 (br s, 1H, NH₂), 7.25 (d, 1H, pyridazine, J = 9.8 Hz), 7.25 (br s, 1H, NH₂), 3.91 (s, 2H, SCH₂), 3.22-3.31 (m, 1H, cyclohexyl), 1.97-2.05 (m, 2H, cyclohexyl), 1.78-1.88 (m, 2H, cyclohexyl), 1.68-1.75 (m, 2H, cyclohexyl), 1.63-1.69 (m, 2H, cyclohexyl), 1.30-1.52 (m, 2H, cyclohexyl). |
| 15 | calculated for C₁₂H₁₅N₅OS·H was 278.1070 found 278.1068 | 8.12 (d, 1H, pyridazine, J = 9.4 Hz), 7.68 (br s, 1H, NH₂), 7.25 (d, 1H, pyridazine, J = 9.7 Hz), 7.25 (br s, 1H, NH₂), 3.92 (s, 2H, SCH₂), 3.62 (quint, 1H, cyclopentyl CH, J = 8.1 Hz), 2.09-2.19 (m, 2H, cyclopentyl), 1.90-2.00 (m, 2H, cyclopentyl), 1.75-1.86 (m, 2H, cyclopentyl), 1.65-1.75 (m, 2H, cyclopentyl). |
| 16 | calculated for C₁₂H₁₀N₆OS·H was 287.0710 found 287.0705 | 8.78-8.82 (m, 1H, pyridyl), 8.38 (d, 1H, pyridyl, J = 7.9 Hz), 8.32 (d, 1H, pyridyl, J = 9.7 Hz), 8.07 (d t, 1H, pyridyl, J = 1.8, 7.7 Hz), 7.93 (br s, 1H, NH), 7.58-7.62 (m, 1H, pyridyl), 7.44 (d, 1H, pyridazine, J = 9.7 Hz), 7.33 (br s, 1H, NH₂), 3.90 (s, 2H, SCH₂). |
| 19 | calculated for C₁₃H₁₀FN₅OS·H was 304.0663 found 304.0603 | 8.42-8.49 (m, 2H, Ph), 8.27 (d, 1H, pyridazine, J = 9.8 Hz), 7.75 (br s, 1H, NH₂), 7.41-7.48 (m, 2H, Ph), 7.39 (d, 1H, pyridazine, J = 9.7 Hz), 7.34 (br s, 1H, NH₂), 4.00 (s, 2H, SCH₂). |
| 26 | calculated for C₁₁H₉N₅O₂S·H was 276.0550 found 276.0551 | 8.27 (d, 1H, pyridazine, J = 9.8 Hz), 8.01 (m, 1H, furanyl, J = 9.4 Hz), 7.77 (br s, 1H, NH₂), 7.58 (d d, 1H, furanyl, J = 0.8, 3.5 Hz), 7.39 (d, 1H, pyridazine, J = 9.8 Hz), 7.33 (br s, 1H, NH₂), 6.78 (dd, 1H, furanyl, J = 1.6, 3.5 Hz), 4.04 (s, 2H, SCH₂). |
| 29 | calculated for C₁₅H₁₁N₅OS₂·H was 342.0478 found 342.0480 | 8.61 (s, 1H, thienyl), 8.32 (d, 1H, pyridazine, J = 9.8 Hz), 8.07-8.13 (m, 2H, benzo), 7.89 (br s, 1H, NH₂), 7.46-7.52 (m, 2H, benzo), 7.46 (d, 1H, pyridazine, J = 9.4 Hz), 7.45 (br s, 1H, NH₂), 4.14 (s, 2H, SCH₂). |
| 32 | calculated for C₁₅H₁₁N₅O₂S·H was 326.0706 found 326.0711 | 8.34 (app d, 1H, pyridazine, J = 9.8 Hz), 8.05 (br s, 1H, furan), 7.89 (br s, 1H, NH₂), 7.84-7.87 (m, 1H, benzo), 7.48 (d, 1H, pyridazine, J = 9.7 Hz), 7.45-7.50 (m, 1H, benzo), 7.46 (br s, 1H, NH₂), 7.39 (app t, 1H, benzo, J = 7.5 Hz), 4.13 (s, 2H, SCH₂). |
| 35 | calculated for C₁₅H₁₁N₅OS₂·H was 342.0478 found 342.0476 | 9.09 (s, 1H, thienyl), 9.06-9.08 (m, 1H, benzo), 8.34 (d, 1H, pyridazine, J = 9.4 Hz), 8.14-8.17 (m, 1H, benzo), 7.89 (br s, 1H, NH₂), 7.57-7.62 (m, 1H, benzo), 7.50-7.55 (m, 1H, benzo), 7.44 (d, 1H, pyridazine, J = 9.4 Hz), 7.36 (br s, 1H, NH₂), 4.09 (s, 2H, SCH₂). |
| 37 | calculated for C₁₀H₈N₆OS₂·H was 293.0273 found 293.0273 | 8.37 (d, 1H, pyridazine, J = 9.7 Hz), 8.19 (d, 1H, thiazole, J = 3.5 Hz), 8.10 (d, 1H, thiazole, J = 3.1 Hz), 7.86 (br s, 1H, NH₂), 7.51 (d, 1H, pyridazine, J = 9.8 Hz), 7.33 (br s, 1H, NH₂), 4.09 (s, 2H, SCH₂). |
| 40 | calculated for C₁₈H₁₅N₅OS₂·H was 382.0791 found 382.0792 | 8.42 (s, 1H, thienyl), 8.13 (d, 1H, pyridazine, J = 9.8 Hz), 7.67 (br s, 1H, NH₂), 7.24-7.31 (m, 4H, Ph and NH₂), 7.24 (d, 1H, pyridazine, J = 9.8 Hz), 7.45 (br s, 1H, NH₂), 3.85 (s, 2H, SCH₂), 2.40 (s, 3H, CH₃). |
| 44 | calculated for C₁₅H₁₄FN₅OS·H was 332.0976 found 332.0978 | 8.42-8.46 (m, 2H, Ph), 8.27 (d, 1H, pyridazine, J = 9.8 Hz), 7.83 (br s, 1H, NH₂), 7.42-7.48 (m, 2H, Ph), 7.35 (br s, 1H, NH₂), 7.33 (d, 1H, pyridazine, J = 9.7 Hz), 4.37 (d d, 1H, SCH, J = 6.3, 7.5 Hz), 1.92-2.07 (m, 2H, CH₂), 1.04 (t, 3H, CH₃, J = 7.5 Hz). |
| 45 | calculated for C₁₃H₁₃N₅OS₂·H was 320.0634 found 320.0637 | 8.26 (d, 1H, pyridazine, J = 9.8 Hz), 8.22 (d d, 1H, thienyl, J = 1.1, 3.9 Hz), 7.90 (br s, 1H, NH₂), 7.86 (d d, 1H, thienyl, J = 1.2, 5.1 Hz), 7.36 (br s, 1H, NH₂), 7.32 (d, 1H, pyridazine, J = 9.4 Hz), 7.32 (d, 1H, thienyl, J = 1.2 Hz), 4.52 (d d, 1H, SCH, J = 5.5, 7.9 Hz), 1.96-2.14 (m, 2H, CH₂), 1.04 (t, 3H, CH₃, J = 7.5 Hz). |
| 49 | calculated for C₁₂H₁₁N₅OS₂·H was 306.0478 found 306.0486 | 8.23 (d, 1H, pyridazine, J = 9.4 Hz), 8.02 (d, 1H, thienyl, J = 3.6 Hz), 7.78 (br s, 1H, NH₂), 7.35 (d, 1H, pyridazine, J = 9.7 Hz), 7.32 (br s, 1H, NH₂), 6.98 (d d, 1H, thienyl, J = 0.8, 3.5 Hz), 4.05 (s, 2H, SCH₂), 2.56 (s, 3H, CH₃). |

### REFERENCE EXAMPLE 11: 1-(Piperidin-1-yl)-2-((3-(thiophen-2-yl)-[1,2,4]triazolo[4,3-b]pyridazin-6-yl)thio)ethanone

To 2-((3-(thiophen-2-yl)-[1,2,4]triazolo[4,3-b]pyridazin-6-yl)thio)acetic acid (100 mg, 0.34 mmol) dissolved in DMF (3 mL) was added HATU (150 mg), and after 15 minutes stirring, piperidine (40 mg) was added followed by DIPEA (200 µL). The mixture was stirred for room temperature overnight and then evaporated to dryness. 1-(Piperidin-1-yl)-2-((3-(thiophen-2-yl)-[1,2,4]triazolo[4,3-b]pyridazin-6-yl)thio)ethanone was then isolated by preparative TLC (silica gel plates, 9:1 CHCl₃/MeOH).

The following compounds were prepared by an adaptation of this method (examples 34, 90 and 101 are compound according to the invention and the remaining compounds are reference examples):

| Example | MS | NMR |
|---|---|---|
| 7 | calculated for C₁₈H₁₅FN₆OS₂·H was 415.0806 found 415.0804 | 8.31-8.36 (m, 2H, Ph), 8.28 (d, 1H, pyridazine, J = 9.8 Hz), 7.54 (d, 1H, thiazole, J = 3.9 Hz), 7.40 (d, 1H, pyridazine, J = 9.8 Hz), 7.30-7.36 (m, 2H, Ph), 7.26 (d, 1H, thiazole, J = 9.3 Hz), 4.77 (app t, 1H, SCH), 2.05-2.17 (m, 2H, CH₂), 1.03 (t, 3H, CH₃, J = 7.2 Hz). |
| 8 | calculated for C₁₉H₂₀FN₅OS·H was 386.1445 found 386.1448 | 8.36-8.42 (m, 2H, Ph), 8.28 (d, 1H, pyridazine, J = 9.8 Hz), 7.46-7.52 (m, 2H, Ph), 7.33 (d, 1H, pyridazine, J = 9.3 Hz), 4.75 (d d, 1H, SCH, J = 5.5, 7.8 Hz), 3.50-3.57 and 3.27-3.38 (m, 4H, NCH₂), 1.92-2.11 (m, 2H, CH₂), 1.50-1.92 (m, 4H, pyrrolidinyl), 0.97 (t, 3H, CH₃, J = 7.2 Hz). |
| 9 | calculated for C₂₀H₂₂FN₅OS·H was 400.1602 found 400.1603 | 8.35-8.40 (m, 2H, Ph), 8.29 (d, 1H, pyridazine, J = 9.8 Hz), 7.44-7.51 (m, 2H, Ph), 7.35 (d, 1H, pyridazine, J = 9.7 Hz), 5.01 (d d, 1H, SCH, J = 5.4, 7.4 Hz), 3.49-3.58 and 3.33-3.45 (m, 4H, NCH₂), 1.93-2.13 (m, 2H, CH₂), 1.50-1.66 (m, 2H, piperidyl), 1.38-1.50 (m, 4H, piperidyl), 0.96 (t, 3H, CH₃, J = 7.2 Hz). |
| 11 | calculated for C₁₆H₁₇N₅OS₂·H was 360.0947 found 360.0943 | 8.26 (d, 1H, pyridazine, J = 9.8 Hz), 8.13 (d d, 1H, thienyl, J = 0.8, 3.5 Hz), 7.89 (d d, 1H, thienyl, J = 1.2, 5.1 Hz), 7.41 (d, 1H, pyridazine, J = 9.8 Hz), 7.36 (d d, 1H, thienyl, J = 3.5, 5.1 Hz), 4.5 (s, 2H, SCH), 3.60 (br s, 2H, piperidyl NCH₂), 3.43-3.49 (m, 2H, piperidyl NCH₂), 1.64 (br s, 2H, piperidyl CH₂), 1.40-1.50 m, 2H, piperidyl CH₂), 1.24 (br s, 2H, piperidyl CH₂). |
| 12 | calculated for C₂₄H₁₉FN₆OS·H was 459.1398 found 459.1402 | 10.88 (s, 1H, NH), 8.87 (d, 1H, quinolinyl NCH₂), 8.63 (app d, 1H, quinolinyl, J = 2.4 Hz), 8.34-8.40 (m, 2H, Ph), 8.32 (d, 1H, pyridazine, J = 9.8 Hz), 7.96 (br d, 1H, quinolinyl, J = 7.0 Hz), 7.88-7.92 (m, 1H, quinolinyl), 7.63-7.68 (m, 1H, quinolinyl), 7.55-7.61 (m, 1H, quinolinyl), 7.42 (d, 1H, pyridazine, J = 9.8 Hz), 7.28-7.38 (m, 2H, Ph), 5.64 (br s, 1H, NH), 4.69 (t, 1H, SCH, J = 6.9 Hz), 2.19 (app quint, 2H, J = 7.3 Hz), 1.12 (t, 3H, CH₃, J = 7.2 Hz). |
| 14 | calculated for C₂₂H₁₇F₄N₅OS·H was 476.1163 found 476.1166 | 10.03 (s, 1H, NH), 8.38-8.44 (m, 2H, FPh), 8.32 (d, 1H, pyridazine, J = 9.8 Hz), 7.72 (br d, 1H, NPh, J = 7.4 Hz), 7.55 (br t, 1H, NPh), 7.40-7.47 (m, 1H, NPh), 7.40 (d, 1H, pyridazine, J = 9.8 Hz), 7.29-7.35 (app t, 2H, FPh, J = 9.0 Hz), 7.16 (br d, 1H, NPh, J = 7.8 Hz), 4.76 (t, 1H, SCH, J = 6.7 Hz), 2.13 (app quint, 2H, CH₂, J = 7.3 Hz), 1.11 (t, 3H, CH₃, J = 7.3 Hz). |
| 21 | calculated for C₁₅H₁₅N₅OS₂·H was 346.0791 found 346.0793 | 8.26 (d, 1H, pyridazine, J = 9.8 Hz), 8.13 (app d d, 1H, thienyl, J = 1.2, 3.9 Hz), 7.88 (d d, 1H, thienyl, J = 1.0, 4.7 Hz), 7.41 (d, 1H, pyridazine, J = 9.4 Hz), 7.32 (d d, 1H, thienyl, J = 3.5, 4.7 Hz), 4.37 (s, 2H, SCH₂), 3.64 (t, 2H, pyrrolidinyl NCH₂, J = 6.8 Hz) 3.28 (app t, 2H, pyrrolidinyl NCH₂, J = 7.1 Hz), 1.99 (quint, 2H, pyrrolidinyl CH₂, J = 6.8 Hz), 1.83 (quint, 2H, pyrrolidinyl CH₂, J = 6.8 Hz). |
| 22 | calculated for C₁₇H₁₂FN₅O S₂·H was 386.0540 found 386.0540 | 10.55 (s, 1H, NH), 8.28 (d, 1H, pyridazine, J = 9.7 Hz), 8.13 (app d d, 1H, thienyl, J = 1.2, 3.6 Hz), 7.72 (d d, 1H, thienyl, J = 1.0, 4.7 Hz), 7.60-7.65 (m, 2H, Ph), 7.43 (d, 1H, pyridazine, J = 9.8 Hz), 7.12-7.18 (app t, 2H, Ph), 7.00 (d d, 1H, thienyl, J = 3.5, 5.1 Hz), 4.33 (s, 2H, SCH). |
| 23 | calculated for C₁₄H₁₀N₆OS₃·H was 374.997 found 375.0150 | 12.69 (s, 1H, NH), 8.29 (d, 1H, pyridazine, J = 9.7 Hz), 8.03 (d d, 1H, thienyl, J = 1.2, 3.5 Hz), 7.67 (d d, 1H, thienyl, J = 1.1, 5.0 Hz), 7.53 (d, 1H, thiazolyl, J = 3.6 Hz), 7.45 (1H, pyridazine, J = 9.8 Hz), 7.24 (d, 1H, thiazolyl, J = 3.5 Hz), 6.96 (d d, 1H, thienyl, J = 4.6, 4.7 Hz), 4.44 (s, 2H, SCH). |
| 24 | calculated for C₁₅H₁₅N₅O₂S₂·H was 362.0740 found 362.0739 | 8.27 (d, 1H, pyridazine, J = 9.4 Hz), 8.14 (app d d, 1H, thienyl, J = 1.2, 3.9 Hz), 7.88-7.91 (m, 1H, thienyl), 7.42 (d, 1H, pyridazine, J = 9.8 Hz), 7.33 (d d, 1H, thienyl, J = 3.5, 4.7 Hz), 4.48 (s, 2H, SCH), 3.62-3.67 and 3.68-3.73 (m, 4H, morpholino), 3.45-3.49 and 3.56-3.60 (m, 4H, morpholino). |
| 25 | calculated for C₁₈H₁₉N₅OS·H was 354.1383 found 354.1288 | 8.25 (d, 1H, pyridazine, J = 9.8 Hz), 8.23 (d, 2H, Ph, J = 8.3 Hz), 7.38 (d, 1H, pyridazine, J = 9.4 Hz), 7.37 (d, 2H, Ph, J = 8.2 Hz), 4.32 (s, 2H, SCH₂), 3.58 (t, 2H, pyrrolidine NCH₂, J = 6.8 Hz), 3.29 (t, 2H, NCH₂, J = |
| | | 7.8 Hz), 2.41 (s, 3H, CH3), 1.96 (app quint, 2H, pyrrolidine CH₂, J = 6.8 Hz), 1.79 (app quint, 2H, pyrrolidine CH₂, J = 6.8 Hz). |
| 34 | calculated for C₁₈H₂₁N₅OS₂·H was 388.1260 found 388.1268 | 8.28 (d, 1H, pyridazine, J = 9.8 Hz), 8.13 (d d, 1H, thienyl, J = 1.2, 4.0 Hz), 7.92 (d d, 1H, thienyl, J = 1.2, 5.1 Hz), 7.36 (app t, 1H, thienyl, J = 3.9 Hz), 7.34 (d, 1H, pyridazine, J = 9.8 Hz), 5.15-5.20 (m, 1H, SCH), 3.36-3.66 (m, 4H, piperidyl NCH₂), 1.96-2.17 (m, 2H, CH₂), 1.40-1.68 (m, 6H, piperidyl CH₂CH₂), 1.01 (t, 3H, CH₃, J = 7.2 Hz). |
| 43 | calculated for C₁₆H₁₁FN₆OS₂·H was 387.0493 found 387.0490 | 8.29 (d, 1H, pyridazine, J = 9.8 Hz), 8.22-8.29 (m, 2H, Ph), 7.50 (d, 1H, thienyl, J = 3.5 Hz), 7.44 (1H, pyridazine, J = 9.8 Hz), 7.12-7.20 (m, 3H), 4.33 (s, 2H, SCH₂). |
| 46 | calculated for C₁₇H₁₉N₅OS₂·H was 374.1104 found 374.1107 | 8.28 (d, 1H, pyridazine, J = 9.8 Hz), 8.14 (d d, 1H, thienyl, J = 1.2, 3.5 Hz), 7.34-7.37 (m, 1H, thienyl), 7.34 (d, 1H, pyridazine, J = 9.8 Hz), 4.92 (d d, 1H, SCH, J = 5.9, 7.9 Hz), 3.54-3.66 (m, 4H, morpholine NCH₂), 3.32-3.41 (m, 4H, morpholine NCH₂), 1.98-2.18 (m, 2H, CH₂), 1.75-1.95 (m, 4H, morpholine CH₂), 1.03 (t, 3H, CH₃, J = 7.4 Hz). |
| 47 | calculated for C₁₇H₁₉N₅O₂S₂·H was 390.1053 found 390.1052 | 8.28 (d, 1H, pyridazine, J = 9.6 Hz), 8.13 (d d, 1H, thienyl, J = 1.1, 3.6 Hz), 7.91 (d d, 1H, thienyl, J = 1.2, 5.0 Hz), 7.35 (d d, 1H, thienyl, J = 3.7, 5.0 Hz), 7.35 (d, 1H, pyridazine, J = 9.6 Hz), 5.11 (d d, 1H, SCH, J = 6.0, 7.1 Hz), 3.46-3.66 (m, 8H, morpholine CH₂), 1.98-2.18 (m, 2H, CH₂), 1.02 (t, 3H, CH₃, J = 7.4 Hz). |
| 48 | calculated for C₁₈H₁₇N₅OS₃·H was 416.0668 found 416.0669 | 9.11 (t, 1H, NH, J = 5.9 Hz), 8.26 (d, 1H, pyridazine, J = 9.7 Hz), 8.19 (d d, 1H, thienyl, J = 1.1, 3.5 Hz), 7.86 (d d, 1H, thienyl, J = 1.1, 5.0 Hz), 7.35 (d d, 1H, thienyl, J = 1.1, 5.1 Hz), 7.35 (d, 1H, pyridazine, J = 9.8 Hz), 7.30-7.34 (m, 1H, thienyl), 6.96 (d d, 1H, thienyl, J = 1.1, 3.5 Hz), 6.92 (d d, 1H, thienyl, J = 3.5, 5.1 Hz), 4.58 (d d, 1H, SCH, J = 5.4, 8.2 Hz), 4.53 (d, CH₂, J = 5.5 Hz), 4.43 (d d, 1H, CH₂, J = 5.1, 15.6 Hz), 1.99-2.50 (m, 2H, CH₂), 1.02 (t, 3H, CH₃, J = 7.4 Hz). |
| 50 | calculated for C₁₉H₂₃N₅OS₂·H was 402.1417 found 402.1422 | 8.28 (d, 1H, pyridazine, J = 9.8 Hz), 8.13 (d d, 1H, thienyl, J = 0.8, 3.9 Hz), 7.94 (d, 1H, thienyl, J = 4.7 Hz), 7.34-7.38 (m, 1H, thienyl), 7.33 (d, 1H, pyridazine, J = 9.8 Hz), 5.09 (d d, 1H, SCH), 3.42-3.62 (m, 4H, azepanyl NCH₂), 1.97-2.20 (m, 2H, CH₂), 1.59-1.70 and 1.43-1.56 (m, 8H, azepanyl CH₂CH₂), 1.02 (t, 3H, CH₃, J = 7.5 Hz). |
| 51 | calculated for C₁₉H₂₃N₅OS₂·H was 402.1417 found 402.1410 | 8.28 (d, 1H, pyridazine, J = 9.8 Hz), 8.11-8.16 (m, 1H, thienyl), 7.90-7.96 (m, 1H, thienyl), 7.32-7.38 (m, 1H, thienyl), 7.34 (d, 1H, pyridazine, J = 9.4 Hz), 5.06-5.30 (m, 1H, SCH), 4.68-4.82 (m, 1H, piperidyl NCH), 4.26-4.45 (m, 1H, piperidyl NCH), 3.78-3.90 (m, 1H, piperidyl NCH - isomer), 3.16-3.30 (m, 1H, piperidyl NCH - isomer), 2.66-2.80 (m, 1H, piperidyl NCH-isomer), 1.93-2.20 (m, 2H, CH₂), 1.34-1.73 (m, 6H, piperidyl CH₂), 1.10-1.32 (m, 3H, piperidyl CH₃), 0.94-1.6 (m, 3H, CH₃). |
| 57 | calculated for C₁₇H₁₉N₅OS₂·H was 374.1104 found 374.1113 | 8.24 (d, 1H, pyridazine, J = 9.7 Hz), 7.92 (d, 1H, thienyl, J = 3.5 Hz), 7.38 (d, 1H, pyridazine, J = 9.4 Hz), 7.01-7.04 (m, 1H, thienyl), 4.47 (s, 2H, SCH), 3.60 (br s, 2H, piperidinyl), 3.44-3.49 (m, 2H, piperidyl NCH₂), 2.57 (s, 3H, CH₃), 1.64 (br s, 4H piperidyl CH₂), 1.47 (piperidyl CH₂). |
| 58 | calculated for C₁₈H₂₂N₆OS₂·H was 403.1369 found 403.1373 | 8.28 (d, 1H, pyridazine, J = 9.8 Hz), 8.13 (d d, 1H, thiazole, J = 1.2, 3.5 Hz), 7.92 (d d, 1H, thienyl, J = 1.2, 5.1 Hz), 7.53 (app t, 1H, thienyl, J = 3.9 Hz), 7.35 (d, 1H, pyridazine, J = 9.4 Hz), 5.14 (app t, 1H, SCH, J = 6.5 Hz), 3.36-3.67 (m, 4H, piperazine NCH₂), 2.21-2.39 (m, 4H, piperazine NCH₂), 2.17 (s, 3H, CH₃), 1.96-2.17 (m, 2H, CH₂), 1.01 (t, 3H, CH₃, J = 7.4 Hz). |
| 59 | calculated for C₁₉H₂₃N₅OS₂·H was 402.1417 found 402.1423 | 8.25 (d, 1H, pyridazine, J = 9.4 Hz), 7.93 (d, 1H, thienyl, J = 3.5 Hz), 7.32 (d, 1H, pyridazine, J = 9.8 Hz), 7.14-7.17 (m, 1H, thienyl), 5.19 (t, 1H, SCH, J = 6.4 Hz), 3.40-3.64 (m, 4H, piperidyl NCH₂), 2.51 (s, CH₃), 1.92-2.18 (m, 2H, CH₂), 1.38-1.68 (m, 6H, piperidyl CH₂CH₂), 1.00 (t, 3H, CH₃, J = 7.5 Hz). |
| 60 | calculated for C₁₇H₂₀N₆OS₂·H was 389.1213 found 388.1216 | 8.37 (d, 1H, pyridazine, J = 9.7 Hz), 8.20 (d, 1H, thiazole, J = 3.1 Hz), 8.16 (d, 1H, thiazole, J = 3.1 Hz), 7.44 (d, 1H, pyridazine, J = 9.3 Hz), 5.28-5.33 (m, 1H, SCH), 3.36-3.68 (m, 4H, piperidyl NCH₂), 1.94-2.16 (m, 2H, CH₂), 1.42-1.66 (m, 6H, piperidyl CH₂CH₂), 0.99 (t, 3H, CH₃, J = 7.3 Hz). |
| 62 | calculated for C₁₈H₂₀ClN₅OS₂·H was 422.0871 found 422.0874 | 8.29 (d, 1H, pyridazine, J = 9.4 Hz), 8.13 (d, 1H, thienyl, J = 3.9 Hz), 7.28-7.44 (m, 1H, thienyl), 7.36 (d, 1H, pyridazine, J = 9.7 Hz), 5.17 (t, 1H, SCH, J = 6.2 Hz), 3.62-3.73 (m, 2H, piperidyl NCH₂), 3.30-3.56 (m, 2H, piperidyl NCH₂), 1.96-2.17 (m, 2H, CH₂), 1.37-1.70 (m, 6H, piperidyl CH₂CH₂), 1.01 (t, 3H, CH₃, J = 7.2 Hz). |
| 63 | calculated for C₁₇H₁₈ClN₅OS₂·H was 408.0714 found 408.0717 | 8.29 (d, 1H, pyridazine, J = 9.7 Hz), 7.97 (d, 1H, thienyl, J = 3.9 Hz), 7.40 (d, 1H, thienyl, J = 3.9 Hz), 7.36 (d, 1H, pyridazine, J = 9.4 Hz), 4.89 (d d, 1H, SCH, J = 5.9, 7.9 Hz), 3.57-3.64 (m, 2H, pyrrolidinyl NCH₂), 2.01-2.18 (m, 2H, CH₂), 1.76-1.97 (m, 4H, pyrrolidinyl CH₂CH₂), 1.03 (t, 3H, CH₃, J = 7.5 Hz). |
| 64 | calculated for C₁₈H₂₁N₅OS₂·H was 388.1260 found 388.1260 | 8.53 (d d, 1H, thienyl, J = 1.2, 2.7 Hz), 8.27 (d, 1H, pyridazine, J = 9.8 Hz), 7.22 (d d, 1H, thienyl, J = 1.2, 5.1 Hz), 7.85 (d d, 1H, thienyl, J = 3.1, 5.1 Hz), 7.33 (d, 1H, pyridazine, J = 9.8 Hz), 5.07 (t, 1H, SCH, J = 6.7 Hz), 3.54-3.65 and 3.30-3.50 (m, 4H, piperidyl NCH₂), 1.93-2.14 (m, 2H, CH₂), 1.36-1.67 (m, 6H, piperidyl CH₂CH₂), 0.99 (t, 3H, CH₃, J = 7.3 Hz). |
| 86 | calculated for C₁₉H₁₇N₅OS₂·H was 396.0947 found 396.0945 | 8.29 (d, 1H, pyridazine, J = 9.8 Hz), 8.19 (d d, 1H, thienyl, J = 1.2, 3.5 Hz), 7.92 (d d, 1H, thienyl, J = 1.3, 5.0 Hz), 7.58-7.63 (app d, 2H, phenyl, J = 7.5 Hz), 7.38 (d, 7.38 (d, 1H, pyridazine, J = 9.7 Hz), 7.28-7.38 (m, 2H, phenyl), 7.21 (d d, 1H, thienyl, J = 3.5, 5.0 Hz), 7.06-7.11 (app t, 1H, phenyl, J = 7.5 Hz), 4.73 (d d, 1H, SCH, J = 5.9, 7.4 Hz), 2.08-2.21 (m, 2H, CH₂), 1.10 (t, 3H, CH₃, J = 7.1 Hz). |
| 87 | calculated for C₂₀H₂₅N₅OS₂·H was 416.1573 found 416.1582 | 8.26 (d, 1H, pyridazine, J = 9.4 Hz), 7.94 (d, 1H, thienyl, J = 3.5 Hz), 7.31 (d, 1H, pyridazine, J = 9.4 Hz), 7.09 (app d, 1H, thienyl, J = 3.9 Hz), 5.20 (d d, 1H, SCH, J = 5.5, 7.0 Hz), 3.32-3.70 (m, 4H, piperidyl NCH₂), 2.94 (app q, 2H, CH₂), 1.98-2.18 (m, 2H, Et CH₂), 1.36-1.68 (m, 6H, piperidyl CH₂CH₂), 1.32 (t, 3H, CH₃, J = 7.4 Hz), 1.01 (t, 3H, CH₃, J = 7.4 Hz). |
| 90 | calculated for C₁₇H₁₉N₅OS₂·H was 374.1103 found 374.1113 | 8.28 (d, 1H, pyridazine, J = 9.8 Hz), 8.13 (d d, 1H, thienyl, J = 1.1, 3.5 Hz), 7.90 (d d, 1H, thienyl, J = 1.2, 5.1 Hz), 7.34-7.37 (m, 1H, thienyl), 7.34 (d, 1H, pyridazine, J = 9.4 Hz), 5.20 (d d, 1H, SCH, J = 6.6, 7.1 Hz), 3.40-3.63 (m, 4H, piperidyl NCH₂), 1.67 (d, 3H, CH₃, J = 6.6 Hz), 1.51-1.64 (m, 4H, piperidyl), 1.43-1.51 (m, 2H, piperidyl). |
| 93 | calculated for C₁₉H₂₃N₅OS₂·H was 402.1417 found 402.1417 | 8.28 (d, 1H, pyridazine, J = 9.8 Hz), 8.13 (d d, 1H, thienyl, J = 1.2, 3.9 Hz), 7.92 (d d, 1H, thienyl, J = 1.2, 5.1 Hz), 7.34-7.36 (m, 1H, thienyl), 7.33 (d, 1H, pyridazine, J = 9.8 Hz), 5.19 (d d, 1H, SCH, J = 6.0, 7.1 Hz), 3.35-3.66 (m, 4H, piperidyl NCH₂), 1.89-2.11 (m, 2H, Pr CH₂), 1.37-1.66 (m, 6H, piperidyl CH₂CH₂), 0.89 (t, 3H, CH₃, J = 7.2 Hz). |
| 105 | calculated for C₁₆H₁₇N₅OS₂·H was 360.0947 found 360.0939 | 8.28 (d, 1H, pyridazine, J = 9.8 Hz), 8.14 (d d, 1H, thienyl, J = 8.1, 3.5 Hz), 7.89 (d d, 1H, thienyl, J = 1.1, 4.7 Hz), 7.34 (d, 1H, pyridazine, J = 9.4 Hz), 7.32-7.36 (m, 1H, thienyl), 4.99 (q, 1H, SCH, J = 6.7 Hz), 3.62 (t, 2H, pyrrolidinyl NCH₂, J = 6.8 Hz), 3.35 (t, 2H, pyrrolidinyl NCH₂, J = 6.8 Hz), 1.76-1.97 (m, 4H, pyrrolidinyl CH₂), 1.68 (d, 3H, CH₃, J = 7.0 Hz). |
| 101 | calculated for C₁₉H₂₃N₅OS₂·H was 246.0791 found 246.0789 | 8.28 (d, 1H, pyridazine, J = 9.7 Hz), 8.16 (d d, 1H, thienyl, J = 1.1, 3.5 Hz), 7.90 (d d, 1H, thienyl, J = 1.2, 5.1 Hz), 7.34-7.38 (d d, 1H, thienyl, J = 3.9, 5.1 Hz), 7.34 (d, 1H, pyridazine, J = 9.8 Hz), 4.75 (q, 1H, SCH, J = 6.9 Hz), 4.34 (app q, 1H, azetidinyl NCH₂, J = 2.6 Hz), 4.27 (app q, 1H, azetidinyl NCH₂, J = 7.9 Hz), 3.86-3.96 (m, 2H, azetidinyl NCH₂), 2.17-2.29 (m, 2H, azetidinyl CH₂), 1.62 (d, 3H, NCH₃, J = 7.0 Hz). |
| 102 | calculated for C₁₈H₁₉F₂N₅OS₂·H was 424.1072 found 424.1080 | 8.29 (d, 1H, pyridazine, J = 9.8 Hz), 8.14 (d d, 1H, thienyl, J = 1.2, 3.6 Hz), 7.92 (d d, 1H, thienyl, J = 1.2, 5.1 Hz), 7.36 (d, 1H, pyridazine, J = 9.8 Hz), 7.35 (d d, 1H, thienyl, J = 4.0, 5.1 Hz), 5.19 (t, 1H, SCH, J = 6.5 Hz), 3.44-3.77 and 1.85-2.19 (m, 10H, piperidyl CH₂s, CH₂), 1.02 (t, 3H, CH₃, J = 7.2 Hz). |
| 103 | calculated for C₂₁H₂₅N₅O₃S₂·H was 460.1472 found 460.1462 | Complex spectrum due to the partial double bond character of the amide bond. Also, a mixture of diasteromers. 8.26-8.34 (m, 1H, pyridazine), 8.11-8.20 (m, 1H, thienyl), 7.86-7.99 (m, 1H, thienyl), 7.32-7.39 (m, 2H, pyridazine and thienyl), 5.13-5.38 (m, 1H, SCH), 3.60-4.16 (m, 5H, piperidyl NCH₂ and piperidyl CH), 8.16 (d, 1H, thiazole, J = 3.1 Hz), 7.44 (d, 1H, pyridazine, J = 9.3 Hz), 5.28-5.33 (m, 1H, SCH), 4.00-4.30 (m, 2H, CH₂), 3.00-4.00 (m, 4H, piperidyl NCH₂), 1.30-2.40 (m, 4 H, piperidyl CH₂), 1.13-1.22 (m, 3H, CH₃), 0.85-1.04 (m, 3H, CH₃). |
| 104 | calculated for C₁₉H₂₃N₅OS₂·H was 402.1417 found 402.1410 | 8.28 (d, 1H, pyridazine, J = 9.8 Hz), 8.11-8.16 (m, 1H, thienyl), 7.90-7.97 (m, 1H, thienyl), 7.33-7.38 (m, 1H, thienyl), 7.34 (d, 1H, pyridazine, J = 9.4 Hz), 5.06-5.30 (m, 1H, SCH), 4.68-4.82 (br s, 1H, piperidyl NCH), 4.26-4.44 (m, 3H, piperidyl NCH₂), 3.78-3.90 (m, 3H, piperidyl NCH₂), 1.92-2.19 (m, 2H, CH₂), 1.10-1.73 (m, 6H, piperidyl CH₂), 1.12 (app d, 3H, piperidyl CH₃, J = 7.1H), 0.94-1.06 (m, 3H, Et CH₃). |
| 108 | calculated for C₁₇H₁₉N₇OS·H was 370.1445 found 370.1449 | 9.51 (d, 1H, piperazine, J = 1.6 Hz), 8.91 (app d, 1H, piperazine, J = 0.7 Hz), 8.85 (d, 1H, piperazine, J = 0.9 Hz), 8.36 (d, 1H, pyridazine, J = 9.7 Hz), 7.43 (d, 1H, pyridazine, J = 9.8 Hz), 5.15 (q, 1H, SCH, J = 6.8 Hz), 3.30-3.60 (m, 4H, piperidyl NCH₂), 1.58 (d, 3H, CH₃, J = 7.0 Hz), 1.40-1.64 (m, 6H, piperidinyl CH₂). |
| 109 | calculated for C₁₇H₁₉N₇OS·H was 370.1445 found 370.1446 | 9.08 (d, 1H, pyrimidine, J = 5.1 Hz), 8.34 (d, 1H, pyrimidine, J = 9.4 Hz), 7.71 (t, 1H, pyrimidine, J = 4.9 Hz), 7.40 (d, 1H, pyridazine, J = 9.8 Hz), 5.15 (q, 1H, SCH, J = 6.0 Hz), 3.12-3.60 (m, 4H, piperidyl NCH₂), 1.57 (d, 3H, CH₃, J = 7.1 Hz), 1.49-1.58 (m, 2H, piperidinyl CH₂), 1.38-1.46 (m, 4H, piperidyl CH₂). |
| 110 | calculated for C₁₆H₁₈N₆OS₂·H was 375.1056 found 375.1066 | 9.37 (d, 1H, thiazole, J = 1.9 Hz), 8.68 (d, 1H, thiazole, J = 1.6 Hz), 7.36 (d, 1H, pyridazine, J = 9.3 Hz), 5.17 (q, 1H, SCH, J = 6.8 Hz), 3.25-3.60 (m, 4H, piperidyl NCH₂), 1.61 (d, 3H, CH₃, J = 7.0 Hz), 1.39-1.64 (m, 6H, piperidinyl CH₂). |
| 111 | calculated for C₁₅H₁₆N₆OS₂·H was 361.0900 found 361.0909 | 9.37 (d, 1H, thiazole, J = 2.0 Hz), 8.67 (d, 1H, thiazole, J = 2.0 Hz), 8.37 (d, 1H, pyridazine, J = 9.8 Hz), 7.36 (d, 1H, pyridazine, J = 9.4 Hz), 4.93 (q, 1H, SCH, J = 6.8 Hz), 3.52-3.63 (m, 2H, pyrrolidine NCH₂), 3.26-3.37 (m, 2H, pyrrolidine NCH₂), 1.69-1.95 (m, 4H, pyrrolidine NCH₂), 1.61 (d, 3H, CH₃, J = 6.7 Hz). |
| 112 | calculated for C₁₇H₂₁N₇OS·H was 372.1601 found 375.1591 | 8.32 (d, 1H, pyridazine, J = 9.8 Hz), 7.73 (d, 1H, pyrazole, J = 2.0 Hz), 7.39 (d, 1H, pyridazine, J = 9.3 Hz), 7.18 (d, 1H, pyrazole, J = 2.0 Hz), 5.17 (q, 1H, SCH, J = 7.0 Hz), 4.25 (s, 3H, NCH₃), 3.51-3.61 (m, 2H, piperidyl NCH₂), 3.28-3.42 (m, 2H, piperidyl NCH₂), 1.59 (d, 3H, CH₃, J = 7.0 Hz), 1.40-1.64 (m, 6H, piperidinyl CH₂). |
| 113 | calculated for C₁₇H₂₁N₇OS·H was 372.1601 found 375.1593 | 8.24 (d, 1H, pyridazine, J = 9.8 Hz), 7.98 (d, 1H, pyrazole, J = 1.9 Hz), 7.29 (d, 1H, pyridazine, J = 9.4 Hz), 7.03 (d, 1H, pyrazole, J = 1.9 Hz), 5.17 (q, 1H, SCH, J = 6.9 Hz), 4.00 (s, 3H, NCH₃), 3.24-3.62 (m, 4H, piperidyl NCH₂), 1.62 (d, 3H, CH₃, J = 7.1 Hz),1.40-1.64 (m, 6H, piperidinyl CH₂). |
| 114 | calculated for C₁₆H₁₇N₅OS₂·H was 360.0947 found 340.0943 | 8.28 (d, 1H, pyridazine, J = 9.7 Hz), 8.15 (d d, 1H, thienyl, J = 1.2, 3.5 Hz), 7.92 (d d, 1H, thienyl, J = 1.2, 5.1 Hz), 7.36 (d d, 1H, thienyl, J = 3.6, 5.1 Hz), 7.35 (d, 1H, pyridazine, J = 9.4 Hz), 4.67 (d d, 1H, SCH, J = 6.3, 7.9 Hz), 4.20-4.36 (m, 2H, azetidinyl NCH₂), 3.88-3.96 (m, 2H, azetidinyl NCH₂), 2.13-2.29 (m, 2H, azetidinyl CH₂), 1.72-2.10 (m, 3H, Pr CH₂), 1.02 (t, 3H, CH₃, J = 5.5 Hz). |
| 115 | calculated for C16H19N7OS.H was 358.1445 found 358.1438 | 8.22 (d, 1H, pyridazine, J = 9.8 Hz), 8.01 (br s, 1H, pyrazole CH), 7.30 (d, 1H, pyridazine, J = 9.7 Hz), 7.08 (br s, 1H, pyrazole CH), 5.16 (q, 1H, SCH, J = 6.8 Hz), 3.30-3.62 (m, 4H, piperidyl NCH₂), 1.60 (d, 3H, CH₃, J = 7.0 Hz), 1.41-1.68 (m, 6H, piperidinyl CH₂). |
| 116 | calculated for C₁₇H₁₉N₇OS·H was 370.1445 found 370.1438 | 10.09 (d d, 1H, pyridazine, J = 1.1, 1.9 Hz), 9.51 (d d, 1H, pyridazine, J = 1.2, 5.5 Hz), 8.53 (d d, 1H, pyridazine, J = 2.3, 5.5 Hz), 8.39 (d, 1H, pyridazine, J = 9.8 Hz), 7.47 (d, 1H, pyridazine, J = 9.8 Hz), 5.16 (q, 1H, SCH, J = 6.9 Hz), 3.15-3.66 (m, 4H, piperidyl NCH₂), 1.65 (d, 3H, CH₃, J = 7.0 Hz), 1.41-1.65 (m, 6H, piperidinyl CH₂). |
| 117 | calculated for C₁₆H₁₉N₅OS₂·H was 362.1104 found 362.1101 | 8.28 (d, 1H, pyridazine, J = 9.7 Hz), 8.13 (d d, 1H, thienyl, J = 1.1, 3.5 Hz), 7.91 (d d, 1H, thienyl, J = 1.2, 4.7 Hz), 7.35 (d d, 1H, thienyl, J = 3.9, 5.0 Hz), 7.34 (d, 1H, pyridazine, J = 9.8 Hz), 5.17 (q, 1H, SCH, J = 6.7 Hz), 3.40-3.51 and 3.30-3.38 (2m, 4H, NCH₂), 1.70 (d, 3H, CH₃, J = 6.7 Hz), 1.18 (t, 3H, CH₃, J = 7.1 Hz), 1.04 (t, 3H, CH₃, J = 7.1 Hz). |
| 118 | calculated for C₁₄H₁₅N₅OS₂·H was 334.0791 found 334.0785 | 8.27 (d, 1H, pyridazine, J = 9.7 Hz), 8.12 (d d, 1H, thienyl, J = 1.1, 3.9 Hz), 7.89 (d d, 1H, thienyl, J = 1.2, 5.1 Hz), 7.35 (d d, 1H, thienyl, J = 3.5, 4.6 Hz), 7.34 (d, 1H, pyridazine, J = 9.7 Hz), 5.17 (q, 1H, SCH, J = 6.9 Hz), 3.17 (s, 3H, NCH₃), 2.90 (s, 3H, NCH₃), 1.66 (d, 3H, CH₃, J = 7.1 Hz). |
| 119 | calculated for C₁₃H₁₃N₅OS₂·H was 320.0634 found 340.0630 | 8.34-8.40 (m, 1H, C(=O)NH), 8.27 (d, 1H, pyridazine, J = 9.8 Hz), 8.18 (d d, 1H, thienyl, J = 1.1, 5.0 Hz), 7.86 (d d, 1H, thienyl, J = 1.1, 5.0 Hz), 7.33 (d d, 1H, thienyl, J = 3.5, 5.0 Hz), 7.33 (d, 1H, pyridazine, J = 9.4 Hz), 4.63 (q, 1H, SCH, J = 7.0 Hz), 2.62 (d, 3H, NCH₃, J = 4.3 Hz), 1.65 (d, 3H, CH₃, J = 7.1 Hz). |
| 120 | calculated for C₁₇H₂₁N₇OS·H was 372.1601 found 372.1603 | 8.30 (d, 1H, pyridazine, J = 9.4 Hz), 7.51 (br s, 1H, imidazole), 7.35 (d, 1H, pyridazine, J = 9.8 Hz), 7.21 (s, 1H, imidazole), 5.18 (q, 1H, SCH, J = 7.1 Hz), 3.91 (s, 3H, imidazole CH₃), 3.20-3.65 (m, 6H, piperidyl), 1.52 (d, 3H, CH₃, J = 6.6 Hz). |
| 121 | calculated for C₁₅H₁₇N₅OS₂·H was 348.0947 found 348.0949 | 8.28 (d, 1H, pyridazine, J = 9.8 Hz), 8.12 (d d, 1H, thienyl, J = 1.2, 3.5 Hz), 7.91 (d d, 1H, thienyl, J = 1.2, 5.1 Hz), 7.35 (d d, 1H, thienyl, J = 3.5, 5.1 Hz), 7.43 (d, 1H, pyridazine, J = 9.4 Hz), 5.13 (d d, 1H, SCH, J = 5.8, 7.0 Hz), 3.15 (s, 3H, CH₃), 2.91 (s, 3H, CH₃), 1.97-2.16 (m, 3H, CH₂), 1.01 (t, 3H, CH₃, J = 7.2 Hz). |
| 127 | calculated for C₁₉H₂₃N₅OS₂.H was 402.1416 found 402.1420 | 8.26-8.3 (m, 1H, pyridazine), 8.11-8.16 (m, 1H, thienyl), 7.91-7.92 (m, 1H, thienyl), 7.33-7.36 (m, 2H, thienyl and pyridazine), 5.14-5.26 (m, 1H, SCH), 3.85-4.3 (2m, 2H, NCH₂), 1.97-2.15 and 2.32-3.26 (3m, 4H, NCH₂ and CH₂), 1.09-1.79 (m, 5H, 2CH₂ and CH), 0.97-1.02 (m, 3H, CH₃), 0.65-0.87 (m, 3H, CH₃). |
| 122 | calculated for C₁₄H₁₅N₅OS₂ was 350.0947 found 350.0949 | 8.48 (m, 1H, NH), 8.26 (d, 1H, pyridazine, J = 9.8 Hz), 8.20 (d d, 1H, thienyl, J = 0.8, 3.5 Hz), 7.86 (d d, 1H, thienyl, J = 1.1, 5.0 Hz), 7.32-7.36 (m, 1H, thienyl), 7.32 (d, 1H, pyridazine, J = 9.4 Hz), 4.66-4.73 (m, 2H, SCH and OH), 3.39 (q, 2H, CH₂, J = 5.9 Hz), 3.09-3.22 (app heptet, 2H, CH₂), 1.66 (d, 3H, CH₃, J = 6.6 Hz). |
| 123 | calculated for C₁₈H₂₃N₅OS₂ was 390.1417 found 390.1408 | 8.28 (d, 1H, pyridazine, J = 9.8 Hz), 8.12 (d d, 1H, thienyl, J = 1.2, 3.5 Hz), 7.90 (d d, 1H, thienyl, J = 1.2, 5.1 Hz), 7.34-7.37 (m, 1H, thienyl), 7.34 (d, 1H, pyridazine, J = 9.4 Hz), 5.14 (q, H, SCH, J = 6.7 Hz), 3.20-3.37 (m, 4H, NCH₂), 1.71 (d, 3H, CH₃, J = 6.6 Hz), 1.52-1.65 (m, 2H, CH₂), 1.50 (sextet, 2H, CH₂, J = 7.4 Hz), 0.83 (t, 3H, CH₃, J = 7.5 Hz), 0.75 (t, 3H, CH₃, J = 7.4 Hz). |
| 125 | calculated for C₁₆H₁₈N₆OS₂ was 375.1068 found 375.1060 | 8.37 (d, 1H, pyridazine, J = 9.7 Hz), 8.20 (d, 1H, thienyl, J = 3.1 Hz), 8.15 (d, 1H, thienyl, J = 3.1 Hz), 7.45 (d, 1H, pyridazine, J = 9.8 Hz) 5.30 (q, 1H, SCH, J = 6.8 Hz), 3.54-3.59 (m, 1H, NCH₂), 3.46-3.51 (m, 1H, NCH₂), 1.68 (d, 3H, CH₃, J = 6.6 Hz), 1.50-1.65 (m, 1H, CH₂), 1.41-1.52 (m, 1H, CH₂). |
| 126 | calculated for C₁₄H₁₄N₆OS₂ was 347.0743 found 347.0749 | 8.38 (d, 1H, pyridazine, J = 9.8 Hz), 8.21 (d, 1H, thienyl, J = 3.2 Hz), 8.14 (d, 1H, thienyl, J = 3.1 Hz), 7.44 (d, 1H, pyridazine, J = 9.3 Hz), 4.84 (q, 1H, SCH, J = 6.8 Hz), 4.24-4.37 (m, 2H, azetidinyl NCH₂), 3.88-3.95 (m, 1H, azetidinyl NCH₂), 2.15-2.28 (m, 2H, azetidinyl NCH₂), 1.63 (d, 3H, NCH₃, J = 6.6 Hz). |

### Preparation of 6-chloro-3-(2-fluorophenyl)-[1,2,4]triazolo[4,3-b]pyridazine

6-Chloro-3-hydrazinopyridazine (6.9 mmol) was suspended in dioxane (45 mL) with triethylamine (1.1 equiv), and the acyl chloride of 2-fluorobenzoic acid (1.1 equiv) in dioxane (10 ml) was added dropwise over 5-10 min at room temperature. The reaction mixture was stirred at the same temperature for 30-50 min (monitored by TLC), and then the dioxane was removed by rotary evaporation. The residue was refluxed in phosphorus oxychloride (40 ml) for 3-4 hours, and then the solvent was evaporated. 6-Chloro-3-(2-fluorophenyl)-[1,2,4]triazolo[4,3-b]pyridazine was obtained by washing the residue several times with ethyl acetate followed by filtration then adding ice-cold water (50 ml) to the residue. The aqueous layer was neutralized to pH 7 with saturated aqueous sodium hydrogen carbonate, and the resulting solid was collected by filtration, washed twice with water and then with hexane, and dried under vacuum at 50 °C.
¹H NMR (400 MHz, Me₂SO-d₆): 8.57 (d, 1H, pyridazine, J = 9.3 Hz), 7.85-7.89 (td, 1H, Ph, J = 1.6 Hz and 7.4 Hz), 7.68-7.72 (m, 1H, Ph), 7.58 (d, 1H, pyridazine, J = 9.4 Hz), 7.45-7.53 (m, 2H, Ph). FABMS (M+H) calculated for C₁₁H₆ClFN₄.H was 249.0337 found 249.0339. The following compounds were prepared by this method:
a) 6-Chloro-3-(3-fluorophenyl)-[1,2,4]triazolo[4,3-b]pyridazine:
   ¹H NMR (400 MHz, Me₂SO-d₆): 8.57 (d, 1H, pyridazine, J = 9.8 Hz), 8.17-8.2 (m, 1H, Ph), 8.08-8.12 (m, 1H, Ph), 7.68-7.73 (m, 1H, Ph), 7.6 (d, 1H, pyridazine, J = 9.4 Hz), 7.43-7.48 (m, 1H, Ph). FABMS (M+H) calculated for C₁₁H₆ClFN₄.H was 249.0337 found 249.0342.
b) 6-Chloro-3-(4-fluorophenyl)-[1,2,4]triazolo[4,3-b]pyridazine:
   ¹H NMR (400 MHz, Me₂SO-d₆): 8.6 (d, 1H, pyridazine, J = 9.4 Hz), 8.55 (d, 2H, Ph, J = 7.9 Hz), 8.03 (d, 2H, Ph, J = 8.3 Hz), 7.62 (d, 1H, pyridazine, J = 9.4 Hz). FABMS (M+H) calculated for C₁₂H₆ClF₃N₄.H was 299.0306 found 299.0310.
c) 6-Chloro-3-(3,4-diftuorophenyl)-[1,2,4]triazolo[4,3-b]pyridazine:
   ¹H NMR (400 MHz, Me₂SO-d₆): 8.57 (d, 1H, pyridazine, J = 9.4 Hz), 8.28-8.34 (m, 1H, Ph), 8.18-8.22 (m, 1H, Ph), 7.71-7.78 (m, 1H, Ph), 7.6 (d, 1H, pyridazine, J = 9.8 Hz). FABMS (M+H) calculated for C₁₁H₅ClF₂N₄.H was 267.0243 found 267.0249. FABMS (M+H) calculated for C₉H₅ClN₄S.H was 236.9996 found 236.9993.
d) 6-Chloro-3-(4-methoxyphenyl)-[1,2,4]triazolo[4,3-b]pyridazine FABMS (M+H) calculated for C₁₂H₉ClN₄O.H was 261.0538 found 261.0541.
e) 6-Chloro-3-(3-methoxyphenyl)-[1,2,4]triazolo[4,3-b]pyridazine
f) 6-Chloro-3-(2-methoxyphenyl)-[1,2,4]triazolo[4,3-b]pyridazine

### Preparation of 3-(2-fluorophenyl)-[1,2,4]triazolo[4,3-b]pyridazine-6-thiol

A solution of 6-chloro-3-(2-fluorophenyl)-[1,2,4]triazolo[4,3-b]pyridazine (1 mmol) and sodium hydrosulfide hydrate (1.5 equiv.) in ethanol was refluxed for 2-6 hours. The solvent was removed under reduced pressure, water (2 ml) was added, and the pH adjusted to 9 with sodium hydroxide solution. After removing the resulting precipitate, the filtrate was acidified with 6M HCl to a pH of 3, and the formed precipitate was collected, washed with cold water, and dried to give 3-(2-fluorophenyl)-[1,2,4]triazolo[4,3-b]pyridazine-6-thiol. This compound is taken on to the next step without purification

### REFERENCE EXAMPLE 74: Ethyl 2-((3-(2-fluorophenyl)-[1,2,4]triazolo[4,3-b]pyridazin-6-yl)thio)butanoate

To a solution of 3-(2-fluorophenyl)-[1,2,4]triazolo[4,3-b]pyridazine-6-thiol (1 mmol) and cesium carbonate (1.5 equiv.) in DMF (5 mL) was added ethyl 2-bromobutyrate (1.5 equiv.) and the mixture was stirred at 70 °C for 3 hours. The reaction was monitored by thin-layer chromatography and was shown to be complete at this time. The solvent was removed in vacuo and the residue was chromatographed on a silica gel column, to furnish ethyl 2-((3-(2-fluorophenyl)-[1,2,4]triazolo[4,3-b]pyridazin-6-yl)thio)butanoate.

The following compounds were prepared by adaptation of the method used above (the following compounds are reference examples):

| Example | MS | NMR |
|---|---|---|
| 74 | calculated for C₁₇H₁₇FN₄O₂S·H was 361.1129 found 361.1137 | 8.32 (d, 1H, pyridazine, J = 9.7 Hz), 7.86-7.95 (td, 1H, Ph, J = 2 Hz and 7.4 Hz), 7.67-7.72 (m, 1H, Ph), 7.48-7.51 (m, 1H, Ph), 7.42-7.46 (td, 1H, Ph, J = 1.2 Hz and 7.9 Hz), 7.4 (d, 1H, pyridazine, J = 9.8 Hz), 4.29 (t, 1H, SCH, J = 6.8 Hz), 4-4.08 (dq, 1H, OCH, J = 7 Hz and 10.6 Hz), 3.8-3.88 (dq, 1H, OCH, J = 7 Hz and 10.9 Hz), 1.9-1.96 (dq, 2H, CH₂, J = 7 Hz and 3.1 Hz), 1.02 (t, 3H, CH₃, J = 7.5 Hz), 0.94 (t, 3H, CH₃, J = 7.4 Hz). |
| 75 | calculated for C₁₇H₁₇FN₄O₂S·H was 361.1129 found 361.1141 | 8.33 (d, 1H, pyridazine, J = 9.8 Hz), 8.14-8.2 (m, 2H, Ph), 7.63-7.69 (td, 1H, Ph, J = 5.8 Hz and 2 Hz), 7.41-7.46 (m, 1H, Ph), 7.4 (d, 1H, pyridazine, J = 9.8 Hz), 4.55 (t, 1H, SCH, J = 6.8 Hz), 3.96-4.04 and 4.09-4.17 (2dq, 2H, OCH₂, J = 7.1 Hz and 11 Hz), 2.01-2.09 (m, 2H, CH₂), 1.07 (t, 3H, CH₃, J = 7 Hz), 1.05 (t, 3H, CH₃, J = 7.4 Hz). |
| 76 | calculated for C₁₇H₁₆F₂N₄O₂S·H was 379.1034 found 379.1042. | 8.35-8.5 (m, 1H, Ph), 8.32 (d, 1H, pyridazine, J = 9.8 Hz), 8.17-8.22 (m, 1H, Ph), 7.65-7.72 (m, 1H, Ph), 7.41 (d, 1H, pyridazine, J = 9.8 Hz), 4.54 (t, 1H, SCH, J = 7.5 Hz), 4.09-4.17 and 3.97-4.5 (2dq, 2H, OCH₂, J = 7 Hz and 10.7 Hz), 2-2.08 (m, 2H, CH₂), 1.06 (t, 3H, CH₃, J = 7.2 Hz), 1.04 (t, 3H, CH₃, J = 7.4 Hz). |
| 77 | calculated for C₁₈H₁₇F₃N₄O₂S·H was 411.1097 found 411.1103 | 8.58 (d, 2H, Ph, J = 8.2 Hz), 8.35 (d, 1H, pyridazine, J = 9.8 Hz), 7.96 (d, 2H, Ph, J = 8.2 Hz), 7.44 (d, 1H, pyridazine, J = 9.7 Hz), 4.57 (t, 1H, SCH, J = 6.8 Hz), 4.06-4.14 and 3.92-4 (2dq, 2H, OCH₂, J = 7.1 Hz and 10.6 Hz), 2-2.08 (m, 2H, CH₂), 1.05 (t, 3H, CH₃, J = 7.4 Hz), 1.03 (t, 3H, CH₃, J = 7.2 Hz). |
| 65 | calculated for C₁₈H₂₀N₄O₃S·H was 373.1329 found 373.1349 | 8.28-8.32 (d m, 2H, Ph), 8.28 (d, 2H, pyridazine, J = 11.0 Hz), 7.34 (d, 1H, pyridazine, J = 9.7 Hz), 7.15 (d m, 2H, Ph), 4.53 (t, 1H, SCH, J = 6.8 Hz), 4.10-4.18 and 3.97-4.06 (2dq, 2H, OCH₂, J = 7.0 Hz and 11.0 Hz), 3.87 (s, 3H, OCH₃), 1.99-2.09 (m, 2H, CH₂), 1.09 (t, 3H, CH₃, J = 7.0 Hz), 1.05 (t, 3H, CH₃, J = 7.4 Hz). |
| 68 | calculated for C₁₈H₂₀N₄O₃S·H was 373.1329 found 373.1335 | 8.28 (d, 1H, pyridazine, J = 9.8 Hz), 7.59-7.64 (m, 1H, Ph), 7.53 (dd, 1H, Ph, J = 1.5, 7.4 Hz), 7.34 (d, 1H, pyridazine, J = 9.7 Hz), 7.27 (app d, 1H, Ph, J = 7.9 Hz), 7.13 (d t, 1H, Ph, J = 0.7, 7.4 Hz), 4.20 (t, 1H, SCH, J = 6.8 Hz), 3.93-4.02 and 3.68-3.76 (2dq, 2H, OCH₂, J = 7.0 Hz and 10.9 Hz), 3.78 (s, 3H, OCH₃), 1.82-1.99 (m, 2H, CH₂), 1.00 (t, 3H, CH₃, J = 7.0 Hz), 0.90 (t, 3H, CH₃, J = 7.3 Hz). |
| 71 | calculated for C₁₈H₂₀N₄O₃S·H was 373.1329 found 373.1325 | 8.31 (d, 1H, pyridazine, J = 9.8 Hz), 7.92-7.96 (d m, 1H, Ph), 7.86-7.89 (m, 1H, Ph), 7.52 (t, 1H, Ph, J = 8.0 Hz), 7.39 (d, 1H, pyridazine, J = 9.3 Hz), 7.13-7.17 (d m, 1H, Ph), 4.57 (t, 1H, SCH, J = 6.7 Hz), 4.06-4.16 and 3.93-4.02 (2dq, 2H, OCH₂, J = 7.1 Hz and 11.0 Hz), 3.89 (s, 3H, OCH₃), 1.99-2.09 (m, 2H, CH₂), 1.06 (t, 3H, CH₃, J = 7.0 Hz), 1.03 (t, 3H, CH₃, J = 7.4 Hz). |

### REFERENCE EXAMPLE 55: 2-((3-(2-Fluorophenyl)-[1,2,4]triazolo[4,3-b]pyridazin-6-yl)thio)butanoic acid

To a solution of ethyl 2-((3-(2-fluorophenyl)-[1,2,4]triazolo[4,3-b]pyridazin-6-yl)thio)butanoate (1 mmol) in dioxane (3 mL) was added 0.5 ml of sodium hydroxide (2N), and the reaction mixture was stirred at room temperature. After 2 hours, the mixture was evaporated to dryness, and the obtained residue dissolved in water (2 mL) and then acidified with a hydrochloric acid solution. The resulting precipitate was filtered, washed with water, and dried to give 2-((3-(2-fluorophenyl)-[1,2,4]triazolo[4,3-b]pyridazin-6-yl)thio)butanoic acid.

The method above was adapted to give the following compounds which are reference examples:

| Example | MS | NMR |
|---|---|---|
| 53 | calculated for C₁₆H₁₃F₃N₄O₂S.H was 383.0784 found 383.0786 | 8.63 (d, 2H, Ph, J = 8.2 Hz), 8.22 (d, 1H, pyridazine, J = 9.8 Hz), 7.94 (d, 2H, Ph, J = 8.6 Hz), 7.31 (d, 1H, pyridazine, J = 9.4 Hz), 4.26 (t, 1H, SCH, J = 5.5 Hz), 1.96-2.07 (m, 2H, CH₂), 1 (t, 3H, CH₃, J = 7.4 Hz). |
| 54 | calculated for C₁₅H₁₂F₂N₄O₂S.H was 351.0721 found 351.0723. | 8.37-8.43 (m, 1H, Ph), 8.24-8.27 (m, 1H, Ph), 8.21 (d, 1H, pyridazine, J = 9.4 Hz), 7.63-7.7 (m, 1H, Ph), 7.29 (d, 1H, pyridazine, J = 9.8 Hz), 4.27 (t, 1H, SCH, J = 5.8 Hz), 1.99-2.05 (m, 2H, CH₂), 1.01 (t, 3H, CH₃, J = 7.2 Hz). |
| 55 | calculated for C₁₅H₁₃FN₄O₂S·H was 333.0816 found 333.0819 | 8.2 (d, 1H, pyridazine, J = 9.4 Hz), 7.89-7.92 (td, 1H, Ph, J = 1.6 Hz and 7.8 Hz), 7.64-7.7 (m, 1H, Ph), 7.41-7.49 (m, 2H, Ph), 7.28 (d, 1H, pyridazine, J = 9.4 Hz), 4.05 (m, 1H, SCH), 1.86-1.98 (m, 2H, CH₂), 0.87 (t, 3H, CH₃, J = 7.4 Hz). |
| 56 | calculated for C₁₅H₁₃FN₄O₂S.H was 333.0816 found 333.0822 | 8.21-8.28 (m, 2H, Ph), 8.13 (d, 1H, pyridazine, J = 9.8 Hz), 7.62-7.68 (td, 1H, Ph, J = 6.2 Hz and 8.2 Hz), 7.38-7.43 (td, 1H, Ph, J = 2.2 Hz and 7.9 Hz), 7.23 (d, 1H, pyridazine, J = 9.4 Hz), 4.13 (dd, 1H, SCH, J = 4.3 Hz and 3.2 Hz), 1.89-2.08 (m, 2H, CH₂), 0.96 (t, 3H, CH₃, J = 7.4 Hz). |

### REFERENCE EXAMPLE 18: 2-((3-(2-Fluorophenyl)-[1,2,4]triazolo[4,3-b]pyridazin-6-yl)thio) butan-amide

To 10 ml of dry methanol saturated with ammonia was added 0.5 mmol of ethyl 2-((3-(2-fluorophenyl)-[1,2,4]triazolo[4,3-b]pyridazin-6-yl)thio)butanoate. The pressure tube was stoppered tightly, and the solution was stirred for 16 hours at 50 °C. The white precipitate was collected and washed with cold methanol to give 2-((3-(2-fluorophenyl)-[1,2,4]triazolo[4,3-b]pyridazin-6-yl)thio)butanamide.

The following compounds were prepared by adaptation of the method above (the following compounds are reference examples):

| Example | MS | NMR |
|---|---|---|
| 66 | calculated for C₁₆H₁₇N₅O₂S.H was 344.1176 found 344.1185 | 8.32-8.37 (d app t, 2H, Ph, J = 2.9, 12.0 Hz), 8.24 (d, 2H, pyridazine, J = 9.7 Hz), 7.86 (br s, 1H, NH₂), 7.36 (br s, 1H, NH₂), 7.29 (d, 1H, pyridazine, J = 9.6 Hz), 7.16 (d app t, 2H, Ph, J = 2.1, 12.0 Hz), 4.38 (d d, 1H, SCH, J = 5.6, 7.9 Hz), 3.87 (s, 3H, OCH₃), 1.95-2.09 (m, 2H, CH₂), 1.03 (t, 3H, CH₃, J = 7.4 Hz) |
| 69 | calculated for C₁₆H₁₆N₄O₃S.H was 344.1176 found 344.1172 | 8.24 (d, 1H, pyridazine, J = 9.7 Hz), 7.61-7.64 (m, 1H, NH₂), 7.61-7.62 (m, 1H, Ph), 7.55-7.59 (m, 1H, Ph), 7.29 (d, 1H, pyridazine, J = 9.8 Hz), 7.24 (d, 1H, Ph, J = 8.6 Hz), 7.23 (br s, 1H, NH₂), 7.13 (d t, 1H, Ph, J = 0.7, 7.4 Hz), 4.03 (dd, 1H, SCH, J = 5.4, 8.2 Hz), 3.77 (s, 3H, OCH₃), 1.83-1.95 (m, 2H, CH₂), 0.81 (t, 3H, CH₃, J = 7.4 Hz) |
| 72 | calculated for C₁₆H₁₆N₄O₃S.H was 344.1176 found 345.1162 | 8.27 (d, 1H, pyridazine, J = 9.4 Hz), 8.02-8.06 (d m, 1H, Ph), 7.86 (d d, 1H, Ph, J = 1.6, 2.7 Hz), 7.85 (br s, 1H, NH₂), 7.54 (t, 1H, Ph, J = 8.0 Hz), 7.34 (br s, 1H, NH₂), 7.33 (d, 1H, pyridazine, J = 9.7 Hz), 7.14-7.18 (d m, 1H, Ph), 4.38 (d d, 1H, SCH, J = 5.4, 8.6 Hz), 3.87 (s, 3H, OCH₃), 1.94-2.10 (m, 2H, CH₂), 1.01 (t, 3H, CH₃, J = 7.5 Hz). |
| 78 | calculated for C₁₆H₁₄F₃N₅OS.H was 382.0943 found 382.0945 | 8.64 (d, 2H, Ph, J = 8.2 Hz), 8.31 (d, 1H, pyridazine, J = 9.8 Hz), 7.97 (d, 2H, Ph, J = 8.2 Hz), 7.86 (br s, NH), 7.38-7.7.4 (m, 2H, pyridazine and NH), 4.4 (t, 1H, SCH, J = 6.6 Hz), 1.99-2.03 (m, 2H, CH₂), 1.02 (t, 3H, CH₃, J = 7.4 Hz). |
| 79 | calculated for C₁₅H₁₃F₂N₅OS.H was 350.0881 found 350.0888. | 8.34-8.39 (m, 1H, Ph), 8.28-8.30 (d, 1H, pyridazine, J = 9.8 Hz and br s, 1H, Ph), 7.83 (br s, 1H, NH), 7.65-7.72 (m, 1H, Ph), 7.35-7.37 (d, 1H, pyridazine, J = 9.7 Hz and br s, 1H, NH), 4.37 (t, 1H, SCH, J = 6.8 Hz), 1.98-2.04 (m, 2H, CH₂), 1.01 (t, 3H, CH₃, J = 7.4 Hz). |
| 80 | calculated for C₁₅H₁₄FN₅OS.H was 332.0975 found 332.0983 | 8.28 (d, 1H, pyridazine, J = 9.3 Hz), 7.93-7.97 (td, 1H, Ph, J = 2 Hz and 7.5 Hz), 7.66-7.71 (m, 2H, Ph and NH), 7.43-7.5 (m, 2H, Ph), 7.34 (d, 1H, pyridazine, J = 9.4 Hz), 4.26 (brs, 1H, NH), 4.13-4.16 (dd, SCH, J = 4.7 Hz and 8.6 Hz), 1.86-2 (m, 2H, CH₂), 0.88 (t, 3H, CH₃, J = 7.2 Hz). |
| 81 | calculated for C₁₅H₁₄FN₅OS.H was 332.0975 found 332.0979 | 8.29 (d, 1H, pyridazine, J = 9.8 Hz), 8.27 (d, 1H, Ph, J = 8.3 Hz), 8.14-8.17 (m, 1H, Ph), 7.87 (br s, 1H, NH), 7.64-7.7 (m, 1H, Ph), 7.4-7.46 (td, 1H, Ph, J = 2 Hz and 8.2 Hz), 7.35-7.7.37 (m, 2H, pyridazine and NH), 4.37-4.41 (dd, SCH, J = 5.5 Hz and 8.6 Hz), 1.95-2.1 (m, 2H, CH₂), 1.02 (t, 3H, CH₃, J = 7.4 Hz). |

### REFERENCE EXAMPLE 94: 2-((3-(2-Fluorophenyl)-[1,2,4]triazolo[4,3-b]pyridazin-6-yl)thio)-1-(piperidin-1-yl)butan-1-one

To a solution of 3-(2-fluorophenyl)-[1,2,4]triazolo[4,3-b]pyridazine-6-thiol (1 mmol) and cesium carbonate (1.5 equiv.) in DMF (5 mL) was added 2-bromo-1-(piperidin-1-yl)butan-1-one (1.5 equiv.) and the mixture was stirred at 70 °C for 3 hours. The reaction was monitored by thin-layer chromatography and was shown to be complete at this time. The solvent was removed in vacuo and the residue was flash chromatographed to give 2-((3-(2-fluorophenyl)-[1,2,4]triazolo[4,3-b]pyridazin-6-yl)thio)-1-(piperidin-1-yl)butan-1-one.

The following compounds were prepared by an adaptation of the method above (the following compounds are reference examples):

| Example | MS | NMR |
|---|---|---|
| 67 | calculated for C₂₁H₂₅N₅O₂S.H was 412.1802 found 412.1809 | 8.25-8.30 (d app t, 2H, Ph, J = 2.7, 11.7 Hz), 8.24 (d, 2H, pyridazine, J = 10.2 Hz), 7.30 (d, 1H, pyridazine, J = 9.8 Hz), 7.16 (d app t, 2H, Ph, J = 2.7, 9.0 Hz), 5.03 (d d, 1H, SCH, J = 5.6, 7.5 Hz), 3.86 (s, 3H, OCH₃), 3.50-3.60 (m, 2H, NCH₂), 3.32-3.46 (m, 2H, NCH₂), 2.05-2.13 (m, 1H, CH₂), 1.93-2.03 (m, 2H, CH₂), 1.39-1.66 (m, 6H, CH₂CH₂CH₂), 0.97 (t, 3H, CH₃, J = 7.3 Hz). |
| 70 | calculated for C₂₁H₂₅N₅O₂S.H was 412.1802 found 412.1783 | 8.25 (d, 1H, pyridazine, J = 9.8 Hz), 7.60-7.65 (m, 1H, Ph), 7.55 (d d, 1H, Ph, J = 2.0, 7.8 Hz), 7.29 (d, 1H, pyridazine, J = 9.8 Hz), 7.28 (d, 1H, Ph, J = 8.2 Hz), 7.15 (d t, 1H, Ph, J = 0.8, 7.5 Hz), 4.70 (dd, 1H, SCH, J = 5.9, 7.4 Hz), 3.77 (s, 3H, OCH₃), 3.50-3.58 (m, 1H, NCH₂), 3.26-3.34 (m, 1H, NCH₂), 3.10-3.18 (m, 1H, NCH₂), 2.88-2.97 (m, 1H, NCH₂), 1.84-1.96 (m, 1H, CH₂), 1.68-1.79 (m, 1H, CH₂), 1.15-1.59 (m, 5H, CH₂CH₂CH₂), 0.79 (t, 3H, CH₃, J = 7.2 Hz). |
| 73 | calculated for C₂₁H₂₅N₅O₂S.H was 412.1802 found 412.1809 | 8.28 (d, 1H, pyridazine, J = 9.3 Hz), 7.92-7.94 (m, 1H, Ph), 7.90-7.92 (m, 1H, Ph), 7.52-7.57 (m, 1H, Ph), 7.34 (d, 1H, pyridazine, J = 9.4 Hz), 7.16-7.20 (d m, 1H, Ph), 5.09 (d d, 1H, SCH, J = 5.5, 7.4 Hz), 3.86 (s, 3H, OCH₃), 3.54-3.66 (m, 1H, NCH₂), 3.38-3.47 (m, 1H, NCH₂), 1.94-2.12 (m, 2H, CH₂), 1.37-1.68 (m, 5H, CH₂CH₂CH₂), 0.96 (t, 3H, CH₃, J = 7.5 Hz). |
| 94 | calculated for C₂₀H₂₂FN₅OS.H was 400.1601 found 400.1612 | 8.30 (d, 1H, pyridazine, J = 9.4 Hz), 7.88-7.92 (td, 1H, Ph, J = 1.9 Hz and 7.4 Hz), 7.68-7.74 (m, 1H, Ph), 7.5-7.53 (m, 1H, Ph), 7.44-7.48 (td, 1H, Ph, J = 1.2 Hz and 7.8 Hz), 7.35 (d, 1H, pyridazine, J = 9.8 Hz), 4.85 (dd, SCH, J = 5.8 Hz and 7.4 Hz), 3.53-3.58 and 3.38-3.41 (2m, 2H, NCH₂), 3.2-3.26 and 3.11-3.16 (2m, 2H, NCH₂), 1.91-1.98 and 1.76-1.83 (2m, 2H, CH₂), 1.3-1.57 (m, 6H, 3CH₂), 0.82 (t, 3H, CH₃, J = 7.4 Hz). |
| 95 | calculated for C₂₁H₂₂F₃N₅OS.H was 450.1569 found 450.1576 | 8.54 (d, 2H, Ph, J = 7.8 Hz), 8.33 (d, 1H, pyridazine, J = 9.8 Hz), 7.99 (d, 2H, Ph, J = 8.6 Hz), 7.39 (d, 1H, pyridazine, J = 9.4 Hz), 5.03 (dd, SCH, J = 5.9 Hz and 6.5 Hz), 3.39-3.54 (2m, 4H, NCH₂), 1.95-2.11 (m, 2H, CH₂), 1.42-1.6 (2m, 6H, 3CH₂), 0.96 (t, 3H, CH₃, J = 7.2 Hz). |
| 96 | calculated for C₂₀H₂₁F₂N₅OS.H was 418.1507 found 418.1504 | 8.39-8.45 (m, 1H, Ph), 8.3 (d, 1H, pyridazine, J = 9.8 Hz), 8.16-8.2 (m, 1H, Ph), 7.69-7.76 (m, 1H, Ph), 7.37 (d, 1H, pyridazine, J = 9.8 Hz), 5.07 (dd, SCH, J = 5.5 Hz and 7.4 Hz), 3.36-3.62 (2m, 2H, NCH₂), 1.97-2.12 (m, 2H, CH₂), 1.44-1.63 (m, 6H, 3CH₂), 0.95 (t, 3H, CH₃, J = 7.5 Hz). |
| 97 | calculated for C₂₀H₂₂FN₅OS.H was 400.1601 found 400.1586 | 8.3 (d, 1H, pyridazine, J = 9.8 Hz), 8.16-8.22 (m, 2H, Ph), 7.65-7.7 (td, 1H, Ph, J = 6.3 Hz and 8.2 Hz), 7.4-7.47 (m, 1H, Ph), 7.36 (d, 1H, pyridazine, J = 9.8 Hz), 5.08 (dd, SCH, J = 5.4 Hz and 7.8 Hz), 3.55-3.64 (m, 2H, CHNCH), 3.31-3.44 (m, 2H, CHNCH), 1.97-2.13 (m, 2H, CH₂), 1.4-1.63 (m, 6H, 3CH₂), 0.95 (t, 3H, CH₃, J = 6.7 Hz) |

### REFERENCE EXAMPLE 51: 1-(3-Methylpiperidin-1-yl)-2-((3-(thiophen-2-yl)-[1,2,4]triazolo[4,3-b] pyridazin-6-yl)thio)butan-1-one

¹H NMR (400 MHz, Me₂SO-d₆): 8.26-8.3 (m, 1H, pyridazine), 8.11-8.16 (m, 1H, thienyl), 7.91-7.92 (m, 1H, thienyl), 7.33-7.36 (m, 2H, thienyl and pyridazine), 5.14-5.26 (m, 1H, SCH), 3.85-4.3 (2m, 2H, NCH₂), 1.97-2.15 and 2.32-3.26 (3m, 4H, NCH₂ and CH₂), 1.09-1.79 (m, 5H, 2CH₂ and CH), 0.97-1.02 (m, 3H, CH₃), 0.65-0.87 (m, 3H, CH₃). FABMS (M+H) calculated for C₁₉H₂₃N₅OS₂.H was 402.1416 found 402.1420.

### REFERENCE

### EXAMPLE 52: N-Cyclohexyl-2-((3-(thiophen-2-yl)-[1,2,4]triazolo[4,3-b]pyridazin-6-yl)thio) butanamide

¹H NMR (400 MHz, Me₂SO-d₆): 8.31 (d, 1H, NH, J = 7.8 Hz), 8.25 (d, 1H, pyridazine, J = 9.8 Hz), 8.2 (dd, 1H, thienyl, J = 1.2 Hz and 3.9 Hz), 7.88 (dd, 1H, thienyl, J = 1.1 Hz and 5.1 Hz), 7.34 (dd, 1H, thienyl, J = 3.5 and 5.1 Hz), 7.31 (d, 1H, pyridazine, J = 9.4 Hz), 4.5 (dd, 1H, SCH, J = 5 Hz and 9 Hz), 3.51-3.56 (m, 1H, NCH), 1.95-2.14 (2m, 2H, CH₂), 1.08-1.76 (4m, 10H, 5CH₂), 1.02 (t, 3H, CH₃, J = 7.2 Hz). FABMS (M+H) calculated for C₁₉H₂₃N₅OS₂.H was 402.1416 found 402.1423.

### REFERENCE EXAMPLE 106: 2-((3-Phenyl-[1,2,4]triazolo[4,3-b]pyridazin-6-yl)thio)-1-(piperidin-1-yl)butan-1-one

HPLC 99.8%, Column: Bondclon C18, t_{R} = 8.8 minutes, H₃CCN/H₂O(40-90%). ¹H NMR (400 MHz, Me₂SO-d₆): 8.33 (d d, 2H, Ph, J = 2 and 3.9 Hz), 8.28 (d, 1H, pyridazine, J = 9.4 Hz), 7.58-7.64 (m, 3H, Ph), 7.33 (d, 1H, pyridazine, J = 9.8 Hz), 5.03 (d d, SCH, J = 5.5 and 7.8 Hz), 3.36-3.42 and 3.51-3.6 (2 m, 4H, NCH₂), 1.94-2.11 and 1.43-1.6 (2 m, 4H, 4CH₂), 0.95 (t, 3H, CH₃, J = 7.4 Hz). FABMS (M+H) calculated for C₂₀H₂₃N₅OS.H was 382.1696 found 382.1695.

### REFERENCE EXAMPLE 82: Ethyl 2-((3-(thiophen-2-yl)-[1,2,4]triazolo[4,3-b]pyridazin-6-yl)oxy) butanoate

Sodium hydride (1.5 equiv., 60% in mineral oil) was added at 0 °C to a solution of ethyl 2-hydroxybutyrate (1.5 equiv.) in dry DMF (7 mL) under argon atmosphere and the mixture was stirred for 10 min. To this mixture was added 6-chloro-3-(thiophen-2-yl)-[1,2,4]triazolo[4,3-b]pyridazine (1 mmol) dissolved in DMF (3 mL). After 30 min, TLC showed that all the staring material was consumed. The solution was extracted with ethyl acetate, washed with water twice and concentrated. The crude syrup thus obtained was purified by flash column chromatography to give ethyl 2-((3-(thiophen-2-yl)-[1,2,4]triazolo[4,3-b]pyridazin-6-yl)oxy)butanoate. ¹H NMR (400 MHz, Me₂SO-d₆): 8.4 (d, 1H, pyridazine, J = 9.8 Hz), 8.06 (dd, 1H, thienyl, J = 1.2 Hz and 3.5 Hz), 7.86 dd, 1H, thienyl, J = 1.2 Hz and 5.1 Hz), 7.32 (dd, 1H, thienyl, J = 3.9 Hz and 5.1 Hz), 7.25 (d, 1H, pyridazine, J = 9.8 Hz), 5.26 (dd, 1H, SCH, J = 5.4 Hz and 7 Hz), 4.09-4.21 (m, 2H, OCH₂), 1.96-2.09 (m, 2H, CH₂), 1.16 (t, 3H, CH₃, J = 7.2 Hz), 1.07 (t, 3H, CH₃, J = 5.6 Hz). FABMS (M+H) calculated for C₁₅H₁₆N₄O₃S.H was 333.1015 found 333.1022. EXAMPLE 83: 2-((3-(Thiophen-2-yl)-[1,2,4]triazolo[4,3-b]pyridazin-6-yl)oxy)butanoic acid To a solution of ethyl 2-((3-(thiophen-2-yl)-[1,2,4]triazolo[4,3-b]pyridazin-6-yl)oxy)butanoate. (1 mmol) in dioxane (3 mL) was added 0.5 ml of sodium hydroxide (2N), and the reaction mixture was stirred at room temperature. After 2 hours, the mixture was evaporated to dryness, and the obtained residue dissolved in water (2 mL) and then acidified with a hydrochloric acid solution. The resulting precipitate was filtered, washed with water, and dried to give 2-((3-(thiophen-2-yl)-[1,2,4]triazolo[4,3-b]pyridazin-6-yl)oxy)butanoic acid. ¹H NMR (400 MHz, Me₂SO-d₆): 8.3 (d, 1H, pyridazine, J = 9.8 Hz), 8.17 (d, 1H, thienyl, J = 2.7 Hz), 7.79 (dd, 1H, thienyl, J = 0.8 Hz and 5.1 Hz), 7.26 (dd, 1H, thienyl, J = 5.6 Hz and 5.1 Hz), 7.15 (d, 1H, pyridazine, J = 9.8 Hz), 5.04 (br m, 1H, OCH), 1.94-1.99 (m, 2H, CH₂), 1.04 (t, 3H, CH₃, J = 7.4 Hz). FABMS (M+H) calculated for C₁₃H₁₂N₄O₃S.H was 305.0702 found 305.0705.

### REFERENCE EXAMPLE 128: 2-methyl-1-(piperidin-1-yl)-3-((3-(thiophen-2-yl)-[1,2,4]triazolo[4,3-b]pyridazin-6-yl)oxy)propan-1-one

### a) Preparation of 6-chloro-3-(thiophen-2-yl)-[1,2,4]triazolo[4,3-b]pyridazine.

To a solution of 3,6-dichloropyridazine (15.0 g, 100.6 mmol) and thiophene-2-carbohydrazide (15.7 g, 110.7 mmol) in xylene (60 mL) at room temperature was added triethylamine hydrochloride (15.2 g, 110.7 mmol) and stirred under reflux for 6 h, when TLC showed consumption of the starting material. The reaction mixture was cooled to room temperature, concentrated *in vacuo* and the residue was diluted with water (200 ml), extracted with EtOAc (3x100 mL). The combined organic layer was washed with brine solution, dried over anhydrous Na₂SO₄. The solvent was evaporated under reduced pressure and crystallized in diethyl ether to afford the product as yellow color solid (3.60 g).

### b) Preparation of 3-(thiophen-2-yl)-[1,2,4]triazolo[4,3-b]pyridazin-6-ol.

To a solution of 6-chloro-3-(thiophen-2-yl)-[1,2,4]triazolo[4,3-b]pyridazine (0.50 g, 2.10 mmol) in DMSO (10 ml) and water (2 ml) was added K₂CO₃ (0.38 g, 2.70 mmol) and stirred at 100 °C for 4 h. After completion, the reaction mixture was diluted with water (50 ml), extracted with EtOAc (3x30 mL). The combined organic layer was washed with water, brine solution, dried over anhydrous Na₂SO₄. The solvent was evaporated under reduced pressure to afford the product as yellow color solid (0.37 g).

### c) Preparation of 2-(hydroxymethyl)acrylic acid.

To a solution of ethyl 2-(hydroxymethyl)acrylate (1.00 g, 7.60 mmol) in THF (8 mL), MeOH (2 ml) and water (1 ml) was added LiOH×H₂O (0.65 g, 15.0 mmol) at 0 °C and the reaction mixture was stirred for 3 h at room temperature. After completion, the mixture was concentrated and acidified to pH 4 by 1N HCl and extracted with EtOAc (3x30 mL) and combined organic extracts were washed with brine, dried over anhy. Na₂SO₄ and concentrated to afford the product as light yellow color oil (0.680 g).

### d) Preparation of 2-(hydroxymethyl)-1-(piperidin-1-yl)prop-2-en-1-one.

To a stirred solution of 2-(hydroxymethyl)acrylic acid (0.65 g, 6.30 mmol) in DMF (4 mL) at 0 °C were added piperidine (1.08 g, 12.0 mmol), DIPEA (1.60 g, 12.0 mmol) and HATU (2.80 g, 7.50 mmol) at 0 °C and stirred at room temperature for 4 h, when TLC showed completion of the reaction. The reaction mixture was diluted with water (15 ml), extracted with EtOAc (2x50 mL). The combined organic layer was dried over anhy. Na₂SO₄. The solvent was evaporated under reduced pressure to afford the product as light yellow color oil (0.18 g).

### e) Preparation of 3-hydroxy-2-methyl-1-(piperidin-1-yl)propan-1-one.

To a solution of 2-(hydroxymethyl)-1-(piperidin-1-yl)prop-2-en-1-one (0.18 g, 1.06 mmol) in ethanol (3 ml) was added 10 % Pd/C (0.10 g, 50 % wet) slowly and then stirred under hydrogen atmosphere (balloon pressure) at room temperature for 1 h when TLC showed completion of the reaction. After completion, the reaction mixture was filtered through Celite pad and washed with ethanol. The combined filtrate was concentrated under reduced pressure to give the product as light yellow color oil (0.11 g).

### f) Preparation of 2-methyl-1-(piperidin-1-yl)-3-((3-(thiophen-2-yl)-[1,2,4]triazolo[4,3-b]pyri-dazin-6-yl)oxy)propan-1-one

To a stirred solution of 3-(thiophen-2-yl)-[1,2,4]triazolo[4,3-b]pyridazin-6-ol (0.10 g, 0.45 mmol) in THF (2 mL) at 0 °C were added 3-hydroxy-2-methyl-1-(piperidin-1-yl)propan-1-one (0.10 g, 0.64 mmol) and TPP (0.23 g, 0.90 mmol) and stirred for 5 min. Then DIAD (0.18 g, 0.90 mmol) was added at 0 °C and stirred at room temperature for 1 h, when TLC showed completion of the reaction. The reaction mixture was diluted with water (15 ml), extracted with EtOAc (2x15 mL). The combined organic layer was dried over anhy. Na₂SO₄. The solvent was evaporated under reduced pressure and purified by prep HPLC. The appropriate compound fractions were concentrated *in vacuo* to afford the product as off white color solid (0.006 g); ¹H NMR [DMSO d₆]: 8.35 (d, 1H), 8.15 (m, 1H), 7.9 (m, 1H) 7.35 (m, 1H), 7.1 (m, 1H), 4.65 (m, 1H), 4.35 (m, 1H), 3.5 (m, 5H), 1.7-1.4 (m, 6H) and 1.5 ppm (d, 3H); LCMS m/z: 372.42 (M+H), 743.55 (2M+H).

### REFERENCE EXAMPLE 129: 2-Methyl-1-(piperidin-1-yl)-3-((3-(thiophen-2-yl)-[1,2,4]triazolo[4,3-b] pyridazin-6-yl)amino)propan-1-one

### a) Preparation of 6-chloro-3-(thiophen-2-yl)-[1,2,4]triazolo[4,3-b]pyridazine.

To a solution of 3,6-dichloropyridazine (15.0 g, 100.6 mmol) and thiophene-2-carbohydrazide (15.7 g, 110.7 mmol) in xylene (60 mL) at room temperature was added triethylamine hydrochloride (15.2 g, 110.7 mmol) and stirred under reflux for 6 h, when TLC showed consumption of the starting material. The reaction mixture was cooled to room temperature, concentrated *in vacuo* and the residue was diluted with water (200 mL), extracted with EtOAc (3x100 mL). The combined organic layer was washed with brine solution, dried over anhydrous Na₂SO₄. The solvent was evaporated under reduced pressure and crystallized in diethyl ether to afford the product as yellow color solid (3.60 g).

### b) Preparation of ethyl 2-methyl-3-((3-(thiophen-2-yl)-[1,2,4]triazolo[4,3-b]pyridazin-6-yl) amino)propanoate.

To a solution of 6-chloro-3-(thiophen-2-yl)-[1,2,4]triazolo[4,3-b]pyridazine (0.50 g, 2.12 mmol) and ethyl 3-amino-2-methylpropanoate (0.27 g, 2.12 mmol) in DMF (8 mL) at 0 °C was added K₂CO₃ (0.37 g, 2.70 mmol) and stirred at 100 °C for 12 h, when TLC showed completion of the reaction. The reaction mixture was cooled to room temperature, diluted with water (50 mL), extracted with EtOAc (3x30 mL). The combined organic layer was washed with brine solution, dried over anhydrous Na₂SO₄. The solvent was evaporated under reduced pressure to afford the crude product as brown color oil (0.12 g).

### c) Preparation of 2-methyl-3-((3-(thiophen-2-yl)-[1,2,4]triazolo[4,3-b]pyridazin-6-yl)amino) propanoic acid.

To a solution of ethyl 2-methyl-3-((3-(thiophen-2-yl)-[1,2,4]triazolo[4,3-b]pyridazin-6-yl) amino)propanoate (0.10 g, 0.30 mmol) in THF (2 mL), MeOH (0.5 mL) and water (0.5 mL) was added NaOH (0.05 g, 1.20 mmol) at 0 °C and the reaction mixture was stirred for 4 h at room temperature. After completion, the mixture concentrated and the residue was diluted with water and extracted with ether. The aqueous layer was acidified to pH 4 by 1N HCl and extracted with EtOAc (3x30 mL) and combined organic extracts were washed with brine, dried over anhydrous Na₂SO₄ and concentrated to afford the product as white color solid (0.06 g).

### d) Preparation of 2-methyl-1-(piperidin-1-yl)-3-((3-(thiophen-2-yl)-[1,2,4]triazolo[4,3-b]pyri-dazin-6-yl)amino)propan-1-one.

To a stirred solution of 2-methyl-3-((3-(thiophen-2-yl)-[1,2,4]triazolo[4,3-b]pyridazin-6-yl) amino)propanoic acid (0.06 g, 0.19 mmol) in DMF (1 mL) at 0 °C were added piperidine (0.022 g, 0.26 mmol), DIPEA (0.10 g, 0.76 mmol) and HATU (0.093 g, 0.27 mmol) at 0 °C and stirred at room temperature for 12 h, when TLC showed completion of the reaction. The reaction mixture was diluted with water (15 mL), extracted with EtOAc (2x25 mL). The combined organic layer was dried over anhydrous Na₂SO₄. The solvent was evaporated under reduced pressure to give the impure product which was purified by prep. HPLC. The appropriate compound fractions were concentrated *in vacuo* to afford the product as white color solid (0.022 g). ¹H NMR [DMSO d₆] 8.1 (d, 1H), 7.95 (d, 1H), 7.8 (d, 1H), 7.8-7.7 (m, 1H), 7.3 (t, 1H), 6.85 (d, 1H), 3.6-3.4 (m, 3H), 1.6-1.3 (m, 6H) and 1.2 ppm (d, 3H); LCMS m/z: 371.36 (M+H), 741.49 (2M+H).

### REFERENCE EXAMPLE 130: 2-Methyl-3-(methyl(3-(thiophen-2-yl)-[1,2,4]triazolo[4,3-b]pyri-dazin-6-yl)amino)-1-(piperidin-1-yl)propan-1-one

### a) Preparation of 6-chloro-3-(thiophen-2-yl)-[1,2,4]triazolo[4,3-b]pyridazine

To a solution of 3,6-dichloropyridazine (10.0 g, 67.1 mmol) and thiophene-2-carbohydrazide (11.4 g, 80.5 mmol) in xylene (60 mL) at room temperature was added triethylamine hydrochloride (11.08 g, 80.5 mmol) and stirred under reflux for 12 h, when TLC showed consumption of the starting material. The reaction mixture was cooled to room temperature, concentrated *in vacuo* and the residue was diluted with (200 mL), extracted with EtOAc (3x100 mL). The combined organic layer was washed with brine solution, dried over anhydrous Na₂SO₄. The solvent was evaporated under reduced pressure and crystallized in diethyl ether to afford the product as yellow color solid (2.80 g).

### b) Preparation of ethyl 2-methyl-3-(methyl(3-(thiophen-2-yl)-[1,2,4]triazolo[4,3-b]pyridazin-6-yl)amino)propanoate.

To a solution of 6-chloro-3-(thiophen-2-yl)-[1,2,4]triazolo[4,3-b]pyridazine (0.25 g, 1.056 mmol) and ethyl 2-methyl-3-(methylamino)propanoate (0.154 g, 1.056 mmol) in DMF (3 mL) at 0 °C was added K₂CO₃ (0.19 g, 1.37 mmol) and stirred at room temperature for 4 h, when TLC showed presence of most the starting material. The mixture was then heated at 80 °C for 6 h. After completion, the reaction mixture was cooled to room temperature, diluted with water (50 mL), extracted with EtOAc (3x30 mL). The combined organic layer was washed with brine solution, dried over anhydrous Na₂SO₄. The solvent was evaporated under reduced pressure and purified by flash column chromatography with 50 to 52 % EtOAc in hexane. The appropriate compound fractions were concentrated *in vacuo* to afford the product as brown color oil (0.10 g).

### c) Preparation of 2-methyl-3-(methyl(3-(thiophen-2-yl)-[1,2,4]triazolo[4,3-b]pyridazin-6-yl) amino)propanoic acid.

To a solution of ethyl 2-methyl-3-(methyl(3-(thiophen-2-yl)-[1,2,4]triazolo[4,3-b]pyridazin-6-yl)amino)propanoate (0.08 g, 0.23 mmol) in THF (2 mL) and water (1 mL) was added LiOH (0.05 g, 1.15 mmol) at 0 °C and the reaction mixture was stirred for 6 h at room temperature. After completion, the mixture was cooled to 0 °C and acidified to pH 4 by 1N HCl and extracted with EtOAc (3x30 mL). The combined organic extracts were washed with brine, dried over anhydrous Na₂SO₄ and concentrated to afford the product as yellow color semi solid (0.065 g).

### d) Preparation of 2-methyl-3-(methyl(3-(thiophen-2-yl)-[1,2,4]triazolo[4,3-b]pyridazin-6-yl)amino)-1-(piperidin-1-yl)propan-1-one.

To a stirred solution of 2-methyl-3-(methyl(3-(thiophen-2-yl)-[1,2,4]triazolo[4,3-b]pyridazin-6-yl)amino)propanoic acid (0.065 g, 0.20 mmol) in DMF (1 mL) at 0 °C were added piperidine (0.03 g, 0.29 mmol), DIPEA (0.105 g, 0.82 mmol) and HATU (0.10 g, 0.27 mol) at 0 °C and stirred at room temperature for 12 h, when TLC showed completion of the reaction. The reaction mixture was diluted with water (15 mL), extracted with EtOAc (2x15 mL). The combined organic layer was dried over anhydrous Na₂SO₄. The solvent was evaporated under reduced pressure and purified by flash column chromatography with 60 to 65 % EtOAc in hexane. The appropriate compound fractions were concentrated *in vacuo* to afford the product as white color solid (0.026 g). ¹H NMR [400 MHz, DMSO d₆]: 8.19 (broad d, 1H), 7.97 (d, 1H), 7.74 (d, 1H), 7.3-7.2 (m, 2H), 3.8-3.7 (m, 1H), 3.65-3.55 (m, 1H), 3.5-3.4 (m, 2H) 3.4-3.1 (m, 3H) 3.1 (s, 3H), 1.56-1.2 (m, 6H) and 1.06 ppm (d, 3H); LCMS m/z: 385.40 (M+H⁺), 769.49 (2M+H⁺).

### REFERENCE EXAMPLE 131: 1-(Piperidin-1-yl)-3-(3-(thiophen-2-yl)-[1,2,4]triazolo[4,3-b]pyridazin-6-yl)propane-1,3-dione.

### a) Preparation of methyl 3-(thiophen-2-yl)-[1,2,4]triazolo[4,3-b]pyridazine-6-carboxylate.

A solution of methyl 6-chloropyridazine-3-carboxylate (3.00 g, 17.3 mmol) and thiophene-2-carbohydrazide (2.71 g, 19.1 mmol) in *n*-butanol (30 mL) was stirred at 140 °C for 5 h, when TLC showed consumption of the starting material. The reaction mixture was cooled to room temperature, concentrated *in vacuo,* and the residue was washed with diethyl ether and dried to afford the impure product as yellow color solid (4.50 g).

### b) Preparation of 1-(piperidin-1-yl)-3-(3-(thiophen-2-yl)-[1,2,4]triazolo[4,3-b]pyridazin-6-yl)propane-1,3-dione.

To a solution of 1-(piperidin-1-yl)ethanone (1.56 g, 12.0 mmol) in THF (10 ml) at -78 °C was added LiHMDS (lithium bis(trimethylsilyl)amide; 22.8 mL, 1.0 M in THF, 22.0 mmol) and stirred at -78 °C. After 45 min, a solution of methyl 3-(thiophen-2-yl)-[1,2,4]triazolo[4,3-b]pyridazine-6-carboxylate (2.00 g, 7.60 mmol) in THF (10 mL) was added and slowly warmed to room temperature over 1 h and stirred for 4 h. After completion, the reaction mixture was quenched with aq. NH₄Cl solution, acidified (to pH∼1) with 1N HCl, extracted with EtOAc (3x30 mL). The combined organic layer was washed with brine solution, dried over anhydrous Na₂SO₄. The solvent was evaporated under reduced pressure and purified by flash column chromatography over silica gel (100-200 mesh) with 75 to 80 % EtOAc in hexane to give the impure product which was further purified by prep HPLC. The appropriate compound fractions were concentrated *in vacuo* to afford the product as yellow color solid (0.060 g). ¹H NMR [400 MHz, DMSO d₆ with D₂O added]: 8.47 (d, 1H), 8.2 (broad, 1H) 7.92-7.77 (m, 2H), 7.4-7.3 (m, 1H), 3.6-3.3 (m, 4H) and 1.7-1.3 ppm (m, 6H); LCMS m/z: 356.39 (M+H⁺).

### REFERENCE

### EXAMPLE 132: 1-(Piperidin-1-yl)-2-((3-(thiophen-2-yl)-[1,2,4]triazolo[4,3-b]pyridazin-6-yl)methoxy)propan-1-one

### a) Preparation of methyl 3-(thiophen-2-yl)-[1,2,4]triazolo[4,3-b]pyridazine-6-carboxylate.

A solution of methyl 6-chloropyridazine-3-carboxylate (3.00 g, 17.3 mmol) and thiophene-2-carbohydrazide (2.71 g, 19.1 mmol) in *n*-butanol (30 mL) was stirred at 140 °C for 5 h, when TLC showed consumption of the starting material. The reaction mixture was cooled to room temperature, concentrated *in vacuo,* and the residue was washed with diethyl ether and dried to afford the impure product as yellow color solid (4.50 g).

### b) Preparation of 3-(thiophen-2-yl)-[1,2,4]triazolo[4,3-b]pyridazine-6-carboxylic acid.

To a solution of methyl 3-(thiophen-2-yl)-[1,2,4]triazolo[4,3-b]pyridazine-6-carboxylate (1.00 g, 3.84 mmol) in THF (2 mL) and MeOH (2 mL) was added a solution of NaOH (0.46 g, 11.5 mmol) in water (2 mL) at 0 °C and the reaction mixture was stirred for 3 h at room temperature. After completion, the mixture was concentrated, diluted with water, washed with diethyl ether. The aqueous layer was acidified to pH 6 by 1N HCl, extracted with EtOAc (3x30 mL). The combined organic extracts were washed with brine, dried over anhydrous Na₂SO₄ and concentrated to afford the product as yellow color solid (0.80 g).

### c) Preparation of (3-(thiophen-2-yl)-[1,2,4]triazolo[4,3-b]pyridazin-6-yl)methanol.

To a solution of 3-(thiophen-2-yl)-[1,2,4]triazolo[4,3-b]pyridazine-6-carboxylic acid (1.20 g, 4.80 mmol) in THF (15 mL) was added BH₃:DMS (dimethylsulfide) complex (1.50 g, 1.92 mmol) slowly at 0 °C and then stirred at room temperature for 3 h when TLC showed completion of the reaction. After completion, the reaction mixture was quenched with methanol and stirred at room temperature for 4 h. The solvent was concentrated under reduced pressure and purified by flash chromatography over silica gel (100-200 mesh) with 75 to 80 % EtOAc in hexane to give the product as yellow color solid (0.31 g, 27.43 %) which was confirmed by ¹H NMR and LCMS spectra.

### d) Preparation of methyl 2-((3-(thiophen-2-yl)-[1,2,4]triazolo[4,3-b]pyridazin-6-yl)methoxy) propanoate.

To a solution of (3-(thiophen-2-yl)-[1,2,4]triazolo[4,3-b]pyridazin-6-yl)methanol (0.15 g, 0.64 mmol) and methyl 2-bromopropanoate (0.16 g, 0.96 mmol) in DMF (2 mL) at 0 °C was added NaH (0.03 g, 60 % in oil, 1.28 mmol) and stirred at room temperature for 1 h. After completion, the reaction mixture was diluted with water (50 ml), extracted with EtOAc (3x30 mL). The combined organic layer was washed with water, brine solution, dried over anhydrous Na₂SO₄. The solvent was evaporated under reduced pressure and purified by flash column chromatography over silica gel (100-200 mesh) with 75 to 80 % EtOAc in hexane. The appropriate compound fractions were concentrated *in vacuo* to afford the product as yellow color oil (0.075 g).

### e) Preparation of 2-((3-(thiophen-2-yl)-[1,2,4]triazolo[4,3-b]pyridazin-6-yl)methoxy)propanoic acid.

To a solution of methyl 2-((3-(thiophen-2-yl)-[1,2,4]triazolo[4,3-b]pyridazin-6-yl)methoxy) propanoate (0.07 g, 0.22 mmol) in THF (2 mL), MeOH (1 mL) and water (1 mL) was added LiOH (0.037 g, 0.88 mmol) at 0 °C and the reaction mixture was stirred for 2 h at RT. After completion, the mixture was concentrated, diluted with water, washed with diethyl ether. The aqueous layer was acidified to pH 6 by 1N HCl, extracted with EtOAc (3x30 mL). The combined organic extracts were washed with brine, dried over anhydrous Na₂SO₄ and concentrated to afford the product as white color solid (0.052 g).

### f) Preparation of 1-(piperidin-1-yl)-2-((3-(thiophen-2-yl)-[1,2,4]triazolo[4,3-b]pyridazin-6-yl) methoxy)propan-1-one.

To a stirred solution of 2-((3-(thiophen-2-yl)-[1,2,4]triazolo[4,3-b]pyridazin-6-yl)methoxy) propanoic acid (0.050 g, 0.16 mmol) in DMF (2 mL) were added piperidine (0.02 g, 0.23 mmol), DIPEA (0.083 g, 0.64 mmol) and HATU (0.08 g, 0.20 mmol) at 0 °C and stirred at RT for 4 h, when TLC showed completion of the reaction. The reaction mixture was diluted with water (15 mL), extracted with EtOAc (2x15 mL). The combined organic layer was dried over anhydrous Na₂SO₄, evaporated under reduced pressure and purified by prep. HPLC. The appropriate compound fractions were concentrated *in vacuo* to afford the product as sticky white color solid (0.021 g). ¹H NMR [400 MHz, DMSO d₆]: 8.6 (d, 1H), 8.2 (d, 1H), 7.9 (d, 1H), 7.5 (t, 1H), 7.3 (m, 1H), 4.7 (m, 3H), 3.4 (3H) and 1.5-1.3 ppm (m, 10 H); LCMS m/z: 372.19 (M+H⁺).

### REFERENCE EXAMPLE 133: 1-(Piperidin-1-yl)-2-(((3-(thiophen-2-yl)-[1,2,4]triazolo[4,3-b]pyrida-zin-6-yl)methyl)amino)propan-1-one.

### a) Preparation of 3-(thiophen-2-yl)-[1,2,4]triazolo[4,3-b]pyridazine-6-carbonitrile.

A solution of 6-chloropyridazine-3-carbonitrile (5.00 g, 35.8 mmol) and thiophene-2-carbohydrazide (5.09 g, 35.8 mmol) in *n*-butanol (70 mL) was stirred at 110 °C for 3 h, when TLC showed consumption of the starting material. The reaction mixture was cooled to room temperature, diluted with diethyl ether and the precipitated solid was filtered, washed with diethyl ether and dried to afford the product as yellow color solid (7.10 g).

### b) Preparation of (3-(thiophen-2-yl)-[1,2,4]triazolo[4,3-b]pyridazin-6-yl)methanamine.

To a solution of 3-(thiophen-2-yl)-[1,2,4]triazolo[4,3-b]pyridazine-6-carbonitrile (4.00 g, 17.6 mmol) in methanol (500 mL) and CHCl₃ (20 mL) was added 10 % Pd/C (4.00 g, 50 % wet) slowly and then stirred under hydrogen atmosphere (balloon pressure) at room temperature for 48 h when TLC showed completion of the reaction. After completion, the reaction mixture was filtered through a Celite pad and washed with methanol. The combined filtrate was concentrated under reduced pressure and purified by flash chromatography over silica gel (60-120 mesh) with 10 % methanol in CH₂Cl₂ to give the product as brown color solid (1.50 g).

### c) Preparation of methyl 2-(((3-(thiophen-2-yl)-[1,2,4]triazolo[4,3-b]pyridazin-6-yl)methyl) amino)propanoate.

To a solution of (3-(thiophen-2-yl)-[1,2,4]triazolo[4,3-b]pyridazin-6-yl)methanamine (0.70 g, 3.02 mmol) and methyl 2-bromopropanoate (0.66 g, 3.92 mmol) in DMF (3 mL) at 0 °C was added K₂CO₃ (1.24 g, 9.06 mmol) and stirred at room temperature for 6 h. After completion, the reaction mixture was diluted with water (50 mL), extracted with EtOAc (3x30 mL). The combined organic layer was washed with water, brine solution, dried over anhydrous Na₂SO₄. The solvent was evaporated under reduced pressure and purified by flash column chromatography over silica gel (60-120 mesh) with 70 to 80 % EtOAc in hexane. The appropriate compound fractions were concentrated *in vacuo* to afford the product as brown color oil (0.085 g).

### d) Preparation of 2-(((3-(thiophen-2-yl)-[1,2,4]triazolo[4,3-b]pyridazin-6-yl)methyl)amino) propanoic acid.

To a solution of methyl 2-(((3-(thiophen-2-yl)-[1,2,4]triazolo[4,3-b]pyridazin-6-yl)methyl) amino)propanoate (0.085 g, 0.26 mmol) in THF (3 mL), MeOH (0.2 ml) and water (3 mL) was added LiOH (0.067 g, 1.60 mmol) at 0 °C and the reaction mixture was stirred for 6 h at room temperature. After completion, the mixture was concentrated and acidified to pH 4 by 1N HCl and extracted with EtOAc (3x30 mL) and combined organic extracts were washed with brine, dried over anhydrous Na₂SO₄ and concentrated to afford the product as off white color solid (0.080 g).

### e) Preparation of 1-(piperidin-1-yl)-2-(((3-(thiophen-2-yl)-[1,2,4]triazolo[4,3-b]pyridazin-6-yl)methyl)amino)propan-1-one.

To a stirred solution of 2-(((3-(thiophen-2-yl)-[1,2,4]triazolo[4,3-b]pyridazin-6-yl)methyl)amino) propanoic acid (0.080 g, 0.20 mmol) in DMF (3 mL) at 0 °C were added piperidine (0.03 g, 0.30 mmol), DIPEA (0.103 g, 0.82 mmol) and HATU (0.10 g, 0.26 mmol) at 0 °C and stirred at room temperature for 3 h, when TLC showed completion of the reaction. The reaction mixture was diluted with water (15 mL), extracted with EtOAc (2x15 mL). The combined organic layer was dried over anhydrous Na₂SO₄. The solvent was evaporated under reduced pressure and purified by flash column chromatography over silica gel (60-120 mesh) with 5 to 6 % MeOH in CH₂Cl₂. The appropriate compound fractions were concentrated *in vacuo* to afford the product as off white color solid (0.03 g). ¹H NMR [400 MHZ, DMSO d₆]: 8.35 (d, 1H), 8.2 (d, 1H), 7.85 (d, 1H), 7.5 (d, 1H), 7.32 (t, 1H), 3.9 (d, 1H), 3.8 (d, 1H), 3.65 (m, 1H), 3.5-3.2 (m, 5 H), 1.6-1.2 (m, 6H) and 1.1 ppm (d, 3H); LCMS m/z: 371.31 (M+H⁺), 741.38 (2M+H⁺).

### EXAMPLE 134: 2-(Methyl((3-(thiophen-2-yl)-[1,2,4]triazolo[4,3-b]pyridazin-6-yl)methyl)amino)-1-(piperidin-1-yl)propan-1-one.

### a) Preparation of 3-(thiophen-2-yl)-[1,2,4]triazolo[4,3-b]pyridazine-6-carbonitrile.

A solution of 6-chloropyridazine-3-carbonitrile (5.00 g, 35.8 mmol) and thiophene-2-carbohydrazide (5.09 g, 35.8 mmol) in *n*-butanol (70 mL) was stirred at 110 °C for 3 h, when TLC showed consumption of the starting material. The reaction mixture was cooled to room temperature, diluted with diethyl ether and the precipitated solid was filtered, washed with diethyl ether and dried to afford the product as yellow color solid (7.10 g).

### b) Preparation of (3-(thiophen-2-yl)-[1,2,4]triazolo[4,3-b]pyridazin-6-yl)methanamine.

To a solution of 3-(thiophen-2-yl)-[1,2,4]triazolo[4,3-b]pyridazine-6-carbonitrile (4.00 g, 17.6 mmol) in methanol (500 mL) and CHCl₃ (20 mL) was added 10 % Pd/C (4.00 g, 50 % wet) slowly and then stirred under hydrogen atmosphere (balloon pressure) at room temperature for 48 h when TLC showed completion of the reaction. After completion, the reaction mixture was filtered through Celite pad and washed with methanol. The combined filtrate was concentrated under reduced pressure and purified by flash chromatography over silica gel (60-120 mesh) with 10 % methanol in CH₂Cl₂ to give the product as brown color solid (1.50 g).

### c) Preparation of methyl 2-(((3-(thiophen-2-yl)-[1,2,4]triazolo[4,3-b]pyridazin-6-yl)methyl) amino)propanoate.

To a solution of (3-(thiophen-2-yl)-[1,2,4]triazolo[4,3-b]pyridazin-6-yl)methanamine (0.80 g, 3.40 mmol) and Et₃N (1.04 g, 10.2 mmol) in CH₂Cl₂ (20 mL) was added methyl 2-bromopropanoate (0.866 g, 5.10 mmol) and stirred at room temperature for 12 h. After completion, the reaction mixture was diluted with water (50 mL), extracted with CH₂Cl₂ (3x30 mL). The combined organic layer was washed with water, brine solution, dried over anhydrous Na₂SO₄. The solvent was evaporated under reduced pressure and purified by flash column chromatography over silica gel (60-120 mesh) with 70 to 80 % EtOAc in hexane. The appropriate compound fractions were concentrated *in vacuo* to afford the product as brown color oil (0.18 g).

### d) Preparation of methyl 2-(methyl((3-(thiophen-2-yl)-[1,2,4]triazolo[4,3-b]pyridazin-6-yl)methyl)amino)propanoate.

To a solution of methyl 2-(((3-(thiophen-2-yl)-[1,2,4]triazolo[4,3-b]pyridazin-6-yl)methyl) amino)propanoate (0.180 g, 0.50 mmol) and K₂CO₃ (0.14 g, 0.10 mmol) in CH₃CN (10 mL) was added methyl iodide (0.105 g, 0.75 mmol) at 0 °C and stirred at room temperature for 4 h. After completion, the reaction mixture was diluted with water (50 mL), extracted with EtOAc (3x30 mL). The combined organic layer was washed with water, brine solution, dried over anhydrous Na₂SO₄. The solvent was evaporated under reduced pressure to afford the product as brown color oil (0.12 g).

### e) Preparation of 2-(methyl((3-(thiophen-2-yl)-[1,2,4]triazolo[4,3-b]pyridazin-6-yl)methyl) amino)propanoic acid.

To a solution of methyl 2-(methyl((3-(thiophen-2-yl)-[1,2,4]triazolo[4,3-b]pyridazin-6-yl)methyl)amino)propanoate (0.12 g, 0.30 mmol) in THF (3 mL), MeOH (0.1 ml) and water (3 ml) was added LiOH (0.076 g, 1.50 mmol) at 0 °C and the reaction mixture was stirred for 6 h at room temperature. After completion, the mixture was concentrated and acidified to pH 4 by 1N HCl and concentrated under reduced pressure to afford the product as brown color solid (0.080 g).

### f) Preparation of 2-(methyl((3-(thiophen-2-yl)-[1,2,4]triazolo[4,3-b]pyridazin-6-yl)methyl) amino)-1-(piperidin-1-yl)propan-1-one.

To a stirred solution of 2-(methyl((3-(thiophen-2-yl)-[1,2,4]triazolo[4,3-b]pyridazin-6-yl)methyl) amino)propanoic acid (0.080 g, 0.20 mmol) in DMF (5 mL) at 0 °C were added piperidine (0.025 g, 0.30 mmol), DIPEA (0.103 g, 0.82 mmol) and HATU (0.10 g, 0.26 mmol) at 0 °C and stirred at room temperature for 4 h, when TLC showed completion of the reaction. The reaction mixture was diluted with water (15 mL), extracted with EtOAc (2x50 mL). The combined organic layer was dried over anhydrous Na₂SO₄. The solvent was evaporated under reduced pressure and purified by flash column chromatography over silica gel (60-120 mesh) with 5 to 6 % MeOH in CH₂Cl₂. The appropriate compound fractions were concentrated *in vacuo* to afford the product as off white color solid (0.017 g). ¹H NMR [400 MHz, DMSO d₆]: 8.4 (d, 1H), 8.22 (m, 1H), 7.8 (m, 1H), 7.4-7.3 (m, 2H), 4.0-3.4 (m, 7H), 2.2 (s, 3H), 1.6-1.3 (m, 7 H) and 1.15 ppm (d, 3H); LCMS m/z: 385.36 (M+H⁺).

### REFERENCE EXAMPLE 135: (3-(Pyrimidin-2-yl)-[1,2,4]triazolo[4,3-b]pyridazin-6-yl)methanol.

To a suspension butyl 3-(pyrimidin-2-yl)-[1,2,4]triazolo[4,3-b]pyridazine-6-carboxylate and methyl 3-(pyrimidin-2-yl)-[1,2,4]triazolo[4,3-b]pyridazine-6-carboxylate (∼2:1, 140 mg, ∼0.49 mmol) ethanol (5 mL) added NaBH₄ (20 mg, 0.53 mmol). After 2 h quenched with 1N HCl and partitioned between CHCl₃ / iPrOH (7:3) and water. The organic extract was dried over anhydrous Na₂SO₄ and concentrated. The recovered 82 mg of crude product was chromatographed on silica gel eluting CHCl₃ /MeOH (grad 0 → 40% MeOH). Recovered 11 mg of a white solid. HRMS found (M+H)⁺: 229.08324

### REFERENCE EXAMPLE 136: N-(2-Amino-2-oxoethyl)-3-(pyrimidin-2-yl)-[1,2,4]triazolo[4,3-b]pyridazine-6-carboxamide

To a mixture of 3-(pyrimidin-2-yl)-[1,2,4]triazolo[4,3-b]pyridazine-6-carboxylic acid (100 mg, 0.41 mmol) in DMF (2 mL) was added HATU (173 mg, 0.45 mmol). In a separate flask to a mixture of glycinamide hydrochloride (64 mg, 0.58 mmol) in DMF (2 mL) was added DIPEA (0.10 ml, 0.58 mmol). After 30 min., the glycinamide mixture was cannulated into the 3-(pyrimidin-2-yl)-[1,2,4]triazolo[4,3-b]pyridazine-6-carboxylic acid mixture follwed by the addition of DIPEA (0.24 mL, 1.4 mmol). After stirring overnight the mixture was filtered and the collected solid was washed with a small amount of MeOH. NMR indicated that the recovered 85 mg of crude product contained a small amount of DMF. The crude product was suspended in water, heated, cooled and filtered. Recovered: 60 mg of a beige solid. HRMS found (M+H)⁺: 299.09965

### REFERENCE EXAMPLE 137: 3-(3-(Thiazol-2-yl)-[1,2,4]triazolo[4,3-b]pyridazin-6-yl)propan-1-ol

A stirred mixture of 3-(6-chloropyridazin-3-yl)propan-1-ol (WO 2007/048802A1) (101 mg, 0.58 mmol) and diazenyl(thiazol-2-yl)methanone (127 mg, 0.89 mmol) in nBuOH (3 mL) in a sealed vessel was microwaved at 150 °C for 5 h. After cooling the reaction was concentrated then chromatographed on silica gel using CHCl₃ /MeOH (grad 0 → 10% MeOH). Recovered: 99 mg of a solid. HRMS found (M+H)⁺: 262.07599

### REFERENCE EXAMPLE 138: Ethyl 2-(3-(pyrimidin-2-yl)-[1,2,4]triazolo[4,3-b]pyridazine-6-carboxamido)acetate

To a mixture of 3-(pyrimidin-2-yl)-[1,2,4]triazolo[4,3-b]pyridazine-6-carboxylic acid (200 mg, 0.826 mmol) in DMF (2 mL) was added HATU (345 mg, 0.908 mmol). In a separate flask to a mixture of glycine ethyl ester hydrochloride (161 mg, 1.16 mmol) in DMF (2 mL) was added DIPEA (0.20 mL, 1.61 mmol). After 30 min., the glycine ethyl ester mixture was cannulated into the 3-(pyrimidin-2-yl)-[1,2,4]triazolo[4,3-b]pyridazine-6-carboxylic acid mixture follwed by the addition of DIPEA (0.48 mL, 2.76 mmol). After stirring overnight the mixture was partitioned between CHCl₃ and water. The organic extract was washed In aq HCl, water, dried over anhydrous Na₂SO₄ and concentrated. The recovered 248 mg of crude product were chromatographed on silica gel with CHCl₃ /MeOH (grad 0 → 10% MeOH). Recovered 191 mg of a foam. HRMS found (M+H)⁺: 328.11510

### REFERENCE EXAMPLE 139: 2-(6-(3-(Pyrrolidin-1-yl)propyl)-[1,2,4]triazolo[4,3-b]pyridazin-3-yl)thiazole

### a) Preparation of 3-(3-(thiazol-2-yl)-[1,2,4]triazolo[4,3-b]pyridazin-6-yl)propyl 4-methylben-zenesulfonate

To a mixture of 3-(3-(thiazol-2-yl)-[1,2,4]triazolo[4,3-b]pyridazin-6-yl)propan-1-ol (100 mg, 0.38 mmol) in CH₂Cl₂ at 0 °C was added Et₃N (0.11 mL, 0.765 mmol) followed by DMAP (23.2 mg, 0.19 mmol). After 10 min. added TsCl (tosyl chloride; 87.5 mg, 0.46 mmol). After 1 h, the reaction mixture was warmed to ambient temperature and allowed to stir for 3 hrs. After partitioning between CH₂Cl₂ and water the aqueous layer was extracted with CH₂Cl₂. The combined organic extracts were dried over anhydrous Na₂SO₄ and concentrated. The crude product was chromatographed on silica gel using CHCl₃ /MeOH (grad 0 → 5% MeOH). Recovered: two fractions one of which yielded 27 mg of a slightly colored oil.

### b) Preparation of 2-(6-(3-(pyrrolidin-1-yl)propyl)-[1,2,4]triazolo[4,3-b]pyridazin-3-yl)thiazole

To a mixture of 3-(3-(thiazol-2-yl)-[1,2,4]triazolo[4,3-b]pyridazin-6-yl)propyl 4-methylbenzenesulfonate (27 mg, 0.065 mmol) in THF (2 mL) and DMF (0.5 mL) was added pyrrolidine (0.054 mL, 0.65 mmol). After 6 days the reaction mixture was concentrated and chromatographed on silica gel eluting CHCl₃ (A)/ 30 % solution of 2% conc. aq. NH4OH /18% MeOH and 80% CHCl₃ in CHCl₃ (B) (grad 0 → 100% B). Recovered: 9 mg. HRMS found (M+H)⁺: 315.13901

### REFERENCE EXAMPLE 140: (R)-Methyl 2-(3-(pyrimidin-2-yl)-[1,2,4]triazolo[4,3-b]pyridazine-6-carboxamido)propanoate.

The title compound was prepared by an adaptation of the method used in EXAMPLE 136. HRMS found (M+H)⁺: 328.11515

### REFERENCE EXAMPLE 141: 2-(3-(Pyrimidin-2-yl)-[1,2,4]triazolo[4,3-b]pyridazine-6-carboxamido) acetic acid

To a solution of ethyl 2-(3-(pyrimidin-2-yl)-[1,2,4]triazolo[4,3-b]pyridazine-6-carboxamido) acetate (174 mg, 0.532 mmol) in THF (5 mL) and MeOH (2.5 mL) added 2N aq. NaOH (0.27 mL, 0.532 mmol). After 2 h resulting mixture was filtered and collected solid washed small amount MeOH. Recovered: 45 mg of a pink solid. HRMS found (M+H)⁺: 300.08389

### REFERENCE EXAMPLE 142: N-(2-Morpholino-2-oxoethyl)-3-(pyrimidin-2-yl)-[1,2,4]triazolo[4,3-b]pyridazine-6-carboxamide

To a solution of 2-(3-(pyrimidin-2-yl)-[1,2,4]triazolo[4,3-b]pyridazine-6-carboxamido)acetic acid (33 mg, 0.110 mmol) in DMF (ml) was added HATU (52 mg, 0.137 mmol). After 30 min., morpholine (0.014 ml, 0.154 mmol) was added followed by the addition of DIPEA (0.064 mL, 0.364 mmol). After stirring overnight the mixture was partitioned between CHCl₃ and water. The organic extract was dried over anhydrous Na₂SO₄ and concentrated. The crude product was chromatographed on silica gel with CHCl₃ /MeOH (grad 0 → 10% MeOH). Recovered 30 mg. HRMS found (M+H)⁺: 369.14133

### REFERENCE EXAMPLE 143: (R)-2-(3-(pyrimidin-2-yl)-[1,2,4]triazolo[4,3-b]pyridazine-6-carboxamido)propanoic acid

To a solution of (R)-methyl 2-(3-(pyrimidin-2-yl)-[1,2,4]triazolo[4,3-b]pyridazine-6-carboxamido)propanoate (220 mg, 0.672 mmol) in THF (5 mL) and MeOH (2.5 mL) added 2N aq. NaOH (0.336 mL, 0.672 mmol). After 2 h the reaction mixture was concentrated, acidified with 1N HCl and partitioned between CHCl₃ / iPrOH (7:3) and water. The organic extract was dried over anhydrous Na₂SO₄ and concentrated. Recovered: 126 mg of a solid. HRMS found (M+H)⁺: 314.09917

### REFERENCE EXAMPLE 144: (R)-N-(1-Oxo-1-(pyrrolidin-1-yl)propan-2-yl)-3-(pyrimidin-2-yl)-[1,2,4]triazolo[4,3-b]pyridazine-6-carboxamide

The title product was prepared by an adaptation of the method used in EXAMPLE 142. HRMS found (M+H)⁺: 367.16225

### REFERENCE EXAMPLE 145: (R)-N-(1-Morpholino-1-oxopropan-2-yl)-3-(pyrimidin-2-yl)-[1,2,4]triazolo[4,3-b]pyridazine-6-carboxamide

The title product was prepared by an adaptation of the method used in EXAMPLE 142. Recovered: 27 mg. HRMS found (M+H)⁺: 383.15707

### REFERENCE EXAMPLE 146: (R)-N-(1-(Dipropylamino)-1-oxopropan-2-yl)-3-(pyrimidin-2-yl)-[1,2,4]triazolo[4,3-b]pyridazine-6-carboxamide

The title product was prepared by an adaptation of the method used in EXAMPLE 142. Recovered: 22 mg. HRMS found (M+H)⁺: 397.20948

### REFERENCE EXAMPLE 147: 2-Methyl-1-(pyrrolidin-1-yl)-3-(3-(thiazol-2-yl)-[1,2,4]triazolo[4,3-b]pyridazin-6-yl)propan-1-one

### a) Preparation of ethyl 3-(6-chloropyridazin-3-yl)-2-methylpropanoate

To a stirred solution of LDA (2M THF/hexanes/heptanes, 39.4 ml, 78.7 mmol) in THF (50 mL) at -78 °C, ethyl propionate (9.05 ml, 78.7 mmol) in THF (50 mL) was added drop-wise. After 30 min. a solution of 3-chloro-6-(chloromethyl)pyridazine (5.347 g, 32.8 mmol) in THF (50 mL) was added drop-wise and the reaction was allowed to slowly warm RT and stirred overnight. After partitioning between EtOAc and water the organic extract was washed with water (2x), sat. brine, dried over anhydrous Na₂SO₄ and concentrated. The crude product was chromatographed on silica gel eluting EtOAC / hexanes (grad 0 → 50% EtOAc). Recovered: 2.323 g of a reddish oil.

### b) Preparation of ethyl 2-methyl-3-(3-(thiazol-2-yl)-[1,2,4]triazolo[4,3-b]pyridazin-6-yl)propanoate

A stirred mixture of ethyl 3-(6-chloropyridazin-3-yl)-2-methylpropanoate (400 mg, 1.75 mmol) and diazenyl(thiazol-2-yl)methanone (247 mg, 1.725 mmol) in EtOH (10 mL) in a sealed vessel was microwaved at 120 °C for 1 h. After cooling the reaction was concentrated then chromatographed on silica gel eluting CHCl₃/MeOH (grad 0 → 2% MeOH). Recovered: 289 mg of a solid.

### c) Preparation of 2-methyl-3-(3-(thiazol-2-yl)-[1,2,4]triazolo[4,3-b]pyridazin-6-yl)propanoic acid

To a solution of ethyl 2-methyl-3-(3-(thiazol-2-yl)-[1,2,4]triazolo[4,3-b]pyridazin-6-yl)propanoate (394 mg, 1.24 mmol) in THF (20 mL) and MeOH (10 mL) added 2N aq. NaOH (0.62 mL, 1.24 mmol). After 2 h reaction was neutralized with 6N aq. HCl and concentrated. The product was used as is.

### d) Preparation of 2-methyl-1-(pyrrolidin-1-yl)-3-(3-(thiazol-2-yl)-[1,2,4]triazolo[4,3-b]pyri-dazin-6-yl)propan-1-one.

The title compound was prepared by an adaptation of the method used in EXAMPLE 142. Recovered: 12 mg. HRMS found (M+H)⁺: 343.13367

### REFERENCE EXAMPLE 148: Ethyl 2-methyl-3-(3-(pyrimidin-2-yl)-[1,2,4]triazolo[4,3-b]pyridazin-6-yl)propanoate

A stirred mixture of ethyl 3-(6-chloropyridazin-3-yl)-2-methylpropanoate (400 mg, 1.75 mmol) and pyrimidine-2-carbohydrazide (266 mg, 1.92 mmol) in EtOH (10 mL) in a sealed vessel was microwaved at 120 °C for 4 h. After cooling the reaction was concentrated then chromatographed on silica gel eluting CHCl₃/MeOH (grad 0 → 5% MeOH). Recovered: 373 mg of a solid. HRMS found (M+H)⁺: 313.14101

### REFERENCE EXAMPLE 149: 2-Methyl-1-(piperidin-1-yl)-3-(3-(thiazol-2-yl)-[1,2,4]triazolo[4,3-b]pyridazin-6-yl)propan-1-one

The title compound was prepared by an adaptation of the method used in EXAMPLE 142. Recovered: 14 mg. HRMS found (M+H)⁺: 357.14860

### REFERENCE EXAMPLE 150: 2-Methyl-1-morpholino-3-(3-(pyrimidin-2-yl)-[1,2,4]triazolo[4,3-b]pyridazin-6-yl)propan-1-one

The title compound was prepared by an adaptation of the method used in EXAMPLE 142. Recovered: 19 mg. HRMS found (M+H)⁺: 354.16815

### REFERENCE EXAMPLE 151: N,N,2-Trimethyl-3-(3-(pyrimidin-2-yl)-[1,2,4]triazolo[4,3-b]pyrida-zin-6-yl)propanamide

The title compound was prepared by an adaptation of the method used in EXAMPLE 142. Recovered: 21 mg. HRMS found (M+H)⁺: 312.15714

### REFERENCE EXAMPLE 152: 2-Methyl-N,N-dipropyl-3-(3-(pyrimidin-2-yl)-[1,2,4]triazolo[4,3-b]pyridazin-6-yl)propanamide

The title compound was prepared by an adaptation of the method used in EXAMPLE 142. Recovered: 30 mg. HRMS found (M+H)⁺: 368.21960

### REFERENCE EXAMPLE 153: 1-(Pyrrolidin-1-yl)-3-(3-(thiazol-2-yl)-[1,2,4]triazolo[4,3-b]pyridazin-6-yl)propan-1-one

The title compound was prepared by an adaptation of the method used in EXAMPLE 142. Recovered: 36 mg. HRMS found (M+H)⁺: 329.11804

### REFERENCE EXAMPLE 154: 1-Morpholino-3-(3-(thiazol-2-yl)-[1,2,4]triazolo[4,3-b]pyridazin-6-yl) propan-1-one

The title compound was prepared by an adaptation of the method used in EXAMPLE 142. Recovered: 33 mg. HRMS found (M+H)⁺: 329.11804

### REFERENCE EXAMPLE 155: Ethyl 4-(3-(thiazol-2-yl)-[1,2,4]triazolo[4,3-b]pyridazin-6-yl)butanoate

### a) Preparation of ethyl 4-(6-chloropyridazin-3-yl)butanoate:

To a stirred solution of 3-chloro-6-iodopyridazine (500 mg, 2.080 mmol) and Pd(Ph₃P)₄ (168 mg, 0.146 mmol) in THF (16 mL) while bubbling Ar through reaction (∼15 min.) was added a solution of (4-ethoxy-4-oxobutyl)zinc(II) bromide (0.5M in THF, 5.407 mL, 2.70 mmol). After stirring overnight, the reaction mixture was portioned between EtOAc and water / aq. HCl (pH ∼2). The organic extract was dried (Na₂SO₄) and concentrated. The product was purifies by chromatography using silica gel and hexane / EtOAc (grad 0 → 100% EtOAc). Recovered: 314 mg.

### b) Preparation of ethyl 4-(3-(thiazol-2-yl)-[1,2,4]triazolo[4,3-b]pyridazin-6-yl)butanoate:

The title compound was prepared by adaptation of the procedure used for ethyl 2-methyl-3-(3-(thiazol-2-yl)-[1,2,4]triazolo[4,3-b]pyridazin-6-yl)propanoate. Recovered: 273 mg. HRMS found (M+H)⁺: 318.10288

### REFERENCE EXAMPLE 156: 1-(Pyrrolidin-1-yl)-4-(3-(thiazol-2-yl)-[1,2,4]triazolo[4,3-b]pyridazin-6-yl)butan-1-one

The title compound was prepared by an adaptation of the method used in EXAMPLE 142. Recovered: 42 mg. HRMS found (M+H)⁺: 343.13390

### REFERENCE EXAMPLE 157: 1-Morpholino-3-(3-(thiazol-2-yl)-[1,2,4]triazolo[4,3-b]pyridazin-6-yl) propan-1-one

The title compound was prepared by an adaptation of the method used in EXAMPLE 142. Recovered: 33 mg. HRMS found (M+H)⁺: 359.12867

### REFERENCE EXAMPLE 158: Ethyl 4-(3-(thiophen-2-yl)-[1,2,4]triazolo[4,3-b]pyridazin-6-yl)bu-tanoate

The title compound was prepared by adaptation of ethyl 2-methyl-3-(3-(thiazol-2-yl)-[1,2,4]triazolo[4,3-b]pyridazin-6-yl)propanoate. Recovered: 1.201 g. HRMS found (M+H)⁺: 317.10608

### REFERENCE EXAMPLE 159: 1-(Pyrrolidin-1-yl)-4-(3-(thiophen-2-yl)-[1,2,4]triazolo[4,3-b]pyrida-zin-6-yl)butan-1-one

The title compound was prepared by an adaptation of the method used in EXAMPLE 142. Recovered: 22 mg. HRMS found (M+H)⁺: 342.13890

### REFERENCE EXAMPLE 160: N,N-Dimethyl-4-(3-(thiophen-2-yl)-[1,2,4]triazolo[4,3-b]pyridazin-6-yl)butanamide

The title compound was prepared by an adaptation of the method used in EXAMPLE 142. Recovered: 33 mg. HRMS found (M+H)⁺: 316.12178

### REFERENCE EXAMPLE 161: 1-Morpholino-4-(3-(thiophen-2-yl)-[1,2,4]triazolo[4,3-b]pyridazin-6-yl)butan-1-one

The title compound was prepared by an adaptation of the method used in EXAMPLE 142. Recovered: 33 mg. HRMS found (M+H)⁺: 358.13311

### REFERENCE EXAMPLE 162: 2-Methyl-1-(pyrrolidin-1-yl)-3-(3-(thiophen-2-yl)-[1,2,4]triazolo[4,3-b]pyridazin-6-yl)propan-1 -one

The title compound was prepared by an adaptation of the method used in EXAMPLE 142. Recovered: 10.4 mg. HRMS found (M+H)⁺: 342.13846

### REFERENCE EXAMPLE 163: 2-Methyl-1-morpholino-3-(3-(thiophen-2-yl)-[1,2,4]triazolo[4,3-b]pyridazin-6-yl)propan-1-one

The title compound was prepared by an adaptation of the method used in EXAMPLE 142. Recovered: 24 mg. HRMS found (M+H)⁺: 358.13314

### REFERENCE EXAMPLE 164: N,N,2-Trimethyl-3-(3-(thiophen-2-yl)-[1,2,4]triazolo[4,3-b]pyridazin-6-yl)propanamide

The title compound was prepared by an adaptation of the method used in EXAMPLE 142. Recovered: 24.7 mg. HRMS found (M+H)⁺: 316.12242

### REFERENCE EXAMPLE 165: 1-Morpholino-3-(3-(thiophen-2-yl)-[1,2,4]triazolo[4,3-b]pyridazin-6-yl)propan-1-one

The title compound was prepared by an adaptation of the method used in EXAMPLE 142. Recovered: 6.1 mg. HRMS found (M+H)⁺: 344.11767

### REFERENCE EXAMPLE 166: N,N-Dimethyl-3-(3-(thiophen-2-yl)-[1,2,4]triazolo[4,3-b]pyridazin-6-yl)propanamide

The title compound was prepared by an adaptation of the method used in EXAMPLE 142. Recovered: 14.8 mg. HRMS found (M+H)⁺: 302.10738

### REFERENCE EXAMPLE 167: 3-(Pyrimidin-2-yl)-[1,2,4]triazolo[4,3-b]pyridazine-6-carboxylic acid

2-Pyrimidinecarbohydrazide (276.3mg, 2.0 mmol) and methyl 6-chloro-pyridazine-3-carboxylate (345 mg, 2.0 mmol) were dissolved in n-BuOH (30 mL) and the mixture was heated to 140-145°C for 8 h. The reaction was concentrated *in vacuo* and the residue was dissolved in ethyl acetate (50 mL), washed with water and brine, dried over MgSO₄, filtered, and evaporated. The crude product was chromatographed to provide the intermediate methyl ester (332 mg, 65% yield) as a light tan powder.

The methyl ester (300 mg) was dissolved in a 2:1 THF/methanol (12 mL) mixture and treated with 2N NaOH (1.0 mL). The mixture was stirred for 4 h at 20 °C, evaporated *in vacuo,* diluted with water (20 mL) and acidified with 6N HCl to pH∼2. The solid precipitates were collected, washed with water and dried to afford 3-(pyrimidin-2-yl)-[1,2,4]triazolo[4,3-b]pyridazine-6-carboxylic acid (202 mg). Exact Mass Found (M+H)⁺: 243.06285.

### REFERENCE EXAMPLE 168: 3-(Thiazol-2-yl)-[1,2,4]triazolo[4,3-b]pyridazine-6-carboxamide

2-Thiazolylcarbohydrazide (286.3mg, 2.0 mmol) and methyl 6-chloro-pyridazine-3-carboxylate (345 mg, 2.0 mmol) were dissolved in n-BuOH (40 mL) and the mixture was heated to 140-145°C for 8 h in an oil bath. The reaction was concentrated *in vacuo* and the residue was dissolved in ethyl acetate (50 mL), washed with water and brine, dried over MgSO₄, filtered, and evaporated. The crude product was chromatographed (97:3 CHCl₃/MeOH) to provide the intermediate methyl ester (360mg, 69% yield).

Methyl-3-(thiazol-2-yl)-[1,2,4]triazolo[4,3-b]pyridazine-6-carboxylate (52 mg, 0.2 mmol) was dissolved in methanolic ammonia (7N, 10 mL) in a pressure tube and the solution was heated to reflux overnight. After cooling to RT, the solvent was removed under vacuo and the residue was chromatographed (97:3 CHCl₃/MeOH) to afford 3-(thiazol-2-yl)-[1,2,4]triazolo[4,3-b]pyri-dazine-6-carboxamide (30 mg, 61%). Exact Mass Found (M+H)⁺: 247.04007.

### REFERENCE EXAMPLE 169: 3-(Thiazol-2-yl)-[1,2,4]triazolo[4,3-b]pyridazine-6-carboxylic acid

Methyl-3-(thiazol-2-yl)-[1,2,4]triazolo[4,3-b]pyridazine-6-carboxylate (350 mg, 1.24 mmol) was dissolved in a 2:1 THF/methanol (9 mL) mixture and treated with 2N NaOH (1.0 mL). The mixture was stirred for 4 h at 20 °C, evaporated *in vacuo,* diluted with water (20 mL) and acidified with 6N HCl to pH∼2. The solid precipitates were collected, washed with water and dried to afford the product (280 mg, 85%). Exact Mass Found (M+H)⁺: 248.02383.

### REFERENCE EXAMPLE 170: N,N-Diethyl-3-(pyrimidin-2-yl)-[1,2,4]triazolo[4,3-b]pyridazine-6-carboxamide

To a solution of 3-(pyrimidin-2-yl)-[1,2,4]triazolo[4,3-b]pyridazine-6-carboxylic acid (50 mg, 0.21 mmol) in dry DMF (5 mL) HATU (120 mg, 0.32 mmol) was added and the mixture was stirred at RT for 30 min. Then diethylamine (30 mg, 0.42 mmol) was added followed by DIPEA (0.1 mL). Stirring was continued for 4h and water (3 mL) was added. Dichloromethane (2x15 mL) was added and separated the organic layer. The organic extract was dried (MgSO₄), filtered and evaporated under vacuo. The residue was purified by flash chromatography (1-5% MeOH/CHCl₃) to afford the product (55%). Exact Mass Found (M+H)⁺: 298.14163

### REFERENCE EXAMPLE 171: Azetidin-1-yl(3-(pyrimidin-2-yl)-[1,2,4]triazolo[4,3-b]pyridazin-6-yl) methanone

The title compound was prepared analogously to EXAMPLE 170 from 3-(pyrimidin-2-yl)-[1,2,4]triazolo[4,3-b]pyridazine-6-carboxylic acid and azetidine. Exact Mass Found (M+H)⁺: 281.1023

### REFERENCE EXAMPLE 172: N,N-Diethyl-2-((3-(thiophen-2-yl)-[1,2,4]triazolo[4,3-b]pyridazin-6-yl)thio)butanamide

The title compound was prepared by the procedure described in EXAMPLE 170 from 2-((3-(thiophen-2-yl)-[1,2,4]triazolo[4,3-b]pyridazin-6-yl)thio)butanoic acid and diethylamine (26 mg). Exact Mass Found (M+H)⁺: 376.12663.

### REFERENCE EXAMPLE 173: N-Methyl-N-phenyl-3-(pyrimidin-2-yl)-[1,2,4]triazolo[4,3-b]pyri-dazine-6-carboxamide

The title compound was prepared analogously to compound EXAMPLE 170 from 3-(pyrimidin-2-yl)-[1,2,4]triazolo[4,3-b]pyridazine-6-carboxylic acid and N-methyl-N-phenyl amine (24 mg). Exact Mass Found (M+H)⁺: 332.12557.

### REFERENCE EXAMPLE 174: Piperidin-1-yl(3-(pyrimidin-2-yl)-[1,2,4]triazolo[4,3-b]pyridazin-6-yl) methanone

The title compound was prepared analogously to compound EXAMPLE 170 from 3-(pyrimidin-2-yl)-[1,2,4]triazolo[4,3-b]pyridazine-6-carboxylic acid and piperidine (28 mg). Exact Mass Found (M+H)⁺: 310.14133.

### REFERENCE EXAMPLE 175: N,N-Dipropyl-3-(thiazol-2-yl)-[1,2,4]triazolo[4,3-b]pyridazine-6-carboxamide

To a solution of 3-(thiazol-2-yl)-[1,2,4]triazolo[4,3-b]pyridazine-6-carboxylic acid (50 mg, 0.20 mmol) in dry DMF (5mL) HATU (120 mg, 0.32 mmol) was added and the mixture was stirred at RT for 30 min and Dipropylamine (40 mg, 0.40 mmol) was added followed by DIPEA (0.1 mL). Stirring was continued for 4h and water was added. Dichloromethane (2 x 15 mL) was added and separated the organic layer. The organic extract was dried (MgSO₄), filtered and evaporated under vacuo. The residue was purified by flash chromatography (1-5% MeOH/CHCl₃) to afford the product (29 mg). Exact Mass Found (M+H)⁺: 324.15716

### REFERENCE EXAMPLE 176: Morpholino(3-(pyrimidin-2-yl)-[1,2,4]triazolo[4,3-b]pyridazin-6-yl) methanone

The title compound was prepared analogously to EXAMPLE 170 from 3-(pyrimidin-2-yl)-[1,2,4]triazolo[4,3-b]pyridazine-6-carboxylic acid and morpholine (26 mg).
Exact Mass Found (M+H)⁺: 312.12120.

### REFERENCE EXAMPLE 177: (3-Methylpiperidin-1-yl)(3-(pyrimidin-2-yl)-[1,2,4]triazolo[4,3-b]pyri-dazin-6-yl)methanone

The title compound was prepared analogously to EXAMPLE 170 from 3-(pyrimidin-2-yl)-[1,2,4]triazolo[4,3-b]pyridazine-6-carboxylic acid and 3-methyl piperidine (22 mg). Exact Mass Found (M+H)⁺: 324.15716

### REFERENCE EXAMPLE 178: N-(2-(Benzyloxy)pyridin-3-yl)-3-(pyrimidin-2-yl)-[1,2,4]triazolo[4,3-b]pyridazine-6-carboxamide

The title compound was prepared analogously to EXAMPLE 170 from 3-(pyrimidin-2-yl)-[1,2,4]triazolo[4,3-b]pyridazine-6-carboxylic acid and 2-(benzyloxy)pyridin-3-yl amine (28 mg). Exact Mass Found (M+H)⁺: 425.14621.

### REFERENCE EXAMPLE 179: N-(4-Morpholinophenyl)-3-(pyrimidin-2-yl)-[1,2,4]triazolo[4,3-b]pyridazine-6-carboxamide

The title compound was prepared analogously to EXAMPLE 170 from 3-(pyrimidin-2-yl)-[1,2,4]triazolo[4,3-b]pyridazine-6-carboxylic acid and 4-morpholino aniline (32 mg). Exact Mass Found (M+H)⁺: 403.16169.

### REFERENCE EXAMPLE 180: N-(Benzo[d][1,3]dioxol-5-ylmethyl)-3-(pyrimidin-2-yl)-[1,2,4]triazolo [4,3-b]pyridazine-6-carboxamide

The title compound was prepared analogously to EXAMPLE 170 from 3-(pyrimidin-2-yl)-[1,2,4]triazolo[4,3-b]pyridazine-6-carboxylic acid and benzo[d][1,3]dioxol-5-ylmethyl amine (29 mg). Exact Mass Found (M+H)⁺: 376.11513

### REFERENCE EXAMPLE 181: (R)-Ethyl 2-(3-(pyrimidin-2-yl)-[1,2,4]triazolo[4,3-b]pyridazine-6-carboxamido)propanoate

The title compound was prepared analogously to EXAMPLE 170 from 3-(pyrimidin-2-yl)-[1,2,4]triazolo[4,3-b]pyridazine-6-carboxylic acid and (R)-ethyl 2-amino propanoate (32 mg). Exact Mass Found (M+H)⁺: 342.13118

### REFERENCE EXAMPLE 182: N-Benzyl-3-(pyrimidin-2-yl)-[1,2,4]triazolo[4,3-b]pyridazine-6-carboxamide

The title compound was prepared by the procedure described in EXAMPLE 170 by substituting diethylamine with benzylamine (29 mg). Exact Mass Found (M+H)⁺: 332.12465

### REFERENCE EXAMPLE 183: (S)-N-(1-Amino-1-oxo-3-phenylpropan-2-yl)-3-(pyrimidin-2-yl)-[1,2,4]triazolo[4,3-b]pyridazine-6-carboxamide

The title compound was prepared analogously to EXAMPLE 170 from 3-(pyrimidin-2-yl)-[1,2,4]triazolo[4,3-b]pyridazine-6-carboxylic acid and (S)-(1-amino-1-oxo-3-phenylpropan-2-yl amine (24 mg). Exact Mass Found (M+H)⁺: 389.14657

### REFERENCE EXAMPLE 184: N-(2-Morpholinoethyl)-3-(pyrimidin-2-yl)-[1,2,4]triazolo[4,3-b]pyridazine-6-carboxamide

The title compound was prepared analogously to EXAMPLE 170 from 3-(pyrimidin-2-yl)-[1,2,4]triazolo[4,3-b]pyridazine-6-carboxylic acid and 2-morpholinoethyl amine (30 mg). Exact Mass Found (M+H)⁺: 355.6255

### REFERENCE EXAMPLE 185: (R)-Methyl 3-phenyl-2-(3-(pyrimidin-2-yl)-[1,2,4]triazolo[4,3-b]pyridazine-6-carboxamido)propanoate

The title compound was prepared analogously to EXAMPLE 170 from 3-(pyrimidin-2-yl)-[1,2,4]triazolo[4,3-b]pyridazine-6-carboxylic acid and (R)-methyl 3-phenyl-2-amino propanoate (32 mg). Exact Mass Found (M+H)⁺: 404.14598

### REFERENCE EXAMPLE 186: N-(Pyridin-2-ylmethyl)-3-(pyrimidin-2-yl)-[1,2,4]triazolo[4,3-b]pyri-dazine-6-carboxamide

The title compound was prepared analogously to EXAMPLE 170 from 3-(pyrimidin-2-yl)-[1,2,4]triazolo[4,3-b]pyridazine-6-carboxylic acid and pyridin-2-ylmethyl amine (29 mg). Exact Mass Found (M+H)⁺: 333.12074

### REFERENCE EXAMPLE 187: (R)-N-(1-Amino-1-oxo-3-phenylpropan-2-yl)-3-(pyrimidin-2-yl)-[1,2,4]triazolo[4,3-b]pyridazine-6-carboxamide

The title compound was prepared analogously to EXAMPLE 170 from 3-(pyrimidin-2-yl)-[1,2,4]triazolo[4,3-b]pyridazine-6-carboxylic acid and (R)-(1-amino-1-oxo-3-phenylpropan-2-ylamine (27 mg). Exact Mass Found (M+H)⁺: 389.14651

### REFERENCE EXAMPLE 188: (R)-4-Methyl-2-(3-(pyrimidin-2-yl)-[1,2,4]triazolo[4,3-b]pyridazine-6-carboxamido)pentanoic acid

The title compound was prepared analogously to EXAMPLE 170 from 3-(pyrimidin-2-yl)-[1,2,4]triazolo[4,3-b]pyridazine-6-carboxylic acid and (R)-4-methyl-2-amino pentanoic acid (26 mg). Exact Mass Found (M+H)⁺: 356.14621

### REFERENCE EXAMPLE 189: (R)-N-(1-Amino-4-methyl-1-oxopentan-2-yl)-3-(pyrimidin-2-yl)-[1,2,4]triazolo[4,3-b]pyridazine-6-carboxamide

The title compound was prepared analogously to EXAMPLE 170 from 3-(pyrimidin-2-yl)-[1,2,4]triazolo[4,3-b]pyridazine-6-carboxylic acid and (R)-(1-amino-4-methyl-1-oxopentan-2-yl amine (28 mg). Exact Mass Found (M+H)⁺: 355.16230

### REFERENCE EXAMPLE 190: N-(Cyclopropylmethyl)-3-(pyrimidin-2-yl)-[1,2,4]triazolo[4,3-b]pyri-dazine-6-carboxamide

The title compound was prepared analogously to EXAMPLE 170 from 3-(pyrimidin-2-yl)-[1,2,4]triazolo[4,3-b]pyridazine-6-carboxylic acid and cyclopropylmethyl amine (19 mg). Exact Mass Found (M+H)⁺: 296.12582

### REFERENCE EXAMPLE 191: 1-(Piperidin-1-yl)-2-((3-(thiophen-2-yl)-[1,2,4]triazolo[4,3-b]pyrida-zin-6-yl)sulfonyl)butan-1-one

To a solution of 1-(piperidin-1-yl)-2-((3-(thiophen-2-yl)-[1,2,4]triazolo[4,3-b]pyridazin-6-yl)thio)butan-1-one (50 mg, 0.13mmol) in dry DMF (5 mL), 3-chloroperoxy benzoic acid (100 mg) was added and the mixture was heated at 85-90 °C for 3 h and stirred overnight at RT. The reaction was diluted with ethyl acetate (20 mL) and washed with water, sat. NaHCO₃, and brine. The organic layer was dried (Na₂SO₄), filtered and evaporated under vacuo. The crude product was purified by crystallisation (EtOAc) to afford the product (30 mg). Exact Mass Found (M+H)⁺: 420.11540

### REFERENCE EXAMPLE 192: N-(1-Benzylpiperidin-4-yl)-3-(thiazol-2-yl)-[1,2,4]triazolo[4,3-b]pyridazine-6-carboxamide

The title compound was prepared by the procedure described in EXAMPLE 170 by substituting dipropylamine with N-(1-benzylpiperidin-4-yl)amine (32 mg). Exact Mass Found (M+H)⁺: 420.15945

### REFERENCE EXAMPLE 193: 2-(N-Methyl(3-(thiophen-2-yl)-[1,2,4]triazolo[4,3-b]pyridazin-6-yl) amino)-1-(piperidin-1-yl)ethanone

To a solution of 6-chloro-3-(thiophen-2-yl)-[1,2,4]triazolo[4,3-b]pyridazine (125 mg, 0.53 mmol) and 2-(methylamino)-1-(piperidin-1-yl)ethanone hydrochloride (145 mg, 0.75mmol) in dry DMF (3.0 mL), triethylamine (0.5 mL) was added and the resulting mixture was heated to 130-135 °C overnight. The solvent was removed under vacuum. The residue was dissolved in ethylacetate, washed with water, 1N HCl, water and brine. The organic layer was dried (Na₂SO₄), filtered and evaporated under vacuo. The crude product was purified by crystallization from EtOAc (46 mg). Exact Mass Found (M+H)⁺: 357.14949

### REFERENCE EXAMPLE 194: 1-(Piperidin-1-yl)-2-(3-(thiazol-2-yl)-[1,2,4]triazolo[4,3-b]pyridazin-6-yl)ethanone

A solution of ethyl 2-(6-chloropyridazin-3-yl)acetate (0.802 g, 4.0 mmol) and thiazole-2-carbohydrazide (0.69 g, 4.8 mmol) in n-BuOH (80 mL) was refluxed for 24 h in a pressure tube. The solvent was removed under vacuum. Purification by column chromatography using

CHCl₃/MeOH (1-5 % MeOH) as eluent afforded ethyl 2-(3-(thiazol-2-yl)-[1,2,4]triazolo[4,3-b]pyridazin-6-yl)acetate (0.69 g, 59.5%).

The ester (0.64 g, 2.2 mmol) was dissolved in a 2:1 THF/methanol (30 mL) mixture and treated with 2N NaOH (2.5 mL). The mixture was stirred for 4 h at 20 °C, evaporated *in vacuo,* diluted with water (20 mL) and acidified with 6N HCl to pH∼2. The solution was extracted with ethylacetate (2x30 mL), washed with water, brine and dried (MgSO₄). The solvent was removed under vacuum and the residue was purified to afford 2-(3-(thiazol-2-yl)-[1,2,4]triazolo[4,3-b]pyridazin-6-yl)acetic acid (0.36g, 62%).

### REFERENCE EXAMPLE 195: 1-Morpholino-2-(3-(thiazol-2-yl)-[1,2,4]triazolo[4,3-b]pyridazin-6-yl) ethanone

The title compound was prepared by the procedure described in EXAMPLE 194 from 2-(3-(thiazol-2-yl)-[1,2,4]triazolo[4,3-b]pyridazin-6-yl)acetic acid and morpholine (26 mg). Exact Mass Found (M+H)⁺: 331.09771

### REFERENCE EXAMPLE 196: 2-(N-Methyl(3-(thiophen-2-yl)-[1,2,4]triazolo[4,3-b]pyridazin-6-yl) amino)-1-(pyrrolidin-1-yl)ethanone

To a solution of 6-chloro-3-(thiophen-2-yl)-[1,2,4]triazolo[4,3-b]pyridazine (125 mg, 0.53 mmol) and 2-(methylamino)-1-(pyrrolidin-1-yl)ethanone hydrochloride (145 mg, 0.81 mmol) in dry DMF (3.0 mL), triethylamine (0.5 mL) was added and the resulting mixture was heated to 130-135 °C overnight. The solvent was removed under vacuum. The residue was dissolved in ethylacetate, washed with water, 1N HCl, water and brine. The organic layer was dried (Na₂SO₄), filtered and evaporated under vacuo. The crude product was purified by crystallization from EtOAc (40 mg). Exact Mass Found (M+H)⁺: 343.13431

### REFERENCE EXAMPLE 197: N,N-Diethyl-2-(3-(thiazol-2-yl)-[1,2,4]triazolo[4,3-b]pyridazin-6-yl) acetamide

The title compound was prepared by the procedure described in EXAMPLE 194 from 2-(3-(thiazol-2-yl)-[1,2,4]triazolo[4,3-b]pyridazin-6-yl)acetic acid and diethylamine (22 mg). Exact Mass Found (M+H)⁺: 317.11817

### REFERENCE EXAMPLE 198: N,N-Dimethyl-2-(3-(thiazol-2-yl)-[1,2,4]triazolo[4,3-b]pyridazin-6-yl) acetamide

The title compound was prepared by the procedure described in EXAMPLE 194 from 2-(3-(thiazol-2-yl)-[1,2,4]triazolo[4,3-b]pyridazin-6-yl)acetic acid and dimethylamine (22 mg). Exact Mass Found (M+H)⁺: 289.08682

### REFERENCE EXAMPLE 199: N-(Pyridin-4-yl)-2-(3-(thiazol-2-yl)-[1,2,4]triazolo[4,3-b]pyridazin-6-yl)acetamide.

The title compound was prepared by the procedure described in EXAMPLE 194 from 2-(3-(thiazol-2-yl)-[1,2,4]triazolo[4,3-b]pyridazin-6-yl)acetic acid and (pyridin-4-yl)-amine (19 mg). Exact Mass Found (M+H)⁺: 338.08213

### REFERENCE EXAMPLE 200: 1-(Pyrrolidin-1-yl)-2-(3-(thiazol-2-yl)-[1,2,4]triazolo[4,3-b]pyridazin-6-yl)ethanone

The title compound was prepared by the procedure described in EXAMPLE 194 from 2-(3-(thiazol-2-yl)-[1,2,4]triazolo[4,3-b]pyridazin-6-yl)acetic acid and pyrrolidine (27 mg). Exact Mass Found (M+H)⁺: 315.10191

### REFERENCE EXAMPLE 201: 2-(Methyl(3-(thiophen-2-yl)-[1,2,4]triazolo[4,3-b]pyridazin-6-yl) amino)-1-morpholinoethanone

To a solution of 6-chloro-3-(thiophen-2-yl)-[1,2,4]triazolo[4,3-b]pyridazine (130 mg, 0.55 mmol) and 2-(methylamino)-1-morpholinoethanone hydrochloride (139 mg, 0.72 mmol) in dry DMF (3.0 mL), triethylamine (0.5 mL) was added and the resulting mixture was heated to 130-135 °C overnight. The solvent was removed under vacuum. The residue was dissolved in ethylacetate, washed with water, 1N HCl, water and brine. The organic layer was dried (Na₂SO₄), filtered and evaporated under vacuo. The crude product was chromatographed to afford the product (63 mg). Exact Mass Found (M+H)⁺: 359.12845

### REFERENCE EXAMPLE 202: N-((3s,5s,7s)-Adamantan-1-yl)-2-((3-(thiophen-2-yl)-[1,2,4]triazolo [4,3-b]pyridazin-6-yl)thio)propanamide

To a solution of 2-((3-(thiophen-2-yl)-[1,2,4]triazolo[4,3-b]pyridazin-6-yl)thio)propanoic acid (101 mg, 0.33 mmol) in dry DMF (5mL) were added HATU (163 mg, 0.43 mmol) and DIPEA (0.2 mL). The mixture was stirred at RT for 30 min and (3s,5s,7s)-adamantan-1-amine (55 mg, 0.36 mmol) was added. Stirring was continued for 4h and water was added. Dichloromethane (2x15 mL) was added and the organic layer was separated. The organic extract was dried (MgSO₄), filtered and evaporated under vacuo. The residue was purified by flash chromatography (1-5% MeOH/CHCl₃ to afford the product (42 mg). Exact Mass Found (M+H)⁺: 440.15736

### REFERENCE EXAMPLE 203: N,N-Dipropyl-2-(3-(thiazol-2-yl)-[1,2,4]triazolo[4,3-b]pyridazin-6-yl) acetamide

The title compound was prepared by the procedure described in EXAMPLE 194 from 2-(3-(thiazol-2-yl)-[1,2,4]triazolo[4,3-b]pyridazin-6-yl)acetic acid and dipropylamine (27 mg). Exact Mass Found (M+H)⁺: 345.14916

### REFERENCE EXAMPLE 204: 2-(3-(1-Methyl-1H-pyrazol-3-yl)-[1,2,4]triazolo[4,3-b]pyridazin-6-yl) acetic acid

A solution of ethyl 2-(6-chloropyridazin-3-yl)acetate (1.2 g 5.98 mmol) and (1-methyl-1H-pyrazole-3-carbohydrazide (1.2 g, 8.37 mmol) in n-BuOH (80 mL) was refluxed for 40 h in a pressure tube. The solvent was removed under vaccum and the crude product was purified by column chromatography using CHCl₃/MeOH (1-5 % MeOH) as eluent afforded a mixture of butyl and ethyl ester (2:1, 0.9 g, 48 %).

The ester (ethyl and butyl 2-(3-(1-methyl-1H-pyrazol-3-yl)-[1,2,4]triazolo[4,3-b]pyridazin-6-yl)acetate (0.7g, 2.2 mmol) was dissolved in a 2:1 THF/methanol (30 mL) mixture and treated with 2N NaOH (2.5 mL). The mixture was stirred for 4 h at 20 °C, evaporated *in vacuo,* diluted with water (20 mL) and acidified with 6N HCl to pH∼2. The solution was extracted with ethylacetate (2x30 mL), washed with water, brine and dried (MgSO₄). The solvent was removed under vacuum and the residue was purified to afford the product (343 mg, 60%). Exact Mass Found (M+H)⁺: 259.09421

### REFERENCE EXAMPLE 205: 1-(Azetidin-1-yl)-2-(3-(thiazol-2-yl)-[1,2,4]triazolo[4,3-b]pyridazin-6-yl)ethanone

The title compound was prepared by the procedure described in EXAMPLE 194 from 2-(3-(thiazol-2-yl)-[1,2,4]triazolo[4,3-b]pyridazin-6-yl)acetic acid and azetidine (23 mg). Exact Mass Found (M+H)⁺: 301.087

### REFERENCE EXAMPLE 206: 2-(3-(1-Methyl-1H-pyrazol-3-yl)-[1,2,4]triazolo[4,3-b]pyridazin-6-yl) acetamide

Butyl 2-(3-(1-methyl-1H-pyrazol-3-yl)-[1,2,4]triazolo[4,3-b]pyridazin-6-yl)acetate (60 mg, 0.19 mmol) was dissolved in methanolic ammonia (7N, 10 mL) in a pressure tube and the solution was heated to reflux overnight. After cooling to RT, the solvent was removed under vacuo and the residue was chromatographed (97:3 CHCl₃/MeOH) to afford the product (29 mg, 59%). Exact Mass Found (M+H)⁺: 258.1097

### REFERENCE EXAMPLE 207: 2-(3-(1-Methyl-1H-pyrazol-3-yl)-[1,2,4]triazolo[4,3-b]pyridazin-6-yl)-1-(piperidin-1-yl)ethanone

The title compound was prepared by the procedure described in EXAMPLE 194 from 2-(3-(1-methyl-1H-pyrazol-3-yl)-[1,2,4]triazolo[4,3-b]pyridazin-6-yl)acetic acid and piperidine (29 mg). Exact Mass Found (M+H)⁺: 326.17252

### REFERENCE EXAMPLE 208: 2-(3-(1-Methyl-1H-pyrazol-3-yl)-[1,2,4]triazolo[4,3-b]pyridazin-6-yl)-1-morpholinoethanone

The title compound was prepared by the procedure described in EXAMPLE 194 from 2-(3-(1-methyl-1H-pyrazol-3-yl)-[1,2,4]triazolo[4,3-b]pyridazin-6-yl)acetic acid and morpholine (22 mg). Exact Mass Found (M+H)⁺: 328.15186

### REFERENCE EXAMPLE 209: (3-(Thiophen-2-yl)-[1,2,4]triazolo[4,3-b]pyridazin-6-yl)methanamine

To a solution of 6-chloropyridazine-3-carbonitrile (0.3 g, 2.14 mmol) and thiophene-2-carbohydrazide (0.37 g, 2.58 mmol) in xylene (30 mL), triethylamine hydrochloride (0.7 g) was added and the mixture was stirred under reflux for 8 h, when TLC showed consumption of the starting material. The reaction mixture was cooled to room temperature, concentrated *in vacuo* and the residue was diluted with water (20 ml), extracted with EtOAc (3x30 mL). The combined organic layer was washed with brine solution, dried over anhydrous Na₂SO₄. The solvent was evaporated under reduced pressure and the residue was purified to give 3-(thiophen-2-yl)-[1,2,4]triazolo[4,3-b]pyridazine-6-carbonitrile (0.19 g, 39%).

### Alternative synthesis:

To a solution of 3-(thiophen-2-yl)-[1,2,4]triazolo[4,3-b]pyridazine-6-carbonitrile (180 mg, 0.79 mmol) in EtOH:CHCl₃ (50 :1 mL), 10% Pd/C (100 mg) was added and hydrogenated at 50 psi for 22 h. The solution was filtered and evaporated off the solvent under reduced pressure. The residue was purified (5% MeOH/CHCl₃) to give the product (110 mg, 60 %). Exact Mass Found (M+H)⁺: 232.06557

### REFERENCE EXAMPLE 210: Morpholino(4-(3-(thiophen-2-yl)-[1,2,4]triazolo[4,3-b]pyridazin-6-yl) phenyl)methanone

The title compound was prepared by the procedure described in EXAMPLE 194 from (3-(thiophen-2-yl)-[1,2,4]triazolo[4,3-b]pyridazin-6-yl)benzoic acid and morpholine (22 mg). Exact Mass Found (M+H)⁺: 392.11685

### REFERENCE EXAMPLE 211: 2-(Methyl(3-(thiophen-2-yl)-[1,2,4]triazolo[4,3-b]pyridazin-6-yl)amino)-1-(4-methylpiperazin-1-yl)ethanone

To a solution of 6-chloro-3-(thiophen-2-yl)-[1,2,4]triazolo[4,3-b]pyridazine (130 mg, 0.55 mmol) and 2-(methylamino)-1-(4-methylpiperazin-1-yl)ethanone hydrochloride (145 mg, 0.84 mmol) in dry DMF (3.0 mL), triethylamine (0.5 mL) was added and the resulting mixture was heated to 130-135 °C overnight. The solvent was removed under vacuum. The residue was dissolved in ethylacetate, washed with water, 1N HCl, water and brine. The organic layer was dried (Na₂SO₄), filtered and evaporated under vacuo. The crude product was chromatographed to afford the product (49 mg). Exact Mass Found (M+H)⁺: 372.16135

### EXAMPLE 212: 6-(Methylthio)-3-(thiophen-2-yl)-[1,2,4]triazolo[4,3-b]pyridazine

### a) Preparation of 6-chloro-3-(thiophen-2-yl)-[1,2,4]triazolo[4,3-b]pyridazine:

To a solution of 3,6-dichloropyridazine (1.5 g, 10.06 mmol) and thiophene-2-carbohydrazide (1.57 g, 11.07 mmol) in xylene (25 mL) at room temperature was added triethylamine hydrochloride (1.52 g, 11.07 mmol) and stirred under reflux for 6 h, when TLC showed consumption of the starting material. The reaction mixture was cooled to room temperature, concentrated in vacuo and the residue was diluted with water (20 ml), extracted with EtOAc (3x30 mL). The combined organic layer was washed with brine solution, dried over anhydrous Na₂SO₄. The solvent was evaporated under reduced pressure and crystallized in diethyl ether to afford the product as yellow color solid (420 mg).

### b) Preparation of 6-(methylthio)-3-(thiophen-2-yl)-[1,2,4]triazolo[4,3-b]pyridazine:

To a solution of 6-chloro-3-(thiophen-2-yl)-[1,2,4]triazolo[4,3-b]pyridazine (100 mg, 0.42 mmol) in DMF (3 mL), NaSMe (30 mg, 0.42 mmol) was added under argon and the resulting solution was stirred for 1h at 50 °C and at RT overnight. Most of the solvent was removed under vacuum and water (10 mL) was added when solid precipitated. The mixture was stirred at RT for 2 h and the solid product was filtered, washed with water (x3) and dried under high vacuum to yield the product (60 mg). Exact Mass Found (M+H)⁺: 249.02626
BIOLOGICAL TESTING

Compounds were assessed for inhibition of LRRK2 inhibition using the following procedure. Recombinant GST-LRRK2 (delta970), purified from baculovirus infected insect cells (SF9), at least 90% pure and either wild-type sequence or bearing the pathogenic G2019S mutation, was combined into a kinase buffer at 30 nM concentration. Kinase buffer consists of 50 mM Tris HCl pH 7.4, 1 mM EDTA, and 50 µM peptide substrate (amino acid sequence RLGAWRFYTLRRARQGNTKQR). Experimental compounds or control (no compound) were added at desired concentrations in DMSO and water, where final concentration of DMSO in kinase reactions was 0.1% for all drug concentrations. Activation buffer that consists of a final concentration in the reactions of 10 mM MgCl₂ and ATP (∼92 µM for WT-LRRK2 reactions and ∼52 µM for G2019S-LRRK2, which represent respective Km ATP concentrations) were added to each reaction, in addition to 0.5 µCi of gamma-32-P-dATP per reaction. Reactions were incubated at 1400 RPM at 30 °C for 30 min. and then spotted in triplicate directly onto a slot-blot apparatus fitted with phospho-cellulose paper and allowed to dry. Each slot was then washed with 10 mM phosphoric acid buffer until beta-radiation could not be detected in eluted wash buffer, usually ∼1 mL per slot. Paper within each slot were excised and radiation measured by liquid scintillation. Raw CPM were input into GraphPad, and IC-50 values calculated using non-linear regression analysis, where 100% activity is defined by reactions with control (no) inhibitor compounds, and baseline activity is defined by control reactions that did not include LRRK2 enzyme.

The Table below contains the data for the compounds 34, 36, 90, 101, 105 and 134 which are examples according to the invention and the remaining examples are reference examples.

| Example # | LRRK2 (G2019S) IC₅₀ (µM) | Example # | LRRK2 (G2019S) IC₅₀ (µM) |
|---|---|---|---|
| 1 | + | 115 | +++ |
| 2 | + | 133 | ++ |
| 3 | +++ | 134 | ++++ |
| 4 | + | 135 | ++++ |
| 5 | +++ | 136 | +++ |
| 6 | +++ | 137 | + |
| 7 | + | 138 | +++ |
| 8 | +++ | 139 | ++ |
| 9 | ++ | 26 | +++ |
| 10 | + | 27 | + |
| 11 | +++ | 28 | + |
| 12 | + | 29 | + |
| 13 | ++ | 30 | + |
| 14 | + | 31 | + |
| 15 | ++ | 32 | + |
| 16 | + | 33 | +++ |
| 17 | + | 34 | ++++ |
| 18 | +++ | 35 | ++ |
| 19 | ++ | 36 | +++ |
| 20 | + | 37 | + |
| 21 | +++ | 38 | + |
| 22 | + | 39 | + |
| 23 | +++ | 40 | + |
| 24 | +++ | 41 | ++ |
| 25 | ++ | 42 | + |
| 51 | +++ | 43 | + |
| 52 | +++ | 44 | ++ |
| 53 | + | 45 | +++ |
| 54 | +++ | 46 | ++++ |
| 55 | +++ | 47 | +++ |
| 56 | +++ | 48 | +++ |
| 57 | ++ | 49 | +++ |
| 58 | + | 50 | + |
| 59 | + | 77 | + |
| 60 | +++ | 78 | + |
| 61 | + | 79 | ++ |
| 62 | +++ | 80 | +++ |
| 63 | +++ | 81 | +++ |
| 64 | +++ | 82 | + |
| 65 | + | 83 | + |
| 66 | +++ | 84 | + |
| 67 | +++ | 85 | + |
| 68 | + | 86 | + |
| 69 | + | 87 | +++ |
| 70 | + | 88 | ++ |
| 71 | ++ | 89 | +++ |
| 72 | +++ | 90 | ++++ |
| 73 | +++ | 91 | ++ |
| 74 | ++ | 92 | +++ |
| 75 | ++ | 93 | +++ |
| 76 | ++ | 94 | +++ |
| 103 | ++ | 95 | ++ |
| 104 | +++ | 96 | +++ |
| 105 | ++++ | 97 | +++ |
| 106 | +++ | 98 | + |
| 107 | ++ | 99 | + |
| 108 | +++ | 100 | + |
| 109 | +++ | 101 | ++++ |
| 110 | +++ | 123 | ++ |
| 111 | +++ | 124 | +++ |
| 112 | + | 125 | +++ |
| 113 | +++ | 126 | +++ |
| 114 | +++ | | |

| | | | |
|---|---|---|---|
| + IC₅₀ ≥5µM, ++ IC₅₀ ≥ 1.0 to < 5µM, +++ IC₅₀ ≥ 0.1 to < 1.0µM, ++++ IC₅₀ < 0.1µM | | | |

To test whether Example 90 and Example 34 can inhibit LRRK2 phosphorylation, an antibody commercially available from Epitomics directed against the serine residue number 935 on LRRK2 from lysates generated from HEK-293T cells transiently transfected with either WT-LRRK2 or G2019S-LRRK2 protein. Both Example 90 and Example 34 partially inhibited levels of pS935, which demonstrates that the compounds successfully enter the cells and engage and inhibit LRRK2.

Recently, high levels of LRRK2 expression in primary microglia that are the immune cells of the nervous system have been discovered (Moehle et al., J. Neuroscience (2012), PMID 22302802). This publication shows that knockdown of LRRK2 expression using RNA interference attenuates the induction of the pro-inflammatory and neurotoxic factor TNF-alpha. More recently, LRRK2 has also been detected in primary macrophages, specifically macrophages isolated after thioglycolate exposures in mice. To assess whether Example 90 can inhibit the G2019S-LRRK2 induced increase in TNF-alpha to wild-type levels of TNF-alpha induction by LPS, primary macrophages were exposed in culture to 5 µM 1 hour prior to LPS stimulation (100 ng/ml), and TNF-alpha levels were measured 6 hours later. Example 90 successfully returned G2019S-LRRK2 over-expressing cells back to WT levels, indicating that G2019S-LRRK2 was inhibited in these cells.

In keeping with the present disclosure, the triazolopyridazine compounds of the present disclosure can be used alone or in appropriate association, and also may be used in combination with pharmaceutically acceptable carriers and other pharmaceutically active compounds. The active agent may be present in the pharmaceutical composition in any suitable quantity.

The pharmaceutically acceptable carriers described herein, for example, vehicles, adjuvants, excipients, or diluents, are well-known to those who are skilled in the art. Typically, the pharmaceutically acceptable carrier is chemically inert to the active compounds and has no detrimental side effects or toxicity under the conditions of use. The pharmaceutically acceptable carriers can include polymers and polymer matrices.

The choice of carrier will be determined in part by the particular method used to administer the composition. Accordingly, there is a wide variety of suitable formulations of the pharmaceutical composition of the present invention. The following formulations for oral, aerosol, parenteral, subcutaneous, intravenous, intraarterial, intramuscular, intraperitoneal, intrathecal, rectal, and vaginal administration are merely exemplary and are in no way limiting.

Formulations suitable for oral administration can consist of (a) liquid solutions, such as an effective amount of the compound dissolved in diluents, such as water, saline, or orange juice; (b) capsules, sachets, tablets, lozenges, and troches, each containing a predetermined amount of the active ingredient, as solids or granule; (c) powders; (d) suspensions in an appropriate liquid; and (e) suitable emulsions. Liquid formulations may include diluents, such as water, cyclodextrin, dimethyl sulfoxide and alcohols, for example, ethanol, benzyl alcohol, propylene glycol, glycerin, and the polyethylene alcohols including polyethylene glycol, either with or without the addition of a pharmaceutically acceptable surfactant, suspending agent, or emulsifying agent. Capsule forms can be of the ordinary hard-or soft-shelled gelatin type containing, for example, surfactants, lubricants, and inert fillers, such as lactose, sucrose, calcium phosphate, and corn starch. Tablet forms can include one or more of the following: lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible carriers. Lozenge forms can comprise the active ingredient in a flavor, usually sucrose and acacia or tragacanth, as well as pastilles comprising the active ingredient in an inert base, such as gelatin and glycerin, or sucrose and acadia, emulsions, and gels containing, the addition to the active ingredient in an inert base, such as gelatin and glycerin, or sucrose and acadia, emulsions, and gels containing, in addition to the active ingredient, such carriers as are known in the art.

The triazolopyridazine compounds of the present disclosure alone or in combination with other suitable components, can be made into aerosol formulations to be administered via inhalation. These aerosol formulations can be placed into pressurized acceptable propellants, such as dichlorodifluoromethane, propane, and nitrogen. They also may be formulated as pharmaceuticals for non-pressured preparations, such as in a nebulizer or an atomizer. Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The compound can be administered in a physiologically acceptable diluent in a pharmaceutical carrier, such as a sterile liquid or mixture of liquids, including water, saline, aqueous dextrose and related sugar solutions, an alcohol, such as ethanol, isopropanol, or hexadecyl alcohol, glycols, such as propylene glycol or polyethylene glycol such as poly(ethyleneglycol) 400, glycerol ketals, such as 2,2-dimethyl-1, 3-dioxolane-4-methanol, ethers, an oil, a fatty acid, a fatty acid ester or glyceride, or an acetylated fatty acid glyceride with or without the addition of a pharmaceutically acceptable surfactant, such as a soap or a detergent, suspending agent, such as pectin, carbomers, methylcellulose, hydroxypropyl-methylcellulose, or carboxymethylcelluslose, or emulsifying agents and other pharmaceutical adjuvants.

Oils which can be used in parenteral formulations include petroleum, animal, vegetable, or synthetic oils. Specific examples of oils include peanut, soybean, sesame, cottonseed, corn, olive, petrolatum, and mineral. Suitable fatty acids for use in parenteral formulations include oleic acid, stearic acid, and isostearic acid. Ethyl oleate and isopropyl myristate are examples of suitable fatty acid esters. Suitable soaps for use in parenteral formulations include fatty alkali metal, ammonium, and triethanolamine salts, and suitable detergents include (a) cationic detergents such as, for example. dimethyldialkylammonium halides, and alkylpyridinium halides, (b) anionic detergents such as, for example, alkyl, aryl, and olefin sulfonates, alky,l olefin, ether, and monoglyceride sulfates, and sulfosuccinates, (c) nonionic detergents such as, for example, fatty amine oxides, fatty acid alkanolamides, and polyoxyethylene polypropylene copolymers, (d) amphoteric detergents such as, for example, alkyl β-aminopropionates, and 2-alkylimidazoline quaternary ammonium salts, and (e) mixtures thereof.

The parenteral formulations typically contain from about 0.5% to about 25% by weight of the active ingredient in solution. Suitable preservatives and buffers can be used in such formulations. In order to minimize or eliminate irritation at the site of injection, such compositions may contain one or more nonionic surfactants having a hydrophile-lipophile balance (HLB) of from about 12 to about 17. The quantity of surfactant in such formulations ranges from about 5% to about 15% by weight. Suitable surfactants include polyethylene sorbitan fatty acid esters, such as sorbitan monooleate and the high molecular weight adducts of ethylene oxide with a hydrophobic base, formed by the condensation of propylene oxide with propylene glycol.

Pharmaceutically acceptable excipients are also well-known to those who are skilled in the art. The choice of excipient will be determined in part by the particular compound, as well as by the particular method used to administer the composition. Accordingly, there is a wide variety of suitable formulations of the pharmaceutical composition of the present disclosure. The following methods and excipients are merely exemplary and are in no way limiting. The pharmaceutically acceptable excipients preferably do not interfere with the action of the active ingredients and do not cause adverse side-effects. Suitable carriers and excipients include solvents such as water, alcohol, and propylene glycol, solid absorbants and diluents, surface active agents, suspending agent, tableting binders, lubricants, flavors, and coloring agents. The formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets. The requirements for effective pharmaceutical carriers for injectable compositions are well known to those of ordinary skill in the art. See Pharmaceutics and Pharmacy Practice, J.B. Lippincott Co., Philadelphia, PA, Banker and Chalmers, Eds., 238-250 (1982) and ASHP Handbook on Injectable Drugs, Toissel, 4th ed., 622-630 (1986). Formulations suitable for topical administration include lozenges comprising the active ingredient in a flavor, usually sucrose and acacia or tragacanth; pastilles comprising the active ingredient in an inert base, such as gelatin and glycerin, or sucrose and acacia; and mouthwashes comprising the active ingredient in a suitable liquid carrier; as well as creams, emulsions, and gels containing, in addition to the active ingredient, such carriers as are known in the art. Additionally, formulations suitable for rectal administration may be presented as suppositories by mixing with a variety of bases such as emulsifying bases or water-soluble bases. Formulations suitable for vaginal administration may be presented as pessaries, tampons, creams, gels, pastes, foams, or spray formulas containing, in addition to the active ingredient, such carriers as are known in the art to be appropriate.

One skilled in the art will appreciate that suitable methods of exogenously administering a compound of the present disclosure to an animal are available, and, although more than one route can be used to administer a particular compound, a particular route can provide a more immediate and more effective reaction than another route.

As regards these applications, the present method includes the administration to an animal, particularly a mammal, and more particularly a human, of a therapeutically effective amount of the compound effective in the inhibition of kinase protein and especially LRRK2. The method also includes the administration of a therapeutically effect amount of the compound for the treatment of patient having a predisposition for being afflicted with Parkinson's disease.

The dose administered to an animal, particularly a human, in the context of the present invention should be sufficient to affect a therapeutic response in the animal over a reasonable time frame. One skilled in the art will recognize that dosage will depend upon a variety of factors including the condition of the animal, the body weight of the animal, as well as the severity and stage of the cancer.

The total amount of the compound of the present disclosure administered in a typical treatment is preferably between about 10 mg/kg and about 1000 mg/kg of body weight for mice, and between about 100 mg/kg and about 500 mg/kg of body weight, and more preferably between 200 mg/kg and about 400 mg/kg of body weight for humans per daily dose. This total amount is typically, but not necessarily, administered as a series of smaller doses over a period of about one time per day to about three times per day for about 24 months, and preferably over a period of twice per day for about 12 months.

The size of the dose also will be determined by the route, timing and frequency of administration as well as the existence, nature and extent of any adverse side effects that might accompany the administration of the compound and the desired physiological effect. It will be appreciated by one of skill in the art that various conditions or disease states, in particular chronic conditions or disease states, may require prolonged treatment involving multiple administrations.

Exemplary embodiments of the present disclosure include:
Embodiment A: A compound of formula I 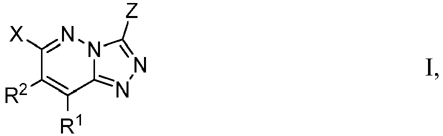 wherein
   - Z: is a 5- or 6- membered substituted or unsubstituted aryl or substituted or unsubstituted heterocyclo ring,
   - R¹ and R²: are identical or different and each is hydrogen, halogen, alkyl, or substituted or unsubstituted aryl containing 5 or 6 carbon atoms in the aryl ring;
   - X: is selected from

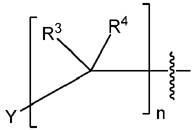

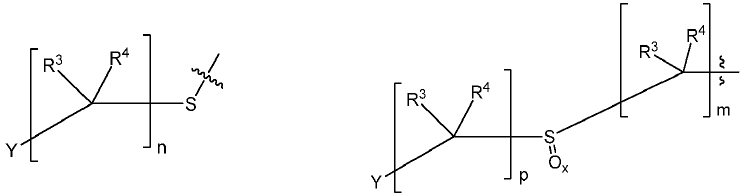

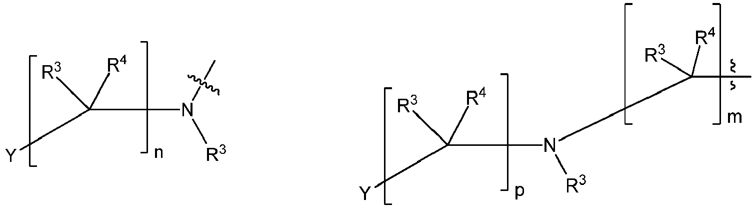

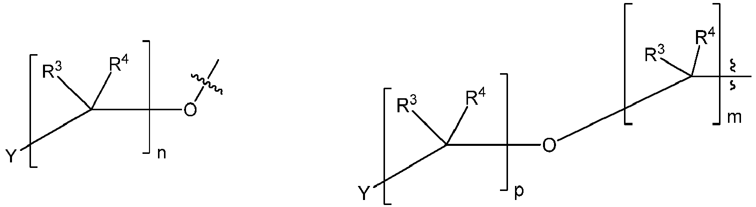

   - R³: is hydrogen, alkyl or haloalkyl;
   - R⁴: is hydrogen, alkyl, haloalkyl, -OH, -O-R⁶ or -NH₂, -NHR⁶, -NR⁶R⁷, -S-R⁶; or
   - R³ and R⁴,: together with the carbon atom to which they are bonded, form a ring moiety; or form a carbonyl to give a ketone, ester or amide; or
   - Y: form a double or triple bond with the proviso that they are not substituted with a S(O)ₓ,-NH₂, -NH(R⁶),-N(R⁶R⁷)-, -OH or -O-R⁶ to give an enol, enolether, vinyl sulfone, vinylthioether, vinylsulfoxide, enamine;. is absent, or is hydrogen, COR⁵, or S(O)ₓR⁵; or is substituted or unsubstituted aryl or substituted or unsubstituted heterocyclo group containing 4 to 7 atoms in the ring;
   - R⁵: is OH, OR⁶, NH₂, NHR⁶, NR⁶R⁷, substituted or unsubstituted aryl or substituted or unsubstituted heterocyclo group containing 4 to 7 atoms in the ring;
   - m: is 1 or 2;
   - n: is 1 to 5;
   - p: is 0 to 3;
   - x: is 0, 1 or 2;
   - R⁶ and R⁷: are identical or different and each independently is alkyl or haloalkyl; or
   - R⁶ and R⁷: of the NR⁶R⁷ moiety, together with the nitrogen to which they are bonded, form an unsubstituted or substituted 4- to 8-membered ring consisting of the nitrogen atom, carbon ring members and optionally an additional ring member selected from the group consisting of: O, S, SO, SO₂, N, and N(C₁-C₄-alkyl);
   or a derivative thereof selected from the group consisting of pharmaceutically acceptable salts, prodrugs, deuterated forms, radio-actively labeled forms, isomers, solvates and combinations thereof.
Embodiment B: A compound according to the foregoing Embodiment A which is represented by formula II 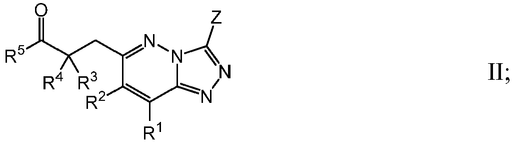 or a derivative thereof, wherein Z, R¹ to R⁵ are as defined for formula I.
Embodiment C: A compound according to the foregoing Embodiments A and B which is represented by formula III 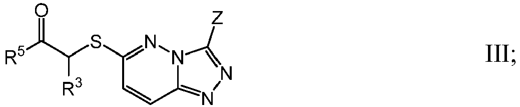 or a derivative thereof, wherein
   - Z: is 2-thiophene, 2-thiazole, 3-thiazole, 3-pyrazole, 3-pyrazole-N-methyl, 4-pyrazole N-methyl, 4-pyrazole, 2-pyridyl, 3-pyridyl, 4-pyridyl, 2- pyrimidine, phenyl, 2-benzofuran, 2-benzothiophene, 2-indole, 2-N-methylindole, 2-benzimidazole, 2-N-methyl benzimidazole, 2-benzothiazole, 2-benzisoxazole, in each case optionally substituted with fluorine iodine or a radioactive isotope of fluorine or iodine;
   - R³: is hydrogen or lower alkyl which is optionally substituted with fluorine or iodine or a radioactive isotope of fluorine or iodine;
   - R⁵: is OH, OR⁶, NH₂, NHR⁶ or NR⁶R⁷;
   - R⁶: is hydrogen or lower alkyl which is optionally substituted with fluorine or iodine or a radioactive isotope of fluorine or iodine;
   - R⁷: is hydrogen or lower alkyl which is optionally substituted with fluorine or iodine or a radioactive isotope of fluorine or iodine; or
   - R⁶ and R⁷,: together with the nitrogen atom to which they are bonded, form a 4- to 8-membered ring consisting of the nitrogen atom, carbon ring members and optionally an additional ring member selected from the group consisting of: O, S, SO, SO₂, N, and N(C₁-C₄-alkyl), which ring is optionally substituted with fluorine or iodine or a radioactive isotope of fluorine or iodine.
Embodiment D: A compound according to the Embodiments A to C which is represented by formula III 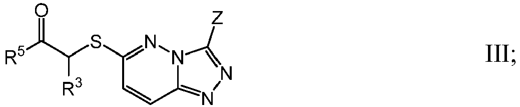 or a derivative thereof, wherein
   - Z: is 2-thiophene, 2-thiazole, 3-thiazole, 3-pyrazole, 3-pyrazole-N-methyl, 4-pyrazole N-methyl, 4-pyrazole, 2-pyridyl, 3-pyridyl, 4-pyridyl, 2- pyrimidine, phenyl, 2-benzofuran, 2-benzothiophene, 2-indole, 2-N-methylindole, 2-benzimidazole, 2-N-methyl benzimidazole, 2-benzothiazole, 2-benzisoxazole, in each case optionally substituted with fluorine, iodine or a radioactive isotope of fluorine or iodine;
   - R³: is hydrogen or lower alkyl which is optionally substituted with fluorine or iodine or a radioactive isotope of fluorine or iodine;
   - R⁵: is OH, OR⁶, NH₂, NHR⁶ or NR⁶R⁷;
   - R⁶: is hydrogen or lower alkyl which is optionally substituted with fluorine or iodine or a radioactive isotope of fluorine or iodine;
   - R⁷: is hydrogen or lower alkyl which is optionally substituted with fluorine or iodine or a radioactive isotope of fluorine or iodine; or
   - R⁶ and R⁷,: together with the nitrogen atom to which they are bonded, form a 4- to 8-membered ring consisting of the nitrogen atom, carbon ring members and optionally an additional ring member selected from the group consisting of: O, S, SO, SO₂, N, and N(C₁-C₄-alkyl), which ring is optionally substituted with fluorine or iodine or a radioactive isotope of fluorine or iodine.
Embodiment E: A compound according to the foregoing Embodiments A to D which is represented by formula III 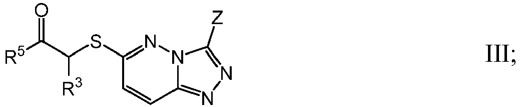 or a derivative thereof, wherein
   - Z: is 2-thiophene, 2-thiazole, 3-thiazole, 3-pyrazole-N-methyl, 2- pyrimidine, phenyl, in each case optionally substituted with fluorine, iodine or a radioactive isotope of fluorine or iodine;
   - R³: is hydrogen or lower alkyl which is optionally substituted with fluorine or iodine or a radioactive isotope of fluorine or iodine;
   - R⁵: is OH, OR⁶, NH₂, NHR⁶ or NR⁶R⁷;
   - R⁶: is hydrogen or lower alkyl which is optionally substituted with fluorine or iodine or a radioactive isotope of fluorine or iodine;
   - R⁷: is hydrogen or lower alkyl which is optionally substituted with fluorine or iodine or a radioactive isotope of fluorine or iodine; or
   - R⁶ and R⁷,: together with the nitrogen atom to which they are bonded, form a 4- to 8-membered ring consisting of the nitrogen atom, carbon ring members and optionally an additional ring member selected from the group consisting of: O, S, SO, SO₂, N, and N(C₁-C₄-alkyl), which ring is optionally substituted with fluorine or iodine or a radioactive isotope of fluorine or iodine.
Embodiment F (invention): A compound or derivative according to any one of the foregoing Embodiments A to E which is selected from the group consisting of:
   1-(piperidin-1-yl)-2-((3-(thiophen-2-yl)-[1,2,4]triazolo[4,3-b]pyridazin-6-yl)thio)butan-1-one,
   2-((3-(thiophen-2-yl)-[1,2,4]triazolo[4,3-b]pyridazin-6-yl)thio)propanoic acid,
   1-(piperidin-1-yl)-2-((3-(thiophen-2-yl)-[1,2,4]triazolo[4,3-b]pyridazin-6-yl)thio)propan-1-one,
   1-(azetidin-1-yl)-2-((3-(thiophen-2-yl)-[1,2,4]triazolo[4,3-b]pyridazin-6-yl)thio)propan-1-one,
   1-(pyrrolidin-1-yl)-2-((3-(thiophen-2-yl)-[1,2,4]triazolo[4,3-b]pyridazin-6-yl)thio)propan-1-one and
   2-(methyl((3-(thiophen-2-yl)-[1,2,4]triazolo[4,3-b]pyridazin-6-yl)methyl)amino)-1-(piperidin-1-yl)propan-1-one,
   or a derivative thereof.
Embodiment G: A compound or derivative according to any one of the foregoing Embodiments A to F wherein the isomers are enantiomers or diastereomers.
Embodiment H: A pharmaceutical composition comprising a compound or derivative according to any one of Embodiments A to G and a pharmaceutically acceptable carrier.
Embodiment I: A method of treating a patient with a disease caused by or associated with abnormal LRRK2 kinase activity, which comprises administering to the patient an effective treatment amount of at least one compound, derivative or composition according to any one of Embodiments A to H.
Embodiment J: A method for treating a patient having a neurodegenerative disease, which comprises administering to the patient an effective treatment amount of at least one compound, derivative or composition according to any Embodiments A to H.
Embodiment K: The method according to Embodiment J, wherein the neurodegenerative disease is Parkinson's disease.
Embodiment L: A method for treating a patient suffering from cancer, which comprises administering to the patient an effective treatment amount of at least one compound, derivative or composition according to any one of Embodiments A to H.
Embodiment M: The method according to Embodiment L, wherein said cancer is renal cancer or thyroid cancer.
Embodiment N: A method for treating a patient having an autoimmune disease, which comprises administering to the patient an effective treatment amount of at least one compound, derivative or composition according to any one of Embodiments A to H.
Embodiment O: The method according to Embodiment N, wherein the autoimmune disease is selected from the group consisting of Crohn's disease, rheumatoid arthritis and psoriasis.
Embodiment P: A method for treating a patient having leprosy, which comprises administering to the patient an effective treatment amount of at least one compound, derivative or composition according to any one of Embodiments A to H.

The term "comprising" (and its grammatical variations) as used herein is used in the inclusive sense of "having" or "including" and not in the exclusive sense of "consisting of." The terms "a", "an" and "the" as used herein are understood to encompass the plural as well as the singular, unless indicated otherwise.

## Claims

1. A compound selected from the group consisting of:
1-(piperidin-1-yl)-2-((3-(thiophen-2-yl)-[1,2,4]triazolo[4,3-b]pyridazin-6-yl)thio)butan-1-one,
2-((3-(thiophen-2-yl)-[1,2,4]triazolo[4,3-b]pyridazin-6-yl)thio)propanoic acid,
1-(piperidin-1-yl)-2-((3-(thiophen-2-yl)-[1,2,4]triazolo[4,3-b]pyridazin-6-yl)thio)propan-1-one,
1-(azetidin-1-yl)-2-((3-(thiophen-2-yl)-[1,2,4]triazolo[4,3-b]pyridazin-6-yl)thio)propan-1-one,
1-(pyrrolidin-1-yl)-2-((3-(thiophen-2-yl)-[1,2,4]triazolo[4,3-b]pyridazin-6-yl)thio)propan-1-one and
2-(methyl((3-(thiophen-2-yl)-[1,2,4]triazolo[4,3-b]pyridazin-6-yl)methyl)amino)-1-(piperidin-1-yl)propan-1-one,
or a derivative thereof, wherein said derivative is selected from the group consisting of pharmaceutically acceptable salts thereof, deuterated forms thereof, radio-actively labeled forms thereof, enantiomers, diastereoisomers, and solvates thereof.

2. A compound or derivative according to claim 1 wherein the derivatives are enantiomers or diastereomers.

3. A pharmaceutical composition comprising a compound or derivative according to any one of claims 1 or 2 and a pharmaceutically acceptable carrier.

4. Compound or derivative according to any one of claims 1 or 2 for use in a method of treating a patient with a disease caused by or associated with abnormal LRRK2 kinase activity.

5. Compound or derivative according to any one of claims 1 or 2 for use in a method for treating a patient having a neurodegenerative disease.

6. Compound or derivative for use in a method according to claim 5, wherein the neurodegenerative disease is Parkinson's disease.

7. Compound or derivative according to any one of claims 1 or 2 for use in a method for treating a patient suffering from cancer.

8. Compound or derivative for use in a method according to claim 7, wherein said cancer is renal cancer or thyroid cancer.

9. Compound or derivative according to any one of claims 1 or 2 for use in a method for treating a patient having an autoimmune disease.

10. Compound or derivative for use in a method according to claim 9, wherein the autoimmune disease is selected from the group consisting of Crohn's disease, rheumatoid arthritis and psoriasis.

11. Compound or derivative according to any one of claims 1 or 2 for use in a method for treating a patient having leprosy.

## Patentansprüche

1. Verbindung ausgewählt aus der Gruppe bestehend aus:
1-(Piperidin-1-yl)-2-((3-(thiophen-2-yl)-[1,2,4]triazol[4,3-b]pyridazin-6-yl)thio)butan-1-on,
2-((3-(Thiophen-2-yl)-[1,2,4]triazol[4,3-b]pyridazin-6-yl)thio)propansäure, 1-(Piperidin-1-yl)-2-((3-(thiophen-2-yl)-[1,2,4]triazol[4,3-b]pyridazin-6-yl)thio)propan-1-on,
1-(Azetidin-1-yl)-2-((3-(thiophen-2-yl)-[1,2,4]triazol[4,3-b]pyridazin-6-yl)thio)propan-1-on,
1-(Pyrrolidin-1-yl)-2-((3-(thiophen-2-yl)-[1,2,4]triazol[4,3-b]pyridazin-6-yl)thio)propan-1-on und
2-(Methyl((3-(thiophen-2-yl)-[1,2,4]triazol[4,3-b]pyridazin-6-yl)methyl)amino)-1-(piperidin-1-yl)propan-1-on,
oder einem Derivat davon, wobei das Derivat aus der Gruppe ausgewählt ist bestehend aus pharmazeutisch akzeptablen Salzen davon, deuterierten Formen davon, radioaktiv markierten Formen davon, Enantiomeren, Diastereomeren und Solvaten davon.

2. Verbindung oder Derivat nach Anspruch 1, wobei die Derivate Enantiomere oder Diastereomere sind.

3. Pharmazeutische Zusammensetzung umfassend eine Verbindung oder ein Derivat nach einem der Ansprüche 1 oder 2 und einen pharmazeutisch akzeptablen Träger.

4. Verbindung oder Derivat nach einem der Ansprüche 1 oder 2 zur Verwendung bei einem Verfahren zum Behandeln eines Patienten mit einer Krankheit, die durch anormale LRRK2-Kinaseaktivität verursacht wird oder damit verbunden ist.

5. Verbindung oder Derivat nach einem der Ansprüche 1 oder 2 zur Verwendung bei einem Verfahren zum Behandeln eines Patienten, der eine neurodegenerative Erkrankung aufweist.

6. Verbindung oder Derivat zur Verwendung bei einem Verfahren nach Anspruch 5, wobei die neurogener Erkrankung Parkinsonkrankheit ist.

7. Verbindung oder Derivat nach einem der Ansprüche 1 oder 2 zur Verwendung bei einem Verfahren zum Behandeln eines Patienten, der an Krebs leidet.

8. Verbindung oder Derivat zur Verwendung bei einem Verfahren nach Anspruch 7, wobei der Krebs Nierenkrebs oder Schilddrüsenkrebs ist.

9. Verbindung oder Derivat nach einem der Ansprüche 1 oder 2 zur Verwendung bei einem Verfahren zum Behandeln eines Patienten, der eine Autoimmunkrankheit aufweist.

10. Verbindung oder Derivat zur Verwendung bei einem Verfahren nach Anspruch 9, wobei die Autoimmunkrankheit aus der Gruppe ausgewählt ist bestehend aus Morbus Crohn, rheumatoider Arthritis und Schuppenflechte.

11. Verbindung oder Derivat nach einem der Ansprüche 1 oder 2 zur Verwendung bei einem Verfahren zum Behandeln eines Patienten, der Lepra aufweist.

## Revendications

1. Composé sélectionné dans le groupe constitué de :
la 1-(pipéridin-1-yl)-2-((3-(thiophén-2-yl)-[1,2,4]triazolo[4,3-b]pyridazin-6-yl)thio)butan-1-one,
l'acide 2-((3-(thiophén-2-yl)-[1,2,4]triazolo[4,3-b]pyridazin-6-yl)thio)propanoïque,
la 1-(pipéridin-1-yl)-2-((3-(thiophén-2-yl)-[1,2,4]triazolo[4,3-b]pyridazin-6-yl)thio)propan-1-one,
la 1-(azétidin-1-yl)-2-((3-(thiophén-2-yl)-[1,2,4]triazolo[4,3-b]pyridazin-6-yl)thio)propan-1-one,
la 1-(pyrrolidin-1-yl)-2-((3-(thiophén-2-yl)-[1,2,4]triazolo[4,3-b]pyridazin-6-yl)thio)propan-1-one, et
la 2-(methyl((3-(thiophén-2-yl)-[1,2,4]triazolo[4,3-b]pyridazin-6-yl)methyl)amino)-1-(pipéridin-1-yl)propan-1-one,
ou un dérivé de celui-ci, dans lequel ledit dérivé est sélectionné dans le groupe constitué de sels pharmaceutiquement acceptables de celui-ci, de formes deutérées de celui-ci, de formes marquées radio-activement de celui-ci, d'énantiomères, de diastéréoisomères, et de solvates de celui-ci.

2. Composé ou dérivé selon la revendication 1 dans lequel les dérivés sont des énantiomères ou des diastéréoisomères.

3. Composition pharmaceutique comprenant un composé ou un dérivé selon l'une quelconque des revendications 1 ou 2 et un véhicule pharmaceutiquement acceptable.

4. Composé ou dérivé selon l'une quelconque des revendications 1 ou 2 pour une utilisation dans une méthode de traitement d'un patient ayant une maladie causée par ou associée à une activité LRRK2 kinase anormale.

5. Composé ou dérivé selon l'une quelconque des revendications 1 ou 2 pour une utilisation dans une méthode de traitement d'un patient ayant une maladie neurodégénérative.

6. Composé ou dérivé pour une utilisation dans une méthode selon la revendication 5, dans laquelle la maladie neurodégénérative est la maladie de Parkinson.

7. Composé ou dérivé selon l'une quelconque des revendications 1 ou 2 pour une utilisation dans une méthode de traitement d'un patient souffrant d'un cancer.

8. Composé ou dérivé pour une utilisation dans une méthode selon la revendication 7, dans laquelle ledit cancer est un cancer du rein ou un cancer de la thyroïde.

9. Composé ou dérivé selon l'une quelconque des revendications 1 ou 2 pour une utilisation dans une méthode de traitement d'un patient ayant une maladie auto-immune.

10. Composé ou dérivé pour une utilisation dans une méthode selon la revendication 9, dans laquelle la maladie auto-immune est sélectionnée dans le groupe constitué de la maladie de Crohn, la polyarthrite rhumatoïde et le psoriasis.

11. Composé ou dérivé selon l'une quelconque des revendications 1 ou 2 pour une utilisation dans une méthode de traitement d'un patient ayant la lèpre.
